(19) Europäisches Patentamt / European Patent Office / Office européen des brevets

(11) **EP 4 151 717 A1**

(12)
# EUROPEAN PATENT APPLICATION
published in accordance with Art. 153(4) EPC

(43) Date of publication:
**22.03.2023 Bulletin 2023/12**

(21) Application number: **21765044.9**

(22) Date of filing: **02.03.2021**

(51) International Patent Classification (IPC):
*C12N 1/04* (2006.01)     *A61K 9/10* (2006.01)
*A61K 35/17* (2015.01)    *A61K 47/02* (2006.01)
*A61K 47/12* (2006.01)    *A61P 31/12* (2006.01)
*A61P 35/00* (2006.01)    *A61P 37/04* (2006.01)
*C12N 5/071* (2010.01)    *C12N 5/0783* (2010.01)

(52) Cooperative Patent Classification (CPC):
**A61K 9/10; A61K 35/17; A61K 47/02; A61K 47/12; A61P 31/12; A61P 35/00; A61P 37/04; C12N 1/04; C12N 5/06**

(86) International application number:
**PCT/JP2021/007863**

(87) International publication number:
**WO 2021/177279 (10.09.2021 Gazette 2021/36)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **02.03.2020 JP 2020035297**

(71) Applicant: **GAIA BioMedicine Inc.**
**Tokyo 162-0821 (JP)**

(72) Inventors:
• **HARADA Yui**
**Fukuoka-shi, Fukuoka 819-0395 (JP)**
• **YONEMITSU Yoshikazu**
**Fukuoka-shi, Fukuoka 819-0395 (JP)**

(74) Representative: **Vossius & Partner**
**Patentanwälte Rechtsanwälte mbB**
**Siebertstraße 3**
**81675 München (DE)**

(54) **METHOD FOR TREATING HIGHLY ACTIVE NK CELLS**

(57)     The object is to provide a method for cryopreserving and thawing cells with maintaining high viability and high activity, which is applicable to highly active NK cells. A method for treating cells, which comprises the following steps of: (1) collecting in vitro-activated cells in a medium for cell culture; (2) suspending the collected cells in a solution for cryopreservation; and (3) freezing the suspended cells. The step (1) preferably includes a treatment with a medium supplemented with any selected from the group consisting of a bile acid and phenylbutyric acid.

EP 4 151 717 A1

[Fig. 1-3]

**Description**

Technical Field

The present invention relates to a method for cryopreserving cells having high cytotoxic activity.

Background Technique

NK cells are important in the rejection of tumor cells and virus-infected cells. In August 2017, chimeric antigen receptor (CAR) T-cell therapy was approved in the United States for the treatment of relapsed and refractory B-cell acute lymphoblastic leukemia (B-ALL) in children and young adults, and in recent years, clinical application of CAR-NK has been expanding in place of CAR-T (see Non-patent document 1).

When attempting to administer cells to a patient, the first consideration is to use cells harvested from the patient himself/herself to avoid rejection. However, depending on the patient's condition, it may be difficult to collect the necessary amount of cells for treatment. In addition, the degree to which cells can be activated and proliferated in vitro varies among individuals, and there are also cases in which proliferation and activation are difficult. In addition, it takes a certain period of time for cells to be activated and proliferated, posing a problem that it is difficult to start treatment immediately. In this respect, it would be desirable if cells could be activated in advance and stored for preparation of administration.

As a method for preserving cells, it is known to preserve cells in suspension without freezing for short-time preservation (e.g., Patent document 1), and to freeze cells for long time preservation (e.g., Patent document 2). Furthermore, use of a solution for freezing containing a sodium salt, potassium salt, sugar, cryoprotectant, and hydrogencarbonate and/or carbonate has been investigated, since use of such a solution may provide cells that maintain high viability even after freeze-thawing (Patent document 3).

Prior Art References

Patent documents

  Patent document 1: Japanese Patent Publication (Kokai) No. 2006-230396
  Patent document 2: Japanese Patent Publication (Kokai) No. 2002-233356
  Patent document 3: WO2011/021618

Non-patent document

Non-patent document 1: Liu E., et al., N. Engl. J. Med., 2020;382:545-53

Summary of the Invention

Object to be achieved by the invention

However, conventional freezing methods using protective agents such as dimethyl sulfoxide can preserve T-cell lines and primary NK cells, but are not sufficient for NK cells with high cytotoxic activity, whose activity and viability are significantly reduced by freeze-thaw operations. This problem could not be solved by use of a programmed freezer, cell density control, addition of dextran, albumin, carboxylated poly-L-lysine, etc. and so forth. In addition, if cells for administration are packaged on the assumption that a certain amount of the cells will die, a step for reactivation culture is required after thawing, and the resulting cells must be further washed. Therefore, frozen and stocked highly active NK cells can currently be used only at facilities that meet the criteria for cell culture processing facilities that handle cells for clinical use (Cell Processing Center, CPC).

An object of the present invention is to provide a method for cryopreserving and thawing cells with maintaining high viability and high activity of the cells, which is applicable to highly active NK cells.

Means for achieving the object

The present invention provides the followings.

  [1] A method for treating cells, which comprises the following steps of:

(1) collecting in vitro-activated cells in a medium for cell culture;
(2) suspending the collected cells in a solution for cryopreservation; and
(3) freezing the suspended cells.

[2] The method according to [1], wherein the step (1) includes a treatment with a medium supplemented with any selected from the group consisting of a bile acid and phenylbutyric acid.

[3] The method according to [1] or [2], which further comprises the following step of:

(4) thawing the cryopreserved cells and suspending them in a thawing solvent I.

[4] The method according to any one of [1] to [3], wherein the thawing solvent I is an aqueous solution containing the followings:

- Sodium chloride 9.00 to 108 mM
- Sodium gluconate 2.30 to 27.7 mM
- Sodium acetate 2.70 to 32.5 mM
- Potassium chloride 0.496 to 5.96 mM, and
- Magnesium chloride 0.148 to 1.78 mM.

[5] The method according to any one of [1] to [4], wherein the thawing solvent I satisfies at least one of the following criteria:

- The solvent does not contain calcium ions at a concentration of 0.423 mM or higher,
- The solvent does not contain glucose at a concentration of 5.55 mM or higher, and
- The solvent does not contain lactate at a concentration of 27.7 mM or higher.

[6] A solution for suspending highly active NK cells, which contains the followings:

- Sodium chloride 9.00 to 108 mM,
- Sodium gluconate 2.30 to 27.7 mM
- Sodium acetate 2.70 to 32.5 mM
- Potassium chloride 0.496 to 5.96 mM, and
- Magnesium chloride 0.148 to 1.78 mM.

[7] The solution according to [6], which is for suspending highly active NK cells that have been suspended in a solution for cryopreservation and frozen.

[8] A pharmaceutical composition containing highly active NK cells, a solution for cryopreservation, and the solution according to [6].

[9] A method for producing a pharmaceutical composition containing cells, which comprises the following steps of:

(1) collecting in vitro-activated cells in a medium for cell culture;
(2) suspending the collected cells in a solution for cryopreservation; and
(3) freezing the suspended cells.

[10] The production method according to [9], wherein the step (1) includes a treatment with a medium supplemented with any selected from the group consisting of a bile acid and phenylbutyric acid.

Brief Description of the Drawings

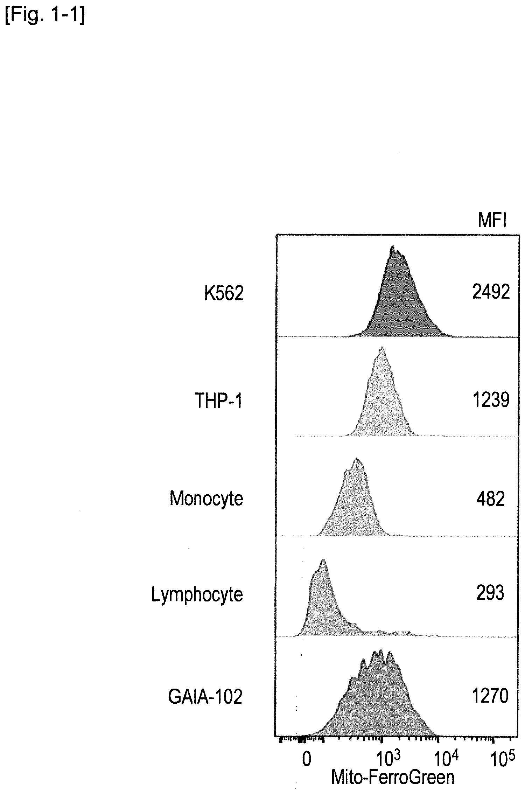

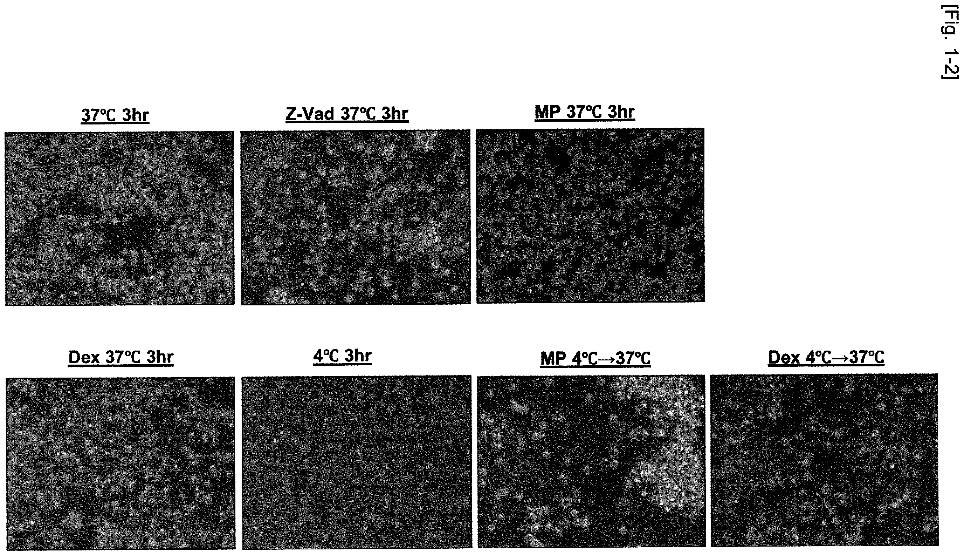

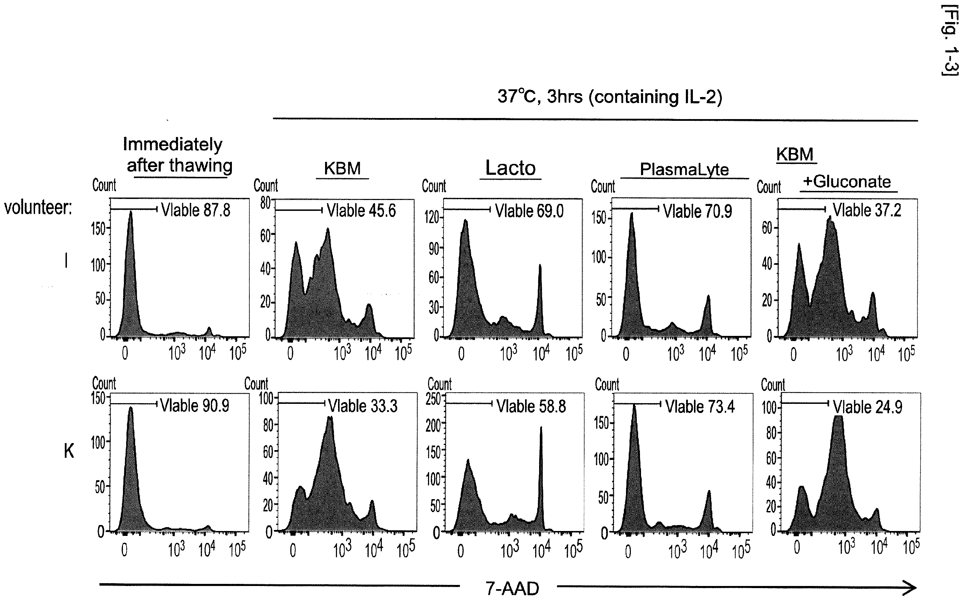

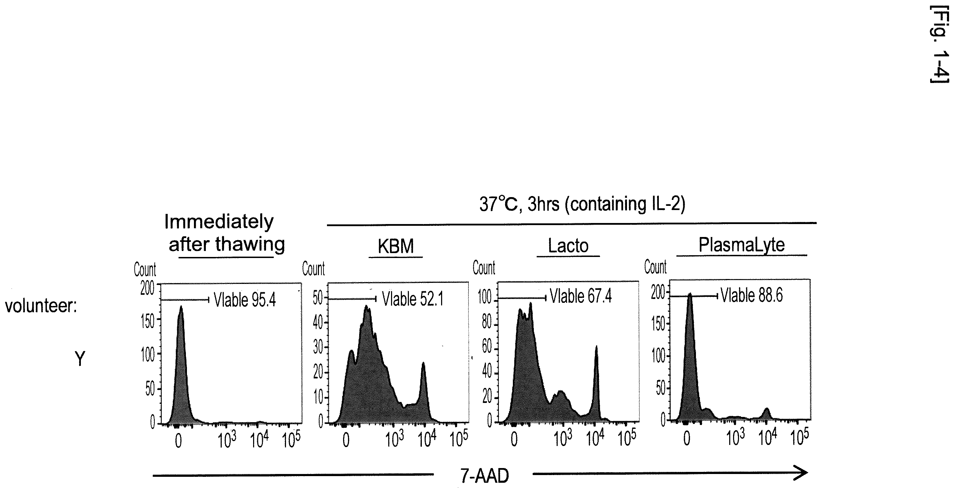

[Fig. 1-1] Results of flow cytometric analysis of various cells subjected to Mito-FerroGreen reaction. Highly active NK cells have a high iron content, and possibility of occurrence of ferroptosis induction is suspected.
[Fig. 1-2] Optical micrographs of highly active NK cells after freeze-thawing. MP represents methylprednisolone, and Dex represents dexamethasone. Significant reduction of viability of the highly active NK cells was confirmed after freeze-thawing.
[Fig. 1-3] Viability of highly active NK cells after thawing. When the cells were diluted with Plasma-Lyte A at the time of thawing, the viability was improved. However, no improvement was observed when gluconic acid was added to the KBM501 medium. Lacto represents Lactoringer.
[Fig. 1-4] Continuation of Fig. 1-3.

[Fig. 2] Viability of highly active NK cells after thawing. Dilution with Plasma-Lyte A at the time of thawing improved viability, but dilution with Plasma-Lyte A containing 40% serum (human type AB serum) worsened viability.

[Fig. 3] Live cell count and viability (right), and cytotoxic activity (left) of highly active NK cells left standing for a certain period of time. Plasma represents Plasma-Lyte A.

[Fig. 4] Effect of pretreatment of highly active NK cells before freezing. A treatment with KBM501 supplemented with 4-PBA before freezing improved their viability after thawing.

[Fig. 5] Recovery rate of highly active NK cells frozen and thawed after pretreatment for 2 hours. A treatment with KBM501 supplemented with 4-PBA or TUDCA before freezing improved their viability after thawing.

[Fig. 6-1] Cytotoxic activity of highly active NK cells frozen and thawed after pretreatment for 2 hours. When Plasma-Lyte A was used as a solution for suspending the thawed cells, cytotoxic activity was improved in a TUDCA concentration-dependent manner at both time points after 1 and 3 hours.

[Fig. 6-2] Continuation of Fig. 6-1.

[Fig. 7-1] Cytotoxic activity of highly active NK cells frozen and thawed after pretreatment for 2 hours. The concentration range of TUDCA for the pretreatment was widened. The target was K562 cells, the cells are mixed at E:T of 2:1 and co-cultured for 2 hours. The number of NK cells was calculated on the basis of the count before freezing. The both graphs show the Cytotoxic activity immediately after thawing (1), and after a treatment with Plasma-Lyte A for 1 hour (2), treatment with Plasma-Lyte A for 3 hours (3), and treatment with Plasma-Lyte A for 1 hour + and KBM501 for 2 hours (4).

[Fig. 7-2] Results of evaluation (3D killing assay) of thawed highly active NK cells in a solid tumor model.

[Fig. 8-1] Flow cytometric analysis of cells after 7-ADD staining. After the thawing, cells were diluted 10-fold with a prescribed solvent, and incubated at room temperature for 2 hours (right), or at 37°C for 3 hours (left).

[Fig. 8-2] Flow cytometric analysis of cells after 7-ADD staining. After the collection, the cells were washed with PBS (left) or KBM501 medium (right), and the thawed cells were diluted 10-fold with a prescribed solvent, and incubated at 37°C for 3 hours.

[Fig. 8-3] Flow cytometric analysis of cells after 7-ADD staining. After the thawing, cells were diluted 10-fold in various solvents, and incubated at 37°C for 3 hours.

[Fig. 8-4] Flow cytometric analysis of cells after 7-ADD staining. After the collection, the cells were washed with PBS (left) or KBM501 medium (right), and the thawed cells were diluted 10-fold with a prescribed solvent, and incubated at 37°C for 3 hours.

[Fig. 8-5] Flow cytometric analysis of cells after 7-ADD staining. After the collection, the cells were washed with PBS (left) or KBM501 medium (right), and the thawed cells were diluted 10-fold with a prescribed solvent, and incubated at 37°C for 3 hours.

[Fig. 9-1] Changes of viability (A), cytotoxic activity (target was K562, E:T was 1:2 to 4:1, 2 hours) (B), and cytotoxic activity (target was K562, E:T was 1:1, 2 to 8 hours) (C) of highly active NK cells after they were washed, frozen, thawed, and left standing at room temperature for 0 to 4 hours in the dosage form.

[Fig. 9-2] Continuation of Fig. 9-1.

[Fig. 9-3] Cytotoxic activity (target was K562, E:T was 1:1, 2 hours) of highly active NK cells after they were washed, frozen, thawed, and left standing at room temperature for each period of time in the dosage form.

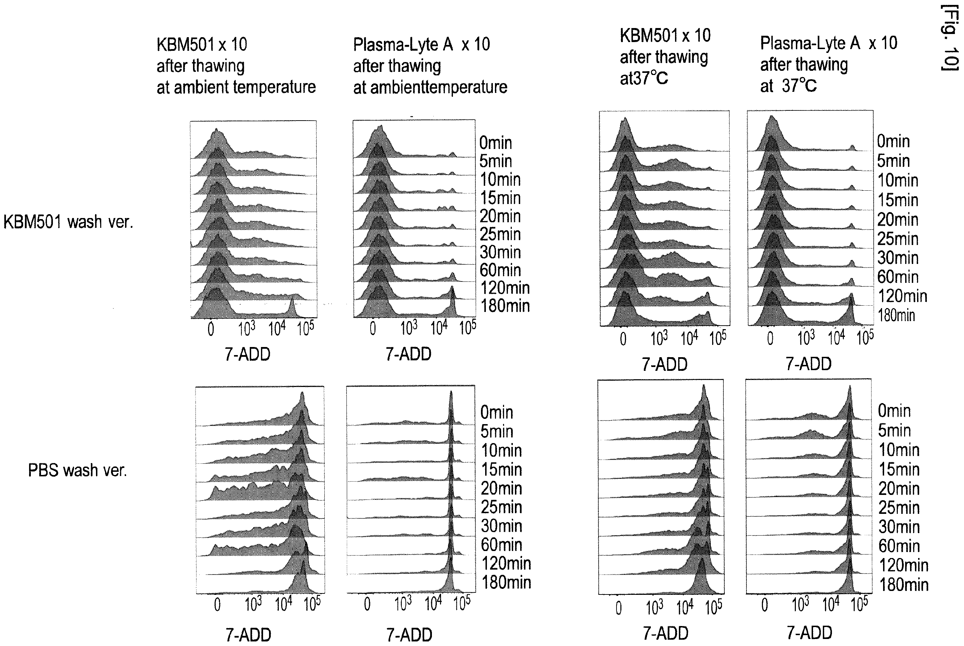

[Fig. 10] Changes of viability of highly active NK cells. The highly active NK cells were washed with KBM501 medium or PBS, then frozen, thawed at room temperature, left standing at room temperature for each period of time without dilution, then diluted 10-fold with KBM501 medium or Plasma-Lyte A, and stained with 7-AAD, and the results were analyzed with the FlowJo software (left). In addition, the highly active NK cells were washed with KBM501 medium or PBS, frozen, then thawed at 37°C, left standing at room temperature for each period of time without dilution, then diluted 10-fold with KBM501 medium or Plasma-Lyte A, and stained with 7-AAD, and the results were analyzed with the FlowJo software (right).

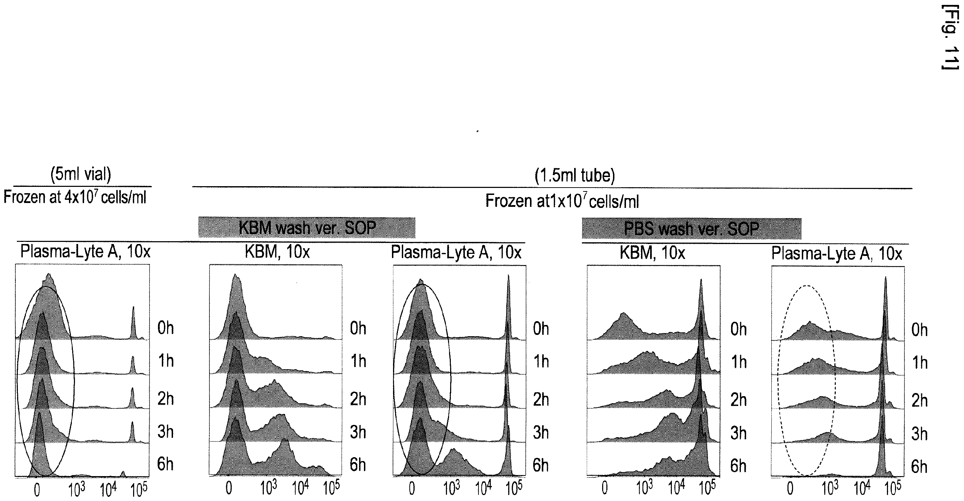

[Fig. 11] Changes of viability of highly active NK cells. The highly active NK cells were washed with KBM501 medium or PBS, frozen, then thawed at 37°C, diluted 10-fold with KBM501 medium or Plasma-Lyte A, left standing at room temperature for 0 to 6 hours, and then stained with 7-AAD, and the results were analyzed with the FlowJo software.

[Fig. 12-1] Changes of viability of highly active NK cells. Effects of dilution with Plasma-Lyte A serially diluted with sterile distilled water and leaving standing at 37°C. Cells were stained with 7-AAD after each treatment, and the results were analyzed with the FlowJo software (the same shall apply to the following figures).

[Fig. 12-2] Continuation of Fig. 12-1.

[Fig. 12-3] Changes of viability of highly active NK cells. Effects of dilution with Plasma-Lyte A serially diluted with saline and leaving standing at room temperature or 37°C.

[Fig. 12-4] Continuation of Fig. 12-3.

[Fig. 12-5] Changes of viability of highly active NK cells. Effects of dilution with Plasma-Lyte A serially diluted with saline or dilution with saline, and leaving standing at room temperature or 37°C.

[Fig. 12-6] Continuation of Fig. 12-5.

[Fig. 12-7] Changes of viability of highly active NK cells. Effects of dilution with various solvents, and leaving standing at room temperature.

[Fig. 12-8] Changes of viability of highly active NK cells. Effects of dilution with various solvents, and leaving standing at 37°C.

[Fig. 12-9] Changes of viability of highly active NK cells. Effects of dilution with various solvents, and leaving standing at room temperature for the cells washed with PBS at the time of collection.

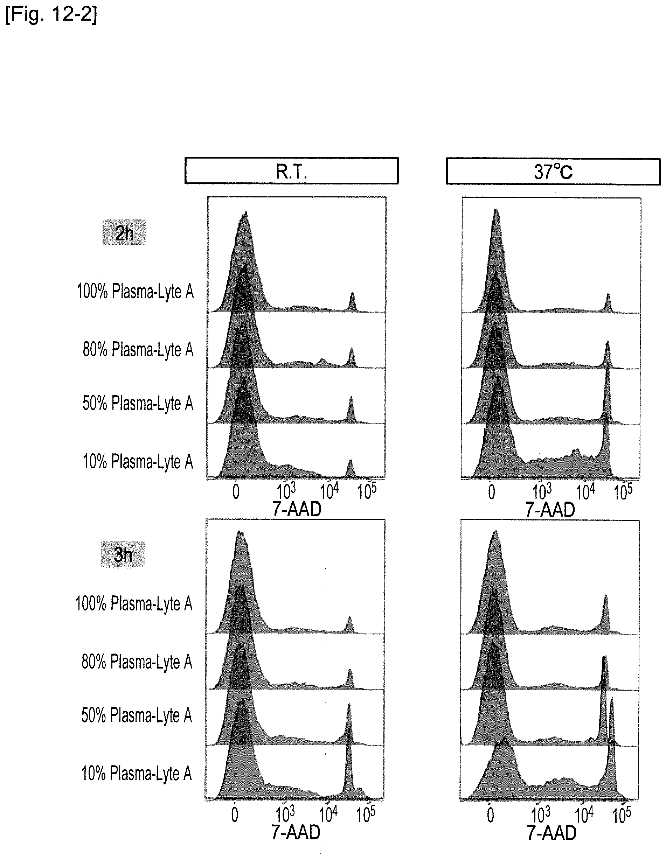

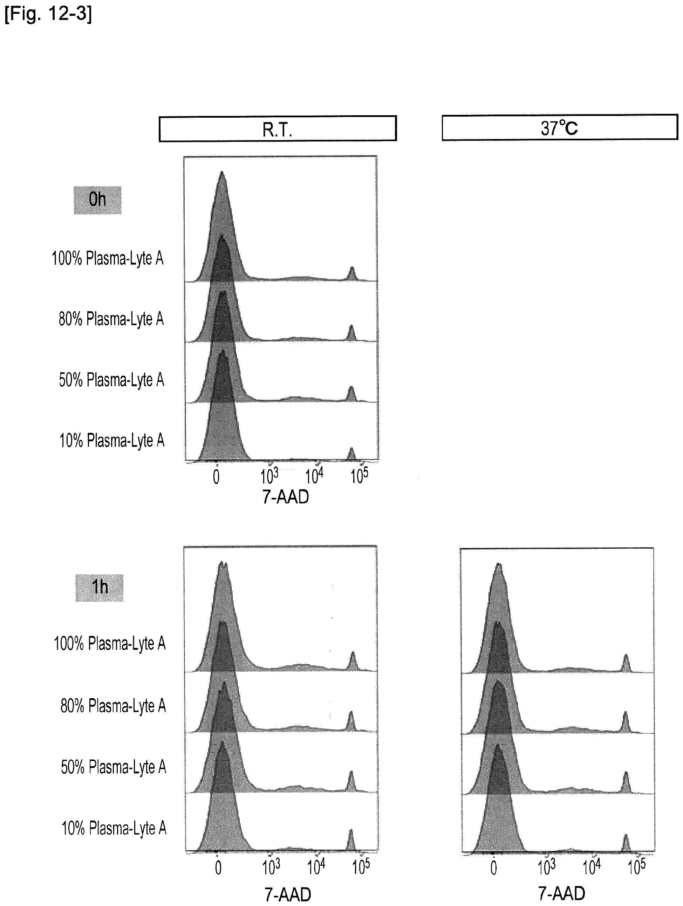

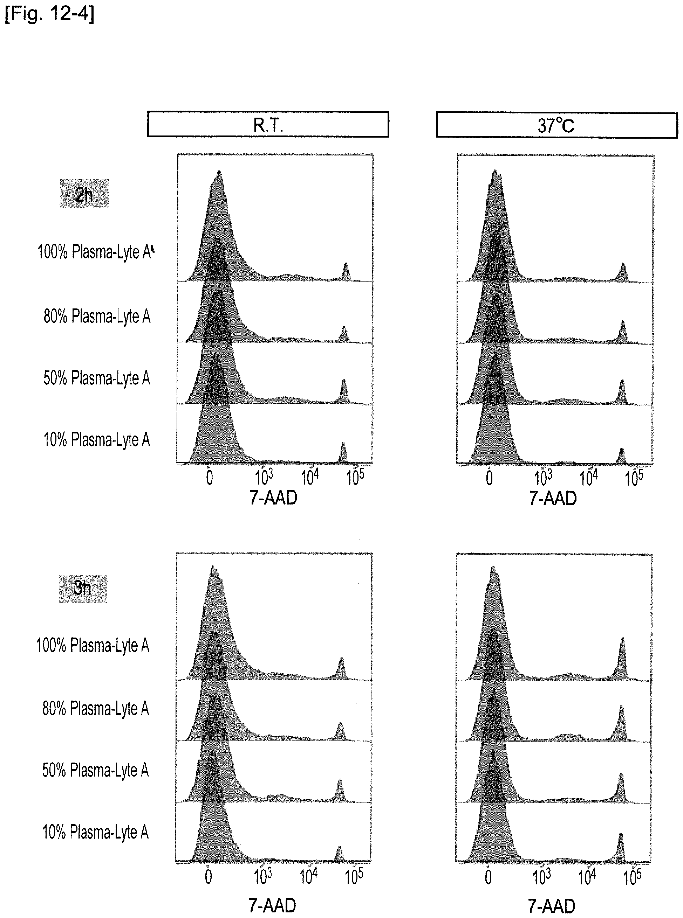

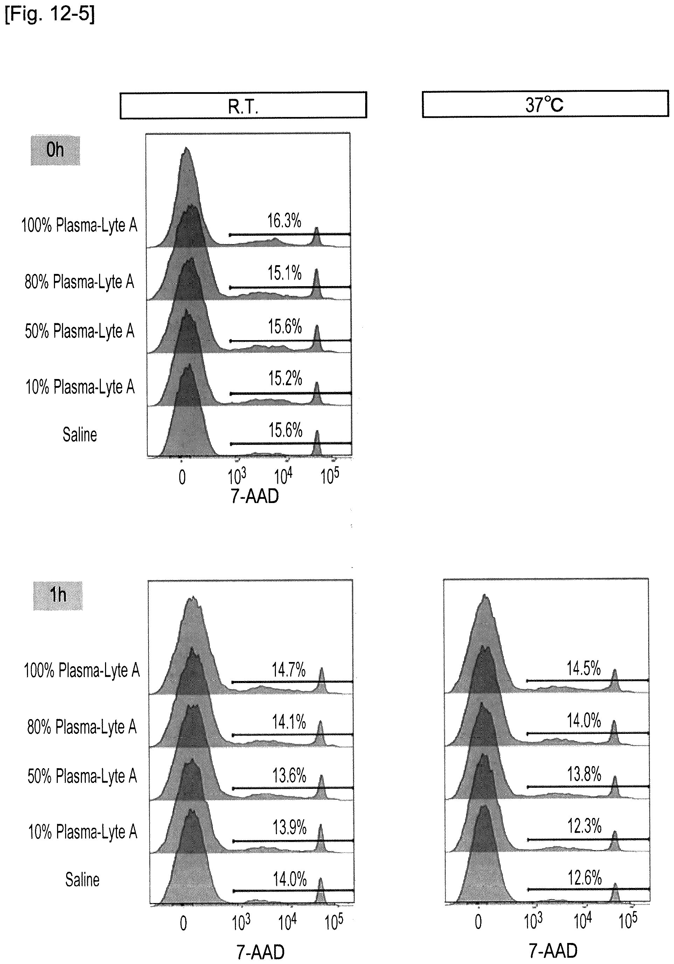

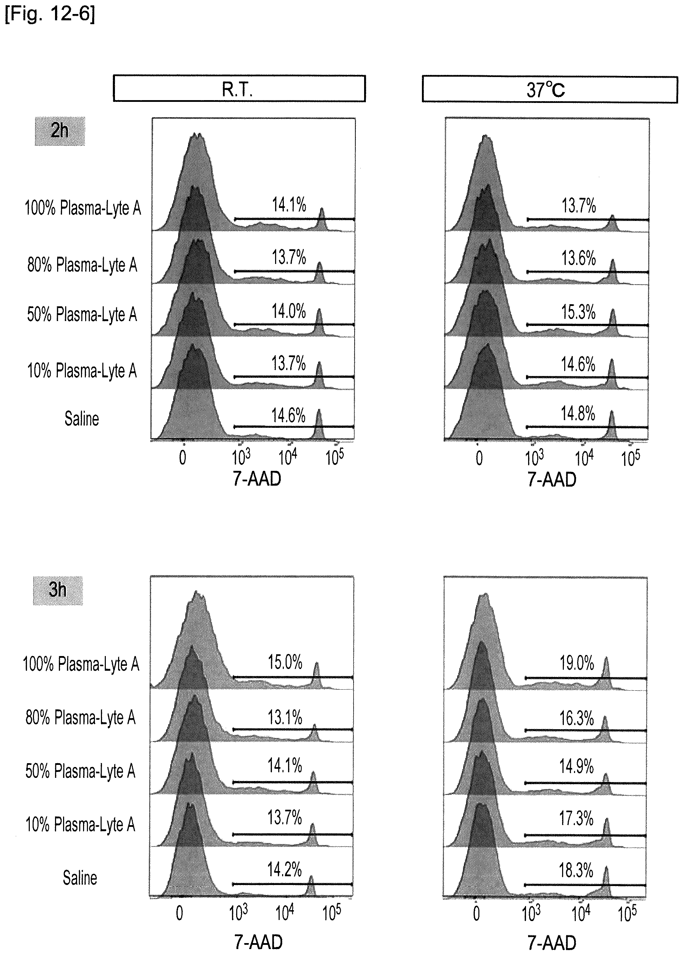

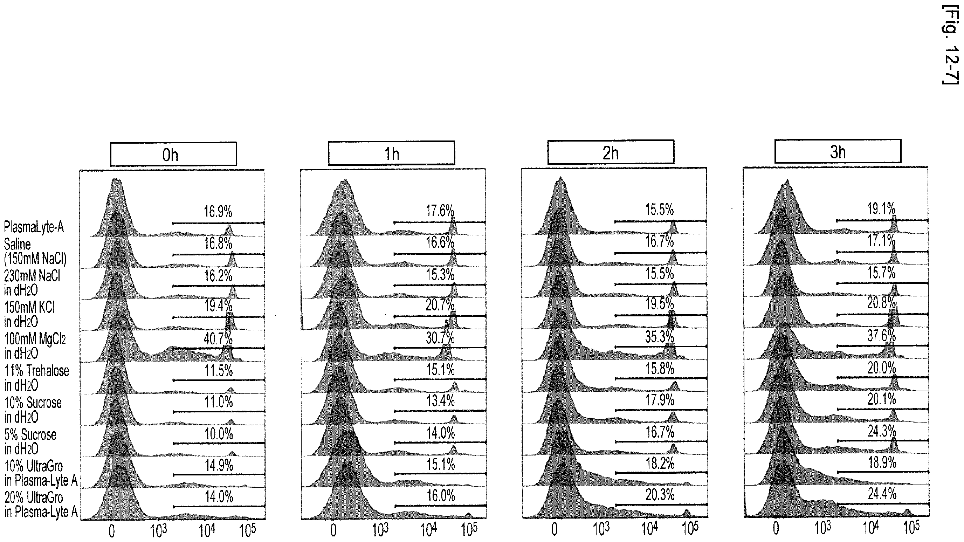

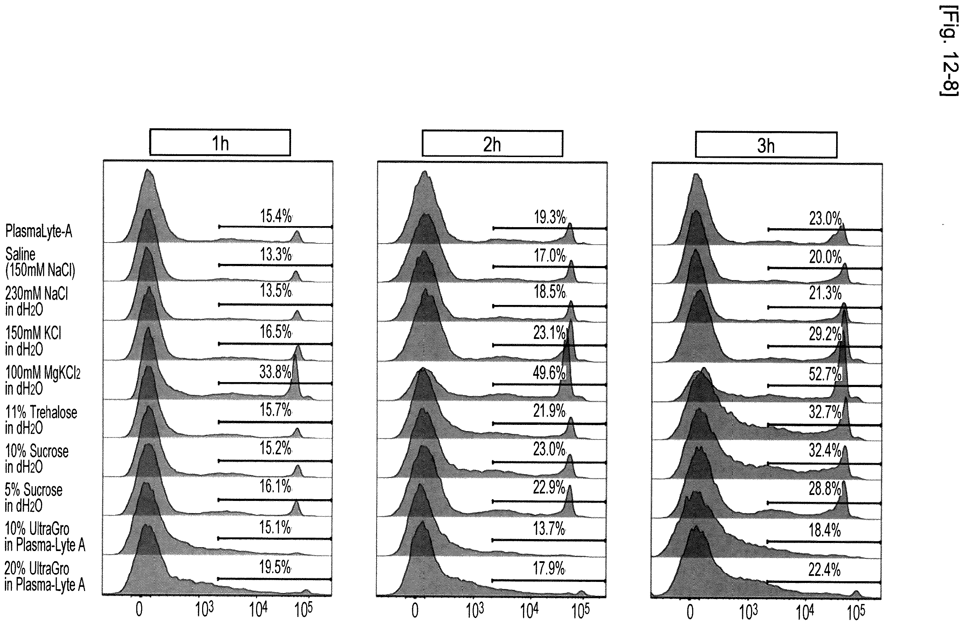

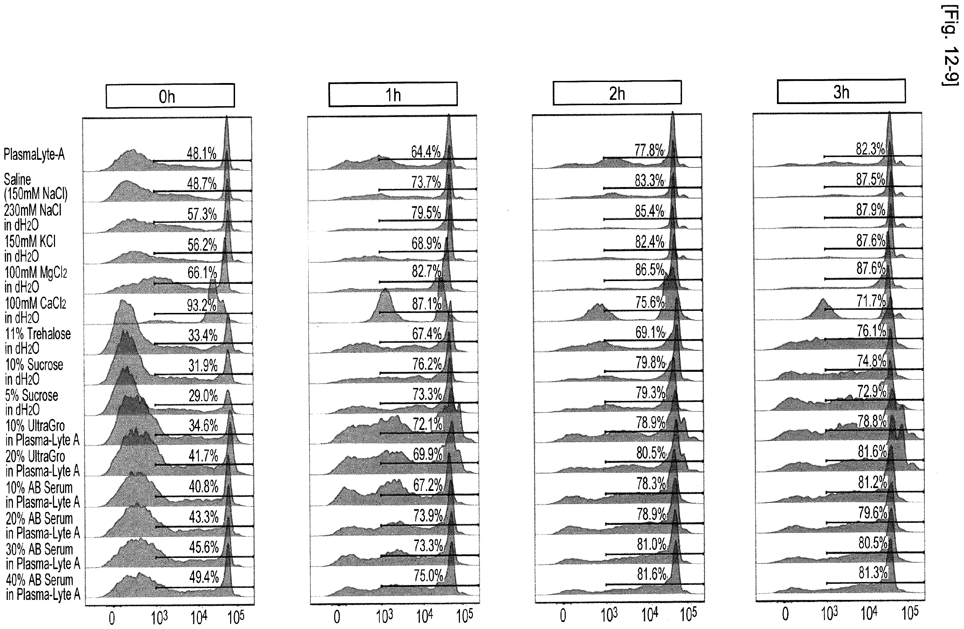

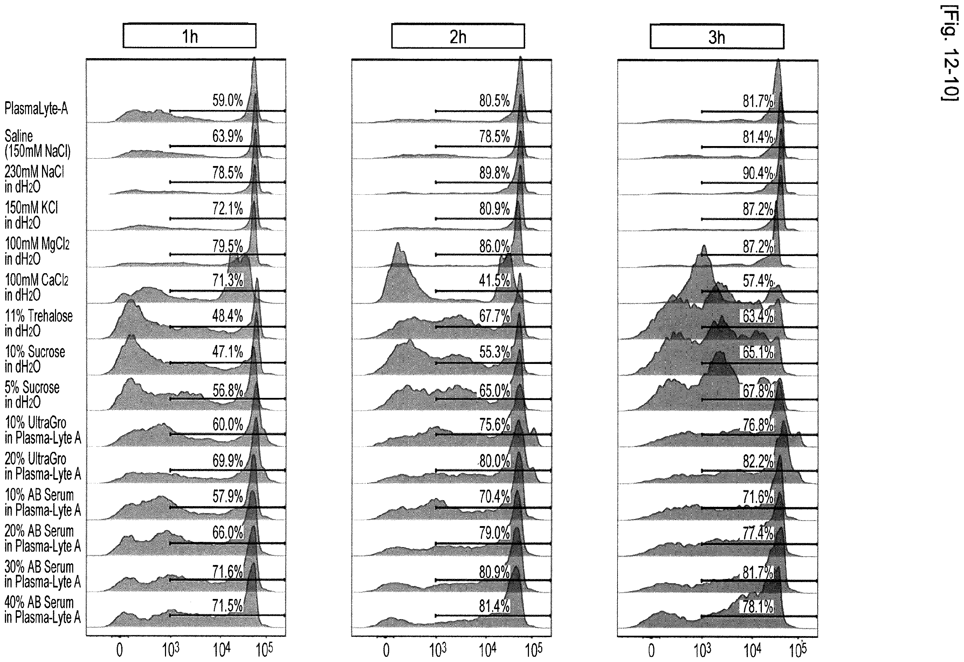

[Fig. 12-10] Changes of viability of highly active NK cells. Effects of dilution with various solvents, and leaving standing at 37°C for the cells washed with PBS at the time of collection.

Modes for Carrying out the Invention

In the explanations of the present invention, mM is used with the same meaning as mmol/L, unless especially noted. When a numerical range is expressed as x to y, the range includes the values x and y at both ends.

The present invention relates to a method for cryopreserving cells having high cytotoxic activity.

[Applicable cells]

The present invention can be applied to a variety of cells. One class of the cells to which the present invention can be preferably applied are cells that have undergone an activation operation by using some cytokine in vitro, such as NK cells with high cytotoxic activity (highly active NK cells). The activation operation is typically based on incubating the cells with a medium containing interleukin (IL)-2.

In general, NK cells are large granular lymphocytes that are not expressing the T cell receptor (TCR), the universal T cell marker, CD3, and the membrane immunoglobulin, B cell receptor, and they are usually CD16-positive and CD56-positive in humans. Whether or not cells are NK cells can be easily determined by those skilled in the art on the basis of expression patterns of cell surface markers, and so forth. NK cells have cytotoxic activity, and the presence or absence and degree of cytotoxic activity can be measured by various known methods. NK cells can include peripheral blood NK cells, cord blood NK cells, primary NK cells, cultured NK cells, and highly active NK cells.

(Raw material)

Raw material of highly active NK cells and so forth to which the present invention can be preferably applied may be peripheral blood, cord blood, bone marrow and/or lymph nodes, and blood collected by apheresis method (apheresis blood). The raw material may also be those prepared from at least one kind of cells selected from hematopoietic stem cells derived from any selected from the group consisting of embryonic stem cells, adult stem cells, and induced pluripotent stem (iPS) cells, hematopoietic stem cells derived from cord blood, hematopoietic stem cells derived from peripheral blood, hematopoietic stem cells derived from bone marrow blood, cord blood mononuclear cells, and peripheral blood mononuclear cells. The donor of the raw material may be a patient himself/herself who will receive an immunotherapy using highly active NK cells or the like, a close relative of the patient, or a healthy person genetically unrelated to the patient. The donor may consist of a plurality of donors.

(Culture medium)

Examples of the culture medium used for culturing highly active NK cells and so forth include the KBM501 medium (Kojin Bio, containing 1,750 JRU/mL of IL-2), Cosmedium 008 (Cosmo Bio, containing 1,750 JRU/mL of IL-2), FKCM101 (Fukoku, containing no IL-2 or 175 IU/mL of IL-2), CellGro SCGM medium (CellGenix, Iwai Chemical), X-VIVO15 medium (Lonza, Takara Bio), Gibco (registered trademark) CTS (registered trademark) AIM V (registered trademark) Medium (Thermo Fisher Scientific, serum-free medium of known composition for growth and manipulation of T cells and dendritic cells), CTS OpTmizer T Cell Expansion Basal Medium (Thermo Fisher Scientific, for growth and proliferation of human T lymphocytes), IMDM, MEM, DMEM, RPMI 1640, and so forth, but not limited to these. Preferred examples are the KBM501 medium, FKCM101, and Cosmedium 008. For the present invention, the expression that culture of cells (cells are cultured) means to maintain cells in a medium or a similar solution for a certain period of time for any purpose selected from the group consisting of maintaining cell viability, amplifying cells, and activating cells, unless especially stated. To cany out a treatment at a specific temperature for a certain period of time may be sometimes referred to as incubation (to incubate).

IL-2 may be added to the medium at such a concentration that the purpose of the invention can be achieved. The concentration of IL-2 may range from 2500 to 2813 IU/mL. IL-2 should preferably have a human amino acid sequence thereof, and be produced by a recombinant DNA technique for safety reasons. IL-2 concentration may be expressed in the national standard unit (Japan Reference Unit, JRU) and international unit (IU). 1 IU is approximately 0.622 JRU, and therefore 1,750 JRU/mL of the existing media is equivalent to approximately 2813 IU/mL.

Together with or instead of IL-2 described above, one selected from the group consisting of IL-12, IL-15, and IL-18 may be added at such a concentration that the purpose of the present invention can be achieved (Non-patent document 2, Leong JW et al., Biol. Blood Marrow Transplant, 20 (2014) 463-473). The concentration of each may be 1 pg/mL to 1 μg/mL irrespective of presence or absence or concentration of other cytokines. IL-2 preferably has a human amino acid sequence thereof, and be produced by recombinant DNA technique for safety reasons.

The medium may be supplemented with the subject's autologous serum, human type AB serum available from BioWhittaker and others, or donated blood human serum albumin available from the Japanese Red Cross Society. Autologous serum and human AB serum are preferably added at a concentration of 1 to 10%, and donated blood human serum albumin is preferably added at a concentration of 1 to 10%. Human platelet lysate (HPL) may be added together with or instead of serum. HPL is commercially available, and those of the UltraGRO™ series (AventaCell BioMedical), and so forth are commercially available. Sodium heparin may be further added to the medium, when HPL is used.

The medium may contain appropriate proteins, cytokines, antibodies, compounds, and other components, on condition that they do not impair the effectiveness of the NK cell culture. Cytokines may be IL-2, IL-12, IL-15, and IL-18 described above, as well as IL-3, IL-7, IL-21, stem cell factor (SCF), and/or FMS-like tyrosine kinase 3 ligand (Flt3L). All of these should preferably have human amino acid sequences thereof, and be produced by recombinant DNA technique for safety reasons.

The medium should preferably be a serum-free medium. The serum-free medium should preferably contain serum albumin, transferrin, and insulin. Serum-free media for culturing lymphocytes have been developed, and are commercially available, and they can be used for the present invention. One preferred example of serum-free medium is a basic medium supplemented with CTS Immune Cell SR (Thermo Fisher Scientific), which is commercially available as a composition that supports the proliferation of human T cells.

The medium may be replaced or replenished at any time after the start of culture, on condition that the desired culture effect is obtained, but the medium is preferably replaced or replenished every 3 to 5 days.

Culture vessels used for the culture include, but are not limited to, commercially available dishes, flasks, plates, and multi-well plates. Culture conditions are not particularly limited, so long as the culture effect of NK cells is not impaired, but culture conditions of 37°C, 5% $CO_2$, and saturated water vapor atmosphere are generally used. Culture period is not particularly limited, on condition that the desired culture effect is obtained.

Highly active NK cells, and so forth to which the present invention is preferably applied include the following [1], [2], [3] and [4].

[1] NK cells having the following characteristics (1) and (2):

    (1) CD1 6-positivity, high CD56 expression, and CD57-negativity, and
    (2) NKG2C-positivity, NKG2A-negativity to low expression, and CD94-positivity.

The highly active NK cells of [1] may show high CD16 expression. The highly active NK cells of [1] may also have the following characteristics, regardless of whether or not they show high CD 16 expression; (3) Cytotoxic activity of 50% or higher when the NK cells are used as effector cells (E) for K562 cells as target cells (T) in co-culture at a mixing ratio (E:T) of 1:1.

The highly active NK cells of [1] can also be expressed as follows:
NK cells obtained by eliminating CD3-positive cells from peripheral blood mononuclear cells derived from a healthy human using CD3 beads (e.g., CliniMACS CD3, Miltenyi Biotech, catalog no. 130-017-601), LD column (e.g., Miltenyi Biotech, catalog no. 130-042-901), and a separation buffer (e.g., PBS containing 0.5% human AB serum (inactivated), and 2 mM EDTA), and culturing the obtained cell population for 14 days in an appropriate medium (e.g., Cosmedium 008 supplemented with 5% human AB serum (inactivated)), and having the following characteristics (1) and (3):

    (1) CD16-positivity, CD56-high expression, and CD57-negativity, and
    (3) Cytotoxic activity of 50% or higher when the NK cells are used as effector cells (E) for K562 cells as target cells (T) in co-culture at a mixing ratio (E:T) of 1:1.

For details of the characteristics, and more specific production methods of the highly active NK cells of [1], Japanese Patent Publication (Kokai) No. 2018-193303 can be referred to.

[2] The following cells:

CCR5-positive, CCR6-positive, CXCR3-positive and CD3-negative cells.
The cells of [2] may also show high CD11c expression.
The cells of [2] can also be expressed as follows
CCR5-positive, CCR6-positive, CXCR3-positive, integrin $\alpha$1-positive, integrin $\alpha$3-positive, integrin $\beta$3-negative, and CD3-negative cells. Alternatively, CCR5-positive, CCR6-positive, CXCR3-positive, highly CD11a-expressing, highly CD11c-expressing, and CD3-negative cells, of which high expressions are determined by comparison with the expressions in a population of NK cells obtained from peripheral blood that have not been substantially cultured.
According to the studies of the inventors of the present invention, the cells of [2] show extremely high cytotoxic activity against solid tumors forming tumor masses. For details of the characteristics, and more specific production methods of the highly active NK cells of [2], Japanese Patent Publication (Kokai) No. 2019-170176 can be referred to.

[3] Highly active NK cells obtainable by the following method:

To mononuclear cells obtained from fresh peripheral blood or frozen apheresis blood, add CD3 beads (e.g., CliniMACS CD3, Miltenyi Biotech, 130-017-601 (5 $\mu$L per $1 \times 10^7$ cells)), and further CD34 beads (e.g., CliniMACS CD34, Miltenyi Biotech, 130-017-501 (2.5 $\mu$L per $1 \times 10^7$ cells)) in the case of using mononuclear cells obtained from frozen apheresis blood, suspend them, incubate the suspension at 4°C for 15 minutes, then add a separation buffer (e.g., PBS containing 0.5% human type AB serum (inactivated at 56°C for 30 minutes), and 2 mM EDTA), suspend them well, and centrifuge the suspension. Remove the supernatant, and suspend the cells in 0.5 mL of the separation buffer in such a number that the cell number in one LD column (e.g., Miltenyi Biotech, 130-042-901) should be $1 \times 10^8$ cells at the maximum. After adding 2 mL of the separation buffer to the LD column beforehand, add the cell suspension to the LD column, and collect eluate from the LD column. Add additional 1 mL of the separation buffer to the LD column, and collect eluate. After centrifuging the collected solution and removing the supernatant, suspend the cells in an appropriate medium (e.g., KBM501 medium containing 5% human AB serum (inactivated at 56°C for 30 minutes) or 5% UltraGRO (AventaCell, HPCPLCRL10) supplemented with 2 U/mL of sodium heparin) at a density of $5 \times 10^5$ cells/mL in the case of using fresh peripheral blood, or $1 \times 10^6$ cells/mL in the case of using frozen apheresis blood, and culture them up to day 14, with appropriately changing the medium.
For the specific production method of the highly active NK cells of [3], the section of Examples in this description can be referred to.
[4] Cells obtained by obtaining any of the cells of [1] to [3] with adding any selected from the group consisting of IL-12, IL-15, and IL-18 at such a concentration that the purpose of the present invention can be achieved together with or instead of IL-2 at the time of culture. For the specific production method of such cells, Non-patent document 2 mentioned above can be referred to.
In the following explanations, the present invention may be explained with reference to the case of using highly active NK cells as an example, but those skilled in the art can also understand other cases using cells subjected to an in vitro activation operation using some cytokine in accordance with the explanations.

(Cytotoxic activity)

For the present invention, the term activity or cytotoxic activity used for highly active NK cells, and so forth refers to an ability of subject cells (effector cells, E) to lyse target cells (T), unless especially stated. Cytotoxic activity can be expressed as the percentage of target cells killed by effector cells, and is calculated in accordance with the following equation.

$$(\text{Cell death observed in co-culture with effector cells - Spontaneous cell death (negative control))/(Maximum cell death (positive control) - Spontaneous cell death (negative control)) x 100}$$

When cytotoxic activity is measured, in general, the mixing ratio of the effector cells to the target cells (E:T) and the time of co-culture of effector cells and target cells can be appropriately determined according to the degree of cytotoxic activity of the effector cells, etc., and depending on the types of cells to be used and the intensity of activity. When NK cells are used as the effector cells, target cells may be, but are not limited to, K562 cells, acute myeloid leukemia cells, and chronic myeloid leukemia cells. The effector cells and target cells, and live cells and dead cells can be distinguished and quantified by using reagents such as antibodies labeled with a radioactive substance, fluorescent dye, or the like. When NK cells are used as the effector cells, the cytotoxic activity can be measured by using K562 cells as the target cells with the conditions of, for example, E:T of 1:0.05 to 10, preferably 1:0.1 to 5, and an incubation time of 0.5 to 18 hours, preferably 1 to 12 hours.

For the present invention, the expression that the activity of NK cells or the like is high means that the cytotoxic activity is 50% or higher when the target cells are K562 cells, and the cells are mixed at E:T of 2:1 and co-cultured for 1 to 3 hours, more specifically 2 hours, unless especially stated. The activity should be preferably 60% or higher, more preferably 70% or higher.

[Collection]

According to the present invention, prior to the freezing step described later, highly active NK cells or the like to be frozen are collected from the culture system. Collection can be performed by centrifuging the culture to separate the cells from the culture medium. If necessary, EDTA may be added at an appropriate concentration to the culture system to detach adhered cells from the surface of the culture vessel. The surface of the culture vessel may also be washed with an appropriate solution after the cells were detached to further obtain remaining cells. The obtained cells are washed with an appropriate solution and suspended in an appropriate solution, if necessary.

In the collection step, solutions such as culture medium, isotonic solution, and buffer may be used to detach and wash the cells. Examples of usable media include KBM501 medium, Cosmedium 008, FKCM101, CellGro SCGM medium, X-VIVO15 medium, Gibco (registered trademark) CTS (registered trademark) AIM V (registered trademark) Medium, CTS OpTmizer T Cell Expansion Basal Medium, IMDM, MEM, DMEM, and RPMI 1640. Isotonic solution refers to a solution with an osmolarity approximately equal to the osmolarity of body fluid (plasma) (285 $\pm$ 5 mOsm/L), and for the present invention, a solution with an osmolarity of 285 $\pm$ 13 mOsm/L. For example, the osmolality of Plasma-Lyte A is 294 mOsm/L, and that of PBS (-) is 280 $\pm$ 4 mOsm/L (freezing point depression method). Examples of usable isotonic solutions include Plasma-Lyte A (Baxter), saline (physiological saline), Ringer's solution (lactated Ringer's solution, acetated Ringer's solution, hydrogencarbonated Ringer's solution, etc.) and 5% glucose solution. Examples of buffers that can be used include phosphate-buffered saline (PBS), Tris-hydrochloric acid buffer, Tris-acetic acid buffer, and HEPES buffer.

One of the preferred examples of the solution used in the collection step is a culture medium, preferably a culture medium for human lymphocytes. The culture medium for human lymphocytes may contain human serum albumin, human transferrin, recombinant human insulin, and recombinant human IL-2. Preferred examples of such a medium are KBM501 medium, FKCM101, and Cosmedium 008. The KBM501 medium contains human serum albumin, human transferrin, recombinant human insulin, and recombinant human IL-2, but no other proteins. The KBM501 medium also contains antibiotics (kanamycin), NaHCO$_3$, L-glutamine, and pH adjuster.

In the collection step, use of PBS(-) may be undesirable, because it may reduce cell viability after thawing. PBS(-) typically contains 136.9 mM sodium chloride, 2.68 mM potassium chloride, 8.1 mM disodium hydrogenphosphate, and 1.47 mM potassium dihydrogenphosphate.

[Pretreatment]

For the present invention, prior to the freezing step described later, the highly active NK cells or the like to be frozen may be subjected to a pretreatment. The pretreatment means suspending the collected cells in a solution containing an additive. The pretreatment includes collecting the cells with a solution containing an additive.

The additive used for the pretreatment can be one selected from the group consisting of a bile acid and phenylbutyric acid. Examples of bile acid are tauroursodeoxycholic acid (TUDCA), ursodeoxycholic acid (UDCA), kenodeoxycholic acid, cholic acid, hyodeoxycholic acid, deoxycholic acid, 7-oxolithocholic acid, lithocholic acid, iododeoxycholic acid, iocholic acid, taurokenodeoxycholic acid, taurodeoxycholic acid, glycoursodeoxycholic acid, taurocholic acid, glycocholic acid, and analogues and derivatives thereof. Examples of phenylbutyric acid are 4-phenylbutyric acid (4-PBA), glyceryl tri-(4-PBA), phenylacetic acid, 2-POAA-OMe, 2-POAA-NO$_2$, 2-NOAA, pharmaceutically acceptable salts, analogues, derivatives and prodrugs thereof. Particularly preferred examples of the additive used for the pretreatment are any selected from the group consisting of TUDCA and 4-PBA.

When a bile acid is used as the additive for the pretreatment, the concentration thereof may be appropriately determined, but it is preferably 100 to 5000 $\mu$M, more preferably 200 to 2500 $\mu$M, further preferably 400 to 1000 $\mu$M. A concentration in such a range is particularly suitable when TUDCA is used. When a phenylbutyric acid is used as the additive for the pretreatment, the concentration thereof may be appropriately determined, but it is preferably 1 to 1000 $\mu$M, more preferably 5 to 500 $\mu$M, even more preferably 10 to 100 $\mu$M. A concentration in such a range is particularly suitable when 4-PBA is used.

Another example of the additive for the pretreatment is dimethyl sulfoxide (DMSO). The concentration thereof may be appropriately determined, but it is preferably 0.5 to 15%, more preferably 1 to 12.5%, further preferably 2 to 10%.

The solution for the pretreatment can be a solution such as medium, isotonic solution, and buffer, like the solution used for the collection. One preferred example of the solution used in the pretreatment is a culture medium, more preferably a medium for culture of human lymphocyte, further preferably KBM501 medium, FKCM101 or Cosmedium 008. The medium used for the pretreatment may also contain human serum albumin, human transferrin, recombinant human insulin, and recombinant human IL-2, and may contain antibiotics (kanamycin), $NaHCO_3$, L-glutamine, and pH adjuster.

The time for the pretreatment is not particularly limited. After the cells are suspended for the pretreatment, the suspension may be allowed to stand for several minutes to several hours, for example, 5 minutes to 4 hours, more preferably 30 minutes to 3 hours. The suspension may be allowed to stand at ambient temperature (e.g., 1 to 30°C, typically 15 to 25°C), and may be allowed to stand in a $CO_2$ incubator (e.g., 36 to 42°C, typically 37°C).

The cell density during the pretreatment may be appropriately determined, but should be a cell density suitable for cell maintenance. Specifically, the cell density is $1 \times 10^5$ to $1 \times 10^7$ cells/mL, preferably $2 \times 10^5$ to $5 \times 10^6$ cells/mL, more preferably $5 \times 10^5$ to $2 \times 10^6$ cells/mL.

In a particularly preferred embodiment, the pretreatment consists of suspending the cells in the KBM501 medium, FKCM101 or Cosmedium 008 supplemented with 400 to 1000 $\mu$M TUDCA or 10 to 100 $\mu$M 4-PBA at a cell density of $5 \times 10^5$ to $2 \times 10^6$ cells/mL. For this treatment, the cells are preferably incubated at 37°C and 5% $CO_2$ for 30 minutes to 3 hours.

Although the pretreatment is not essential for the present invention, the pretreatment of highly active NK cells or the like with the KBM501 medium supplemented with 4-PBA or TUDCA before freezing can improve the viability (also called recovery rate) after the cells are thawed compared with the case of not performing the pretreatment.

[Freezing]

According to the present invention, the collected and preferably pretreated cells are frozen by normal procedures. Specifically, the cell count and viability are checked as required, the supernatant is removed by centrifugation, and then the cells are suspended in a cryopreservation solution at an appropriate cell density. After dispensing the cell suspension into cryopreservation containers, it is frozen in a deep freezer at -80°C, and stored. If necessary, it is cryopreserved in a liquid nitrogen tank.

Cryopreservation solutions that can be used in the present invention can contain a sodium salt, potassium salt, sugar, hydrogencarbonate, carbonate, and cryoprotectant.

Sodium salts that can be used are not particularly limited so long as a sodium salt that produces sodium ion when it is dissolved in a solvent is used, and it can be an oxoacid, halide, oxide, hydroxide, inorganic salt, organic acid salt, or the like. One kind or a combination of two or more kinds of sodium salts may be used. For the present invention, sodium chloride is preferably used when one kind of salt is used, and sodium chloride and sodium citrate are preferably used when multiple kinds of salts are used. The sodium salt content is not particularly limited, but is preferably 0.01 to 5000 mM, more preferably 0.1 to 1000 mM, further preferably 1 to 300 mM, as the final concentration of total sodium ions contained in the cryopreservation solution.

Potassium salts that can be used are not particularly limited so long as a potassium salt that produces potassium ions when it is dissolved in a solvent is used, and it can be an oxoacid, halide, oxide, hydroxide, inorganic salt, organic acid salt, or the like. One kind of potassium salt may be used, or two or more kinds of potassium salts may be used in combination. Potassium chloride is preferably used in the present invention. The potassium salt content is not particularly limited, but as a final concentration of total potassium ions contained in the cryopreservation solution, it is preferably 0.01 to 5000 mM, more preferably 0.1 to 1000 mM, further preferably 1 to 100 mM.

Hydrogencarbonates that can be used are not particularly limited so long as a hydrogencarbonate that produces hydrogencarbonate ions when it is dissolved in a solvent is used, and salts with various cations can be used. Examples include, for example, ammonium hydrogencarbonate, potassium hydrogencarbonate, calcium hydrogencarbonate, sodium hydrogencarbonate, magnesium hydrogencarbonate, and so forth. Carbonates that can be used are not particularly limited so long as a carbonate that produces carbonate ions when it is dissolved in a solvent is used, and salts with a variety of cations can be used. Examples include ammonium carbonate, potassium carbonate, calcium carbonate, sodium carbonate, barium carbonate, magnesium carbonate, and so forth. One kind of these hydrogencarbonates and/or carbonates may be used, or two or more kinds of them may be used in combination. Sodium hydrogencarbonate is preferably used in the present invention. The content of hydrogencarbonate and/or carbonate is not particularly limited, but it is preferably 0.01 to 1000 mM, more preferably 0.1 to 500 mM, further preferably 1 to 100 mM, as the final concentration of total hydrogencarbonate and carbonate ions contained in the cryopreservation solution.

The concentration ratio of sodium ions to potassium ions (sodium ions/potassium ions) in the cryopreservation solution is preferably 1/1000 to 1000/1, more preferably 1/100 to 100/1, further preferably 1/10 to 100/1, further preferably 1/1 to 100/1, further preferably 10/1 to 5011.

Usable sugars include monosaccharides, oligosaccharides, and sugar alcohols, such as glucose, galactose, fructose, mannose, xylose, arabinose as monosaccharides, trehalose, sucrose, maltose, lactose, cellobiose as oligosaccharides, xylitol, and sorbitol as sugar alcohols. One kind or a combination of two or more kinds of these sugars may be used. For the present invention, the sugar preferably consists of at least one kind of sugar selected from the group consisting of glucose, galactose, fructose, mannose, xylose, and arabinose, more preferably glucose. The content of the sugar in the cryopreservation solution is preferably 0.01 to 100 g/L, more preferably 0.1 to 100 g/L, further preferably 0.25 to 50 g/L.

Examples of usable cryoprotectants include dimethyl sulfoxide (DMSO), hydroxyethyl starch (HES), ethylene glycol, glycerol, and so forth. One kind or a combination of two or more kinds of cryoprotectants may be used. For the present invention, any selected from the group consisting of DMSO and hydroxyethyl starch is preferably used. When DMSO and hydroxyethyl starch are used together as the cryoprotectants, it is preferred that the total content thereof should be in the aforementioned range, and as for the respective concentrations, DMSO concentration is preferably 0.01 to 50%, more preferably 1 to 30%, further preferably 2 to 15%, and hydroxyethyl starch concentration is preferably 0.01 to 50%, more preferably 1 to 30%, further preferably 2 to 15%.

In a preferred embodiment of the present invention, in addition to the essential ingredients of the solution used in the method for cryopreservation of cells of the present invention described above, the solution may further contain ingredients selected from the group consisting of proteins, magnesium salts and calcium salts. Proteins that can be used include, specifically, serum albumin, serum globulin, and so forth. Serum albumin includes human serum albumin, and bovine serum albumin. For the present invention, human serum albumin is preferred. The protein content in the cryo- reservation solution is preferably 0.01 to 50%, more preferably 1 to 30%, further preferably 2 to 15%. Magnesium salts that can be used are not particularly limited so long as a magnesium salt that produces magnesium ions when it is dissolved in a solvent is used, and an oxoacid, halide, oxide, hydroxide, inorganic salt, organic acid salt, and so forth can be used. One kind of magnesium salt may be used, or two or more kinds of magnesium salts may be used in combination. Magnesium chloride is preferably used in the present invention. The magnesium salt content of the cryopreservation solution is not particularly limited, but it is preferably 0.01 to 10 mM, more preferably 0.1 to 5 mM, as the final total concentration of magnesium ions in the cryopreservation solution. Calcium salts that can be used are not particularly limited so long as a calcium salt that produces calcium ions when it is dissolved in a solvent is used, and an oxoacid, halide, oxide, hydroxide, inorganic salt, organic acid salt, or the like can be used. One kind of calcium salt may be used, or two more kinds of calcium salts may be used in combination. Calcium chloride is preferably used in the present invention. The calcium salt content of the cryopreservation solution is not particularly limited, but is preferably 0.01 to 10 mM, more preferably 0.1 to 5 mM, as the final concentration of total calcium ions in the cryopreservation solution. In addition to the above ingredients, the cryopreservation solution may also contain additional substances that are not injurious to cells, for example, vitamins, amino acids, and so forth. In addition to the above ingredients, the cryopreservation solution may also contain phosphate ions from the viewpoint of pH adjustment and buffering.

The osmolarity of the cryopreservation solution should be within such a range that the cells are not damaged during freezing. For example, the osmolarity is, for example, 500 o 8000 mOsm/L, and may be 1000 to 7500 mOsm/L, 1500 to 7000 mOsm/L, or 1800 to 5000 mOsm/L, from the viewpoints of increasing the penetration of the ingredients into cells during freezing, and inhibition of ice crystal formation. The pH of the cryopreservation solution should be in such a range that the cells are not damaged, for example, 3.0 to 10.0, more preferably 4.5 to 9.0.

For the present invention, commercially available cryopreservation solutions may be used. Examples of products that can be used include the cryopreservation solutions for cells and tissues of the CellBanker series (STEM-CELLBANKER (registered trademark)), more specifically, STEM-CELLBANKER (ZENOAQ, CB045).

The cells are preferably in the logarithmic growth phase at the time of freezing.

The cell density at the time of freezing can be appropriately determined, but it is specifically $1 \times 10^6$ to $2 \times 10^8$ cells/mL, preferably $2 \times 10^6$ to $1 \times 10^8$ cells/mL, more preferably $1 \times 10^7$ to $5 \times 10^7$ cells/mL. In one of the preferred embodiments, the cells are stored at a cell density of $4 \times 10^7$ cells/mL in a 5-mL volume container. The characteristic of the highly active NK cells that they can be frozen at a high density at the time of freezing for shipping allows to make the product more compact, and contributes to lower shipping costs.

[Thawing]

In the present invention, cryopreserved cells can be thawed by various procedures. For example, such cells can be quickly thawed by incubating the cryopreservation container containing the cells in a warm bath at 37°C or the like, with shaking the container as required. Alternatively, cryopreserved cells can be spontaneously thawed by leaving them at room temperature without any active heating after they are taken out from the freezer. After thawing, the cells are mixed with an appropriate thawing solvent I. If necessary, the supernatant can be removed by centrifugation, and the cells can be suspended in an appropriate volume of a thawing solvent II, and cultured or subjected to an activation treatment.

(Thawing solvent I)

Concerning the present invention, the solution used for thawing the cells suspended in the solution for cryopreservation and frozen, and diluting them together with the solution for cryopreservation is referred to as thawing solvent I. For cells that have been collected and cryopreserved after a pretreatment, if necessary, according to the present invention, a variety of solvents can be used as the thawing solvent I.

In one of the preferred embodiments, the thawing solvent I can contain a sodium salt, potassium salt, gluconate, and acetate. In addition, it may contain a magnesium salt.

Sodium salts that can be used in the thawing solvent I can be oxoate, halide, oxide, hydroxide, inorganic salt, organic acid salt, or the like. One kind of sodium salt or a combination of two or more kind of sodium salts may be used. The thawing solvent I preferably contains any or more selected from the group consisting of sodium chloride, sodium gluconate, and sodium acetate, and more preferably, it contains sodium chloride, sodium gluconate, and sodium acetate. The content of sodium salt in the thawing solvent I is preferably 14.0 to 200 mM, more preferably 28.0 to 182 mM, further preferably 70.0 to 168 mM, as the final concentration of total sodium ions. Alternatively, the thawing solvent I preferably contains 9.00 to 108 mM of sodium chloride, 2.30 to 27.7 mM of sodium gluconate, and 2.70 to 32.5 mM of sodium acetate.

Potassium salts that can be used in the thawing solvent I can be oxoate, halide, oxide, hydroxide, inorganic salt, organic acid salt, or the like. One kind of potassium salt or a combination of two or more kind of potassium salts may be used. The thawing solvent I preferably contains any or more selected from the group consisting of potassium chloride, potassium gluconate, and potassium acetate, and more preferably, it contains potassium chloride. The potassium salt content of the thawing solvent I is preferably 0.50 to 8.0 mM, more preferably 1.0 to 7.0 mM, further preferably 2.5 to 6.0 mM, as the final concentration of total potassium ions. Alternatively, the thawing solvent I preferably contains 0.496 to 5.96 mM of potassium chloride.

Gluconates that can be used in the thawing solvent I are not particularly limited so long as a gluconate that produce gluconate ions when it is dissolved in the solvent is used, and salts with various cations can be used. Examples include, for example, sodium gluconate, potassium gluconate, and so forth. The thawing solvent I preferably contains sodium gluconate. The gluconate content of the thawing solvent I is preferably 2.3 to 32.3 mM, more preferably 4.6 to 29.9 mM, further preferably 12.5 to 27.7 mM, as the final concentration of total gluconate ions.

Acetates that can be used in the thawing solvent I are not particularly limited so long as an acetate that produce acetate ions when it is dissolved in the solvent is used, and salts with various cations can be used. Examples include sodium acetate, potassium acetate, and so forth. The thawing solvent I preferably contains sodium acetate. The content of acetate in the thawing solvent I is preferably 2.7 to 37.8 mM, more preferably 5.4 to 35.1 mM, further preferably 13.5 to 32.5 mM, as the final concentration of total acetate ions.

Commercially available isotonic solutions may be used as the thawing solvent I. Examples of products that can be used include Plasma-Lyte A and solutions obtained by diluting it with water. Specifically, a solution containing the following ingredients can be used.

- Sodium chloride 9.00 to 108 mM
- Sodium gluconate 2.30 to 27.7 mM
- Sodium acetate 2.70 to 32.5 mM
- Potassium chloride 0.496 to 5.96 mM
- Magnesium chloride 0.148 to 1.78 mM

In another preferred embodiment, the thawing solvent I can contain only a sodium salt. Sodium salts that can be used in such thawing solvent I can be oxoacid, halide, oxide, hydroxide, inorganic salt, organic acid salt, or the like. One kind of sodium salt may be used, or two or more kinds of sodium salts may be used in combination. The thawing solvent I preferably contains only sodium chloride. The sodium salt content of the thawing solvent 1 is preferably 15.4 to 216 mM, more preferably 30.8 to 200 mM, further preferably 70.0 to 185 mM, as the final concentration of total sodium ions.

Saline or a solution obtained by diluting it with water may also be used as the thawing solvent I.

Cells suspended in the thawing solvent I together with the solution for cryopreservation can be used as they are for administration. From the viewpoint of use for administration, it is preferred that the mixture of the cryopreservation solution and thawing solvent I should be isotonic (i.e., it has an osmolarity approximately equal to that of body fluid, specifically 285 $\pm$ 13 mOsm/L). Since the solution for cryopreservation is a hypertonic solution (e.g., 1500 to 7000 mOsm/L) in a preferred embodiment, the thawing solvent I may be a low osmotic solution. Those skilled in the art can determine concentrations of the ingredients of the thawing solvent 1 in consideration of the magnitude of dilution of the solution for cryopreservation with the thawing solvent I.

If the magnitude of dilution mentioned above is high (e.g., 15 times or more), it can be said that the composition of the solution for cryopreservation has little effect on the osmolarity of the mixture, and therefore it is preferable to use an isotonic solution as the thawing solvent I. From such a viewpoint, the thawing solvent I is specifically a solution containing the followings.

- Sodium chloride 85.5 to 94.5 mM
- Sodium gluconate 21.8 to 24.2 mM
- Sodium acetate 25.6 to 28.5 mM
- Potassium chloride 4.71 to 5.21 mM
- Magnesium chloride 1.40 to 1.55 mM

Alternatively, the thawing solvent I is specifically a solution containing the following.

- Sodium chloride 146.3 to 161.7 mM

In any composition of the thawing solvent I, it is preferred that the thawing solvent I does not contain calcium ions at a concentration of 0.423 mM or higher. Further, in any composition of the thawing solvent I, it is preferred that the thawing solvent I does not contain glucose at a concentration of 5.55 mM or higher. In addition, in any composition of the thawing solvent I, it is preferred that the thawing solvent I does not contain lactate at a concentration of 27.7 mM or higher. This is because concentrations of the ions out of the aforementioned ranges may reduce viability or cytotoxic activity of thawed cells. In this context, RPMI medium, Hanks' Balanced Salt Solution (HBSS) (+), and lactate Ringer's solution may not be suitable for use as the thawing solvent I.

In any composition of the thawing solvent I, it is preferred that the thawing solvent I does not contain serum at a concentration of 40% or higher, and it is more preferred that it does not contain serum at all. This is because if the solvent contains serum, cell viability may be reduced.

Although the density of the cells suspended in the thawing solvent I can be optionally chosen, it may be a cell density suitable for maintenance of the cells or cell density suitable for administration. Specifically, it is $1 \times 10^5$ to $1 \times 10^7$ cells/mL, preferably $2 \times 10^5$ to $5 \times 10^6$ cells/mL, more preferably $5 \times 10^5$ to $2 \times 10^6$ cells/mL.

The cells can be maintained in the thawing solvent I for a relatively long period of time. After suspending the cells in the thawing solvent I, the suspension may be allowed to stand for several minutes to several hours, e.g., 5 minutes to 6 hours, more preferably 30 minutes to 4 hours. They may be allowed to stand at ambient temperature (e.g., I to 30°C, typically 15 to 25°C), or in a $CO_2$ incubator (e.g., 36 to 42°C, typically 37°C).

(Thawing solvent II)

According to the present invention, after the frozen cells are thawed by using the thawing solvent I, the cells can further be cultured or activated using a thawing solvent II. Concerning the present invention, a solution used for collecting the cells suspended in the thawing solvent I and re-suspending them for culture, activation, etc. is referred to as thawing solvent II. Various types of solvents can be used as the thawing solvent II, depending on the purpose.

[Use in pharmaceutical compositions]

The present invention provides a pharmaceutical composition containing highly active NK cells, which have been collected by an appropriate method, pretreated as required, and cryopreserved, and so forth.

The pharmaceutical composition provided by the present invention can be used for the treatment and/or prevention of various diseases susceptible to highly active NK cells, and so forth. Examples of such diseases are cancers and infectious diseases, and specifically, they include, but not limited to, skin cancer, oral cancer, gallbladder cancer, bile duct cancer, lung cancer, liver cancer, stomach cancer, colon cancer, pancreatic cancer, kidney cancer, ovarian cancer, bladder cancer, prostate cancer, neuroblastoma, leukemia, and infectious diseases caused by viruses, bacteria or the like. The inventors of the present invention confirmed the effect of use of cells frozen and thawed by the method of the present invention on animal models of colon cancer, which should otherwise die within 30 days without any treatment.

A cell therapy using the pharmaceutical composition of the present invention may be performed solely, or in combination with surgical therapy, chemotherapy, radiation therapy, antibody drugs, and so forth.

The characteristics of one embodiment of the pharmaceutical composition provided by the present invention are shown below.

(Dosage form)

Injection (cell suspension)

(Ingredient and content)

Component cells: Highly active NK cells, etc.
Content: $6 \times 10^6$ to $4.8 \times 10^9$ cells/60 kg

(Sub-ingredients)

Complex electrolyte solution 10 to 45%
Sodium chloride solution 10 to 45%
20 to 30% Human serum albumin solution 5 to 30%
Dimethyl sulfoxide 2 to 15%
Others
or
Cryopreservation solution acceptable as a pharmaceutical additive 100%

(Preparation method)

The frozen composition is thawed in a thermostatic bath at 37°C or the like until it is completely thawed. Immediately after the thawing, the cells are aseptically suspended in a separately prepared isotonic solution acceptable as a pharmaceutical additive.

(Stability after thawing)

The shelf life of the composition after thawing is 6 hours, preferably 4 hours, when it is stored at room temperature.

Examples

[Methods commonly used in Reference Examples and Examples]

A) Method for culturing highly active NK cells

Raw material 1: Peripheral blood

Peripheral blood was collected from healthy volunteers, and peripheral blood mononuclear cells (PBMCs) were separated by density gradient centrifugation using Ficoll (GE Healthcare, 17144002).

Raw material 2: Frozen apheresis blood

Frozen apheresis blood (HemaCare, PB001CLP) was thawed, washed and concentrated using Lovo Cell Processing System (Fresenius Kabi) to obtain PBMCs.

To the obtained PBMCs, CD3 beads[*1] and CD34 beads[*2] (when frozen apheresis blood was used) were added, and suspended therein, the mixture was incubated at 4°C for 15 minutes, then the separation buffer[*3] was added to the cells to sufficiently suspend them, and the suspension was centrifuged at 300 x g for 10 minutes. The supernatant was removed, and the cells were suspended in 0.5 mL of the separation buffer so that the maximum cell count should be up to $1 \times 10^8$ cells per one LD column (Miltenyi Biotech, 130-042-901). After 2 mL of the separation buffer was added an LD column beforehand, the cell suspension was added to the LD column, and the eluate from the LD column was collected. Further, 1 mL of the separation buffer was added to the LD column, and the eluate was collected. The column was then washed with 1 mL of the separation buffer, and the number of the cells in the collected liquids was counted to calculate the total cell count. The cell suspension was centrifuged at 500 x g for 5 minutes, the supernatant was removed, and then the cells were suspended in the KBM501 medium[*4] at a density of $5 \times 10^5$ cells/mL in the case of using the raw material 1 (peripheral blood), or $1 \times 10^6$ cells/mL in the case of using the raw material 2 (frozen apheresis blood). The culture was performed on a 6-well plate (Thermo Fisher Scientific, 140675), in T-75 flask (Thermo Fisher Scientific, 156499), or in adhesion culture bag (Nipro) in a $CO_2$ incubator (37°C, 5% $CO_2$). The KBM501 medium was added so that the final liquid volume on day 9 of the culture should be 6 mL per well in the case of the 6-well plate, 50 mL per flask in the case of the T-75 flask, or 500 mL per bag in the case of the bag, and incubation was performed until day 14.

Hereinafter, the cells obtained from PBMCs through the aforementioned culture step will be referred to as "highly activated NK cell-like CD3-negative cells", or simply as "highly active NK cells".

* 1: CliniMACS CD3, Miltenyi Biotech, 130-017-601 (5 μL per $1 \times 10^7$ cells).

*2: CliniMACS CD34, Miltenyi Biotech, 130-017-501 (2.5 μL per $1 \times 10^7$ cells).

*3: 0.5% Human type AB serum (CosmoBio, 12181301, inactivated at 56°C for 30 minutes), PBS (Nacalai Tesque, 14249-24) containing 2 mM EDTA (Thenno Fisher Scientific, 15575-020).

*4: KBM501 (Kohjin Bio, 16025015) containing either 5% human type AB serum (CosmoBio, 12181301, inactivated at 56°C for 30 minutes) or 5% UltraGRO (AventaCell, HPCPLCRL10) supplemented with 2 U/mL heparin sodium (Nipro).

B) Collection method of highly active NK cells

Collection method 1: Conventional method

On day 14 of the culture, the culture medium was collected, further the cells adhering to the culture vessel was detached by adding 1 mM EDTA to the culture vessel, and after the detached cells were collected, the culture vessel was washed with PBS (Nacalai Tesque, 14249-24). All the cell collection liquids were centrifuged, and then the cells were washed with and resuspended in PBS.

Collection method 2: Method of the present invention

On day 14 of the culture, the culture medium was collected, further the cells adhering to the culture vessel was detached by adding 1 mM EDTA to the culture vessel, and after the detached cells were collected, the culture vessel was washed with the KBM501 medium. All the cell collection liquids were centrifuged, and then the cells were washed with and resuspended in the KBM501 medium.

C) Method for staining with 7-AAD

The highly activated NK cells ($1 \times 10^5$ cells/well) thawed under each condition were centrifuged on a 96-well plate (IWAKI, 4870-800SP), the supernatant was removed, and then a 7-amino-actinomycin D (7-AAD) solution diluted with PBS (Beckman Coulter, A 07704) was added to the cells to suspend them, and the suspension was incubated at room temperature (room temperature means 15 to 25°C, the same shall apply in the following experiments) for 20 minutes. The stained cells were measured by using a flow cytometer (BD LSR Fortessa, BD Biosciences).

[Reference Example]

The highly active NK cells obtained by the procedures of the culture method and the collection method 1 for highly active NK cells, PBMCs, K562 (human chronic myeloid leukemia cell line), and THP-1 (human acute monocytic leukemia cell line) were reacted with Mito-FerroGreen (Dojin Chemical Laboratory, M489), and measurement was performed by using a flow cytometer (BD LSR Fortessa, BD Biosciences).

As shown in Fig. 1-1, the highly active NK cells had a high iron content. On the basis of morphological observation results and time to death, it was supposed that the marked reduction of the viability of the highly active NK cells during freeze-thawing is caused by 5 kinds of mechanisms: ferroptosis (oxidative stress), apoptosis, autophagy, necrosis, and necroptosis. The inhibitors for each mechanism are listed below.

[Table 1]

| Ferroptosis (oxidative stress) | Apoptosis |
| --- | --- |
| Liproxstatin | zVAD |
| Co-enzyme Q10 | |
| Tocopherol | **Autophagy** |
| Ferrostatin-1 | Chloroquine |

(continued)

| Ferroptosis (oxidative stress) | Apoptosis |
|---|---|
| DFO (Deferoxamin) | |
| CPX (Ciclopirox olamine) | **Necrosis** |
| Ascorbate | IM-54 |
| Oxaloacetic acid | |
| $\alpha$-ketoglutaric acid | **Necroptosis** |
| GSH (Glutathione) | Necrostatin |
| NAC | |

Number of viable cells of highly active NK cells obtained by the procedures described as the culture method and collection method 1 for highly active NK cells was counted, and $1 \times 10^7$ cells were suspended in 1 mL of STEM-CELLBANKER (ZENOAQ, CB045), and frozen at -80°C. The NK cells frozen for 48 hours or longer were thawed at 37°C in a water bath, then diluted 10-fold in the solvents mentioned below, left to stand at the temperatures for the times mentioned below, then observed and photographed under an optical microscope.

<1> Diluted in the KBM501 medium, and left to stand at 37°C for 3 hours.
<2> Diluted in the KBM501 medium containing Z-Vad, and left to stand at 37°C for 3 hours.
<3> Diluted in the KBM501 medium containing methylprednisolone, and left to stand at 37°C for 3 hours.
<4> Diluted in the KBM501 medium containing dexamethasone, and left to stand at 37°C for 3 hours.
<5> Diluted in the KBM501 medium, and left to stand at 4°C for 3 hours.
<6> Diluted in the KBM501 medium containing methylprednisolone, and left to stand at 4°C for 3 hours, and then at 37°C for 3 hours.
<7> Diluted in the KBM501 medium containing dexamethasone, and left to stand at 4°C for 3 hours, and then at 37°C for 3 hours.

The results are shown in Fig. 1-2. It was confirmed that the viability of the highly active NK cells was markedly decreased by the freeze-thawing. This was not improved by the addition of methylprednisolone, dexamethasone, or Z-Vad after the freeze-thawing.

The highly active NK cells obtained by the procedures described as the culture method and collection method 1 for highly active NK cells were collected, then frozen and thawed by the methods described.

The conditions and results of freeze-thawing are summarized in the following tables. Under the conditions tested, the cells frozen after washing with PBS were unresponsive to all methods for improving viability. It was considered difficult to obtain the desired effect by the generally known cell protection methods.

[Table 2-1]

| Additive | Concentration | Pretreatment before freezing | Addition to solution for freezing (SBC) | Solution for thawing | Result |
|---|---|---|---|---|---|
| Liproxstatin-1 | 100nM | - | + | Plasma-Lyte A or KBM containing inhibitor | × |
| CoQ10 | 10μM | - | + | Plasma-Lyte A or KBM containing inhibitor | × |
| $\alpha$-Tocopherol | 1μM | - | + | Plasma-Lyte A or KBM containing inhibitor | × |
| Liproxstatin-1 | 100nM | | | | |
| CoQ10 | 10μM | - | + | Plasma-Lyte A or KBM containing inhibitor | × |
| $\alpha$-Tocopherol | 1μM | | | | |

(continued)

| Additive | Concentration | Pretreatment before freezing | Addition to solution for freezing (SBC) | Solution for thawing | Result |
|---|---|---|---|---|---|
| Liproxstatin-1 | 100nM | - | | KBM containing inhibitor | × |
| CoQ10 | 10μM | | | | |
| α-Tocopherol | 1μM | | | | |
| Ferrostatin-1 | 1μM | | | | |
| Ciclopirox Olamine (CPX) | 10μM | | | | |
| Deferoxamine mesylate (DFO) | 100μM | | | | |
| Ascorbic acid | 1μM | | | | |
| Glutathione | 1mM | | | | |
| Necrostatin-1 | 1μM | - | | KBM containing inhibitor | × |
| IM-54 | 5μM | - | | KBM containing inhibitor | × |
| Z-VAD | 20μM | - | - | KBM containing inhibitor | × |
| Liproxstatin-1 | 100nM | - | | KBM containing inhibitor | × |
| CoQ10 | 10μM | | | | |
| α-Tocopherol | 1μM | | | | |
| Ferrostatin-1 | 1μM | | | | |
| Ciclopirox Olamine (CPX) | 10μM | | | | |
| Deferoxamine mesylate (DFO) | 100μM | | | | |
| Ascorbic acid | 1μM | | | | |
| Glutathione | 1mM | | | | |
| Necrostatin-1 | 1μM | | | | |
| IM-54 | 5μM | | | | |
| Z-VAD | 20μM | | | | |
| Liproxstatin-1 | 100nM | O/N | + | KBM containing inhibitor | × |
| CoQ10 | 10μM | | | | |
| α-Tocopherol | 1μM | | | | |
| Ferrostatin-1 | | | | | |
| Ciclopirox Olamine (CPX) | 10μM | | | | |
| Chloroquine diphosphate (CQ) | 100μM | O/N | + | KBM containing inhibitor | × |

(continued)

| Additive | Concentration | Pretreatment before freezing | Addition to solution for freezing (SBC) | Solution for thawing | Result |
|---|---|---|---|---|---|
| Liproxstatin-1 | 100nM | O/N | + | KBM containing inhibitor | × |
| CoQ10 | 10μM | | | | |
| α-Tocopherol | 1μM | | | | |
| Ferrostatin-1 | 1μM | | | | |
| Ciclopirox Olamine (CPX) | 10μM | | | | |
| Chloroquine diphosphate (CQ) | 100μM | | | | |
| Z-VAD | 10μM | | | | |

[Table 2-2]

| | | | | | |
|---|---|---|---|---|---|
| Liproxstatin-1 | 100nM | 5days | | | × |
| CoQ10 | 10μM | | | | |
| α-Tocopherol | 1μM | | | | |
| Ferrostatin-1 | 1μM | | | | |
| Ciclopirox Olamine (CPX) | 10μM | | | | |
| Deferoxamine mesylate (DFO) | 100μM | | | | |
| Ascorbic acid | 1μM | | | | |
| Glutathione | 1mM | | | | |
| Necrostatin-1 | 1μM | 5days | + | KBM containing inhibitor | × |
| IM-54 | 5μM | | | | |
| Z-VAD | 20μM | 5days | + | KBM containing inhibitor | × |
| Liproxstatin-1 | 100nM | 5days | | | × |
| CoQ 10 | 10μM | | | | |
| α-Tocopherol | 1μM | | | | |
| Ferrostatin-1 | 1μM | | | | |
| Ciclopirox Olamine (CPX) | 10μM | | | | |
| Deferoxamine mesylate (DFO) | 100μM | | | | |
| Ascorbic acid | 1μM | | | | |
| Glutathione | 1mM | | | | |
| Necrostatin-1 | 1μM | | | | |
| IM-54 | 5μM | | | | |
| Z-VAD | 20μM | | | | |
| Liproxistatin-1 | 100nM | O/N | + | KBM containing inhibitor | × |
| CoQ10 | 10μM | O/N | + | KBM containing inhibitor | × |
| Ferrostatin-1 | 1μM | O/N | + | KBM containing inhibitor | × |
| Ciclopirox Olamine (CPX) | 10μM | O/N | + | KBM containing inhibitor | × |

(continued)

| | | | | | |
|---|---|---|---|---|---|
| Deferoxamine mesylate (DFO) | 100$\mu$M | O/N | + | KBM containing inhibitor | × |
| $\alpha$-Tocopherol | 1$\mu$M | O/N | / | | - |
| Ascorbic acid | 1$\mu$M | | | | |
| Glutathione | 1mM | | | | |
| Oxaloacetic acid | 10mM | | | | |
| Ketoglutaric acid | 10mM | | | | |
| Pyruvic acid | 10mM | | | | |
| Liproxistatin-1 | 100nM | 5days | + | KBM containing inhibitor | × |
| CoQ10 | 10$\mu$M | | | | |
| $\alpha$-Tocopherol | 1$\mu$M | | | | |
| Ferrostatin-1 | 1$\mu$M | | | | |
| Ciclopirox Olamine (CPX) | 10$\mu$M | | | | |
| Deferoxamine mesylate (DFO) | 100$\mu$M | 5days | + | KBM containing inhibitor | × |
| NAC | 200$\mu$M | 5days | + | KBM containing inhibitor | x |
| NAC | 200$\mu$M | 5days | - | KBM | - |
| IM-54 | 5$\mu$M | 5days | + | KBM containing inhibitor | x |
| NAC | 200$\mu$M | 5days | - | KBM | x |
| Z-VAD | 20$\mu$M | 5days | + | KBM containing inhibitor | x |

[Table 2-3]

| | | | | | |
|---|---|---|---|---|---|
| Liproxistatin-1 | 100nM | O/N | + | KBM containing inhibitor | × |
| CoQ10 | 10$\mu$M | O/N | + | KBM containing inhibitor | × |
| Ferrostatin-1 | 1$\mu$M | O/N | + | KBM containing inhibitor | × |
| Deferoxamine mesylate (DFO) | 100$\mu$M | O/N | + | KBM containing inhibitor | × |
| NAC | 200$\mu$M | O/N | + | KBM containing inhibitor | × |
| Ascorbic acid | 1mM | 5days | + | KBM containing inhibitor | × |
| Ascorbic acid | 100$\mu$M | 5days | + | KBM containing inhibitor | × |
| Ascorbic acid | 1$\mu$M | 5days | + | KBM containing inhibitor | × |
| Ascorbic acid | 100$\mu$M | 5days | + | KBM containing inhibitor | × |
| $\alpha$-Tocopherol | 100$\mu$M | | | | |
| NAC | 200$\mu$M | | | | |
| $\alpha$-Tocopherol | 100$\mu$M | 5days | + | KBM containing inhibitor | × |

(continued)

| | | | | | |
|---|---|---|---|---|---|
| Ascorbic acid | 100μM | O/N | + | KBM containing inhibitor | × |
| NAC | 200μM | O/N | + | KBM containing inhibitor | × |
| α-Tocopherol | 100μM | O/N | + | KBM containing inhibitor | × |
| Ascorbic acid | 100μM | O/N | + | KBM containing inhibitor | × |
| NAC | 200μM | | | | |
| α-Tocopherol | 100μM | | | | |
| CoQ10 | 10μM | O/N | + | KBM containing inhibitor | × |
| Ferrostatin-1 | 1μM | | | | |
| α-Tocopherol | 100μM | | | | |
| NAC | 200μM | | | | |
| z-VAD | 20μM | O/N | + | KBM containing inhibitor | × |
| Chloroquine diphosphate (CQ) | 10μM | O/N | + | KBM containing inhibitor | × |
| IM-54 | 5μM | O/N | + | KBM containing inhibitor | × |
| Necrostatin-1 | 1μM | O/N | + | KBM containing inhibitor | × |
| CoQ10 | 10μM | O/N | + | KBM containing inhibitor | × |
| Ferrostatin-1 | 1μM | | | | |
| α-Tocopherol | 100μM | | | | |
| NAC | 200μM | | | | |
| z-VAD | 20pM | | | | |
| Chloroquine diphosphate (CQ) | 10μM | | | | |
| IM-54 | 5μM 5pM | | | | |
| Necrostatin-1 | 1μM | | | | |
| IL-2 | 10.000IU/ml | O/N | + | KBM | × |
| UG (-) | 5% v/v | O/N | + | KBM | × |
| - | - | O/N | Cell count 9.3E+06/200 μL | KBM | × |
| Salubrinal | 100μM | O/N | + | KBM containing inhibitor | × |
| Cydoheximide | 1μM | O/N | + | KBM containing inhibitor | × |
| Salubrinal | 100μM | O/N | + | KBM containing inhibitor | × |
| Cycloheximide | 1μM | | | | |
| 1% O2 | 1% | O/N | - | KBM | × |
| | | | | | |
| | | ※ 5days: day9-14 | | | |

(continued)

| | | | |
|---|---|---|---|
| | ※ O/N: day14-15 | | |

[Example 1]

(1-1) Numbers of viable cells of highly active NK cells obtained from each of two healthy volunteers by the procedures described as the culture method and collection method 1 for highly active NK cells were counted, and $1 \times 10^7$ cells and $8 \times 10^6$ cells of each were suspended in 1 mL of STEM-CELLBANKER (ZENOAQ, CB045), and frozen at -80°C. The NK cells frozen for 48 hours or longer were thawed in a water bath at 37°C, and $1 \times 10^5$ cells were dispensed on a 96-well plate (IWAKI) immediately after the thawing. The cells were diluted 10-fold with and suspended in the KBM501 medium, Lactoringer (Lactated Ringer's Solution, Fuso Pharmaceutical Industries) containing 3500 units/mL of IL-2 (Celeuk (registered trademark) For Injection 40, Takeda Pharmaceutical) and 139.9 mM maltose, Plasma-Lyte A (Baxter) containing 3500 units/mL of IL-2, and KBM501 medium containing 5.02 mg/mL of sodium gluconate (Nakalai, 16720-22), and incubated at 37°C for 3 hours under 5% $CO_2$. The cells dispensed immediately after thawing were centrifuged immediately after they were dispensed, and the cells of the other four groups were centrifuged after incubation of 3 hours, measurement was performed by the method for staining with 7-AAD described above, and the results were analyzed with the FlowJo software (FLOWJO, LLC) to calculate viable cell rates.

(1-2) In addition, $5 \times 10^6$ cells of highly active NK cells obtained from one healthy volunteer by the procedures described as the culture method and collection method 1 for highly active NK cells were frozen and thawed in the same manner, treated in the same manner, measurement was performed in the same manner, and the results were analyzed with the FlowJo software to calculate viable cell rate.

The results are shown in Figs. 1-3 and 1-4. The viability of frozen highly active NK cells was improved by dilution with Plasma-Lyte A at the time of thawing. Dilution with the KBM501 medium at the time of thawing did not show improvement, even when gluconic acid (sodium gluconate is one of the ingredients contained in Plasma-Lyte A) was added.

[Example 2]

Number of viable cells of highly active NK cells obtained by the procedures described as the culture method and collection method 1 for highly active NK cells was counted, and $5 \times 10^6$ cells and $1 \times 10^7$ cells were suspended in 1 mL of STEM-CELLBANKER, respectively, and frozen at -80°C. The NK cells frozen for 48 hours or longer were thawed at 37°C in a water bath, and then $1 \times 10^5$ of the cells were dispensed on a 96-well plate immediately after the thawing. The cells were diluted 10-fold with and suspended in the KBM501 medium, Lactoringer containing 3500 units/mL of IL-2, Plasma-Lyte A containing 3500 units/mL of IL-2, and Plasma-Lyte A containing 40% serum (human type AB serum), and incubated at 37°C for 3 hours under 5% $CO_2$. The cells dispensed immediately after thawing were centrifuged immediately after they were dispensed, and the cells of the other four groups were centrifuged after incubation of 3 hours, measurement was performed by the method for staining with 7-AAD described above, and the results were analyzed with the FlowJo software (FLOWJO, LLC) to calculate viable cell rates.

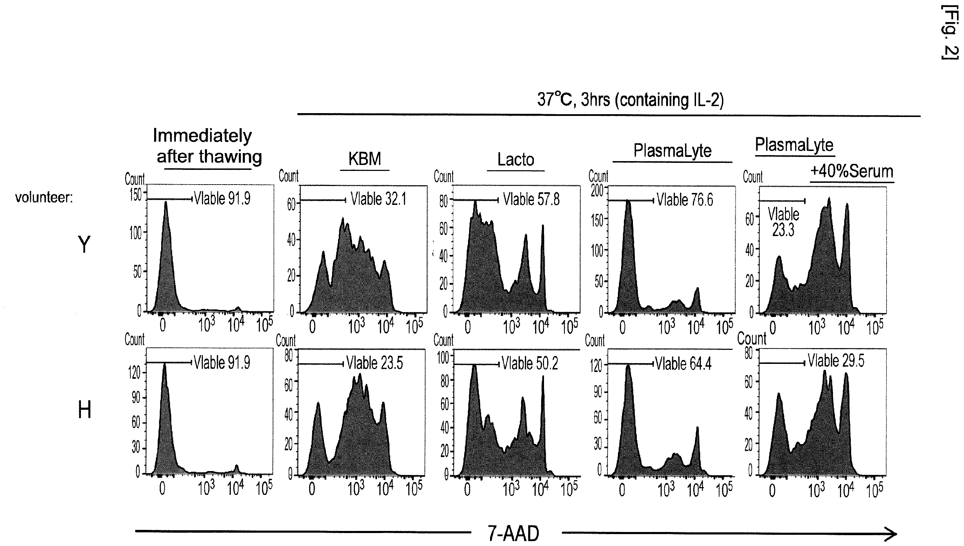

The results are shown in Fig. 2. The viability of the frozen highly active NK cells was improved when they were diluted with Plasma-Lyte A at the time of thawing, but worsened when they were diluted with Plasma-Lyte A containing 40% serum (human type AB serum).

[Example 3]

Number of viable cells of highly active NK cells obtained by the procedures described as the culture method and collection method 1 for highly active NK cells was counted, and $1 \times 10^7$ of the cells were suspended in 1 mL of STEM-CELLBANKER, and frozen at -80°C. The NK cells frozen for 48 hours or longer were thawed at 37°C in a water bath, and then diluted in Plasma-Lyte A containing 3500 units/mL of IL-2 (Immunace, Shionogi & Co., Ltd.) (solvent (1)) or the KBM501 medium (solvent (2)), and the number and percentage of viable cells, and cytotoxic activity (% Lysis) were calculated for each of the groups described below.

<0> Separated from STEM-CELLBANKER as cells immediately after thawing.
<1> Diluted 10-fold in the solvent (1), left standing at 37°C for 1 hour, and then after addition of an equal volume of the KBM501 medium, incubated at 37°C for 3 hours.
<2> Diluted 10-fold in the solvent (1), left standing at 37°C for 1 hour, and then after addition of an equal volume of Lactoringer containing IL-2, incubated at 37°C for 3 hours.

<3> Diluted 10-fold in the solvent (1), left standing at 37°C for 1 hour, and then after addition of an equal volume of the KBM501 medium, incubated at 37°C overnight.

<4> Diluted 10-fold in the solvent (1), left standing at 37°C for 1 hour, and then after addition of an equal volume of Lactoringer containing IL-2, incubated at 37°C overnight.

<5> Diluted 10-fold in the solvent (1), and left standing at 37°C for 3 hours.

<6> Diluted 10-fold in the solvent (2), and left standing at 37°C for 3 hours.

<7> Diluted 10-fold in the solvent (2), and left standing at 37°C overnight.

(Calculation of cytotoxic activity rate)

For the measurement of cytotoxic activity, a group of the K562 cells reacted with the NK cells, a group consisting solely of the K562 cells as a negative control, and a group of the K562 cells fixed with 10% formalin as a positive control were prepared.

«NK cells»

After incubation for the times indicated for the groups, the cells were collected, and prepared at a density of $2 \times 10^6$ cells/mL in 10% FBS/RPMI 1640.

«K562 cells»

The K562 cells (human chronic myelogenous leukemia cell line) were suspended in serum-free RPMI 1640 medium, stained with PKH26 Red Fluorescent Cell Linker Kit (Sigma, PKH26GL-1KT), and finally prepared at a density of $2 \times 10^6$ cells/mL in 10% FBS/RPMI 1640.

The NK cells and K562 cells were added and mixed in wells of 96-well plate (IWAKI, 4870-800SP) at a cell ratio of 1:1, and allowed to react at 37°C for 2 hours under 5% $CO_2$. After the reaction, the plate was centrifuged (500 x g, 5 minutes), and the supernatant was removed. Then, a 7-AAD solution diluted with PBS was added to the cells to suspend the cells, and the suspension was incubated at room temperature for 20 minutes. Measurement was performed by using a flow cytometer, and the results were analyzed with the FlowJo software to calculate the cytotoxic activity rate (% Lysis)[*5].

$$*5: \text{Cytotoxic activity rate} = (\text{SKOV3 cell dead cell rate} - \text{Negative control dead cell}$$
$$\text{rate})/(\text{Positive control dead cell rate} - \text{Negative control dead cell rate}) \text{ x } 100$$

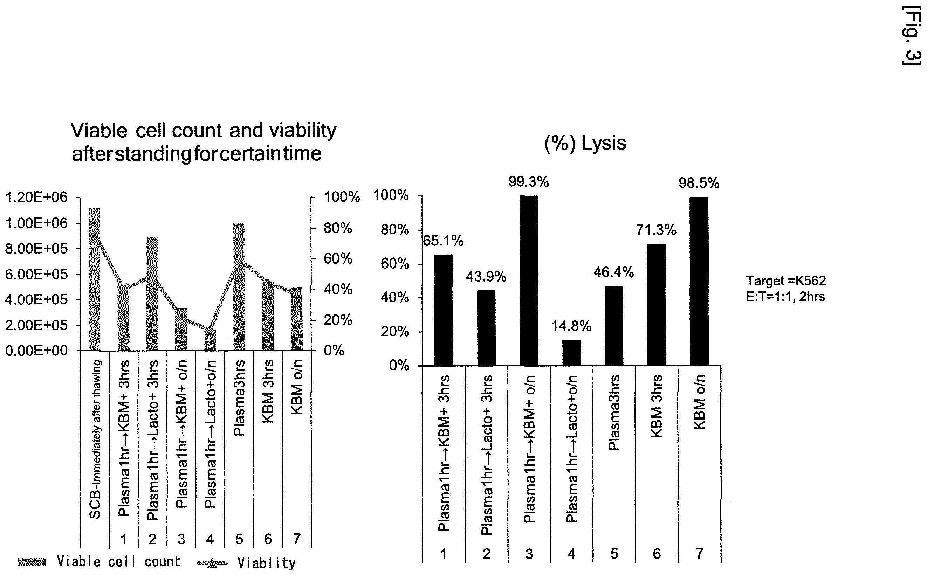

The results are shown in Fig. 3. The number of viable cells and viability of the frozen and thawed highly active NK cells correlated with each other. In addition, there was also observed an inverse correlation tendency between the number of viable cells or viability and cytotoxic activity. Furthermore, viability decreased when the solution for treating the frozen and thawed cells was changed from Plasma-Lyte A to other solutions.

[Example 4]

Number of viable cells of highly active NK cells obtained by the procedures described as the culture method and collection method 1 for highly active NK cells was counted, and the cells were suspended in the six kinds of solvents mentioned below at a density of $1 * 10^6$ cells/mL, and inoculated onto a low-adsorption 6-well plate (IWAKI, 4810-800SP) for pretreatment before freezing. As the pretreatment solvents, (1) the KBM501 medium containing 10 $\mu$M 4-phenylbutyric acid (4-PBA, Tokyo Kasei Kogyo, P0643), (2) KBM501 medium containing 100 $\mu$M 4-phenylbutyric acid, (3) Plasma-Lyte A, (4) Plasma-Lyte A containing 3000 units/mL of IL-2 (Immunace, Shionogi & Co., Ltd.), (5) Plasma-Lyte A containing 10 $\mu$M salubrinal (TOCRIS, 2347), and (6) PBS containing 23 mM sodium gluconate were used, and the cells suspended in each solvent were incubated at 37°C under 5% $CO_2$ in the case of the groups of (1) and (2), or at room temperature for 2 hours in the case of the groups of (3) to (6). After the pretreatment, the cells were suspended in STEM-CELLBANKER at a density of $1 \times 10^7$ cells/mL on the basis of the cell count at the time of the treatment, and cryopreserved at -80°C. The pretreated NK cells frozen for 48 hours or longer were thawed at 37°C in a water bath, and diluted as follows. After the thawing, all the dilution and incubation operations were performed on a low-absorption 6-well plate.

«Pretreatment (1), KBM501 medium containing 10 $\mu$M 4-phenylbutyric acid»

The cells were diluted 11-fold in Plasma-Lyte A containing 1000 units/mL of IL-2, or Plasma-Lyte A containing 1000 units/mL of IL-2 and 100 μM 4-PBA, incubated at room temperature for 1 hour, and then diluted 5-fold in the KBM501 medium.

«Pretreatment (2), KBM501 medium containing 100 μM 4-phenylbutyric acid»

The cells were diluted 11-fold in Plasma-Lyte A, incubated at room temperature for 1 hour, then diluted 5-fold in the RPMI medium*6 or RPMI medium containing 2400 units/mL of IL-2.

*6: RPMI medium (Nacalai Tesque, 30264-56) supplemented with 10% FBS (Sigma, 172012-500ML, inactivated at 56°C for 30 minutes).

«Pretreatment (3), Plasma-Lyte A»

The cells were diluted 11-fold in Plasma-Lyte A containing 1000 units/mL of IL-2 or Plasma-Lyte A containing 1000 units/mL of IL-2 and 100 μM 4-PBA, incubated at room temperature for 1 hour, and then diluted 5-fold in the KBM501 medium.

«Pretreatment (4), Plasma-Lyte A containing 3000 units/mL of IL-2»

The cells were diluted 11 -fold in Plasma-Lyte A, incubated at room temperature or 37°C for 1 hour under 5% $CO_2$, and then diluted 5-fold in the RPMI medium and RPMI medium containing 2400 units/mL of IL-2.

«Pretreatment (5), Plasma-Lyte A containing 10 μM salubrinal»

The cells were diluted 11-fold in Plasma-Lyte A containing 1000 units/mL of IL-2 or Plasma-Lyte A containing 1000 units/mL of IL-2 and 100 μM 4-PBA, incubated at room temperature for 1 hour, and then diluted 5-fold in the KBM501 medium.

«Pretreatment (6), PBS containing 23 mM gluconic acid»

The cells were diluted 11-fold in Plasma-Lyte A, incubated at room temperature or 37°C for 1 hour under 5% $CO_2$, and then diluted 5-fold in the RPMI medium or RPMI medium containing 2400 units/mL of IL-2.

The cells of the groups diluted in the media were incubated overnight at 37°C under 5% $CO_2$, and then the numbers of viable cells were counted. The experimental conditions are summarized below.

[Table 3]

| No. | Pretreatment for freezing | 11-Fold dilution at Thawing | 5-Fold dilution after 3 hours |
|---|---|---|---|
| 1 | 10uM 4-PBA KBM | PlasmaLyte, IL-2 | KBM, AB |
| 2 | 10uM 4-PBA KBM | PlasmaLyte 4-PBA, IL-2 | KBM, AB |
| 3 | PkasmaLyte | PlasmaLyte, IL-2 | KBM, AB |
| 4 | PkasmaLyte | PlasmaLyte 4-PBA, IL-2 | KBM, AB |
| 5 | 10uM salbrinal PlasmaLyte | PlasmaLyte, IL-2 | KBM, AB |
| 6 | 10uM salbrinal PlasmaLyte | PlasmaLyte 4-PBA, IL-2 | KBM, AB |
| 7 | 100uM 4-PBA KBM | PlasmaLyte R.T. | RPMI |
| 8 | 100uM 4-PBA KBM | PlasmaLyte R.T. | RPMI, IL-2 |
| 9 | IL-2 PlasmaLyte | PlasmaLyte R.T. | RPMI |
| 10 | IL-2 PlasmaLyte | PlasmaLyte R.T. | RPMI, IL-2 |
| 11 | IL-2 PlasmaLyte | PlasmaLyte 37°C | RPMI |
| 12 | IL-2 PlasmaLyte | PlasmaLyte 37°C | RPMI, IL-2 |
| 13 | 23mM Gluconate PBS | PlasmaLyte R.T. | RPMI |
| 14 | 23mM Gluconate PBS | PlasmaLyte R.T. | RPMI, IL-2 |
| 15 | 23mM Gluconate PBS | PlasmaLyte 37°C | RPMI |
| 16 | 23mM Gluconate PBS | PlasmaLyte 37°C | RPMI, IL-2 |

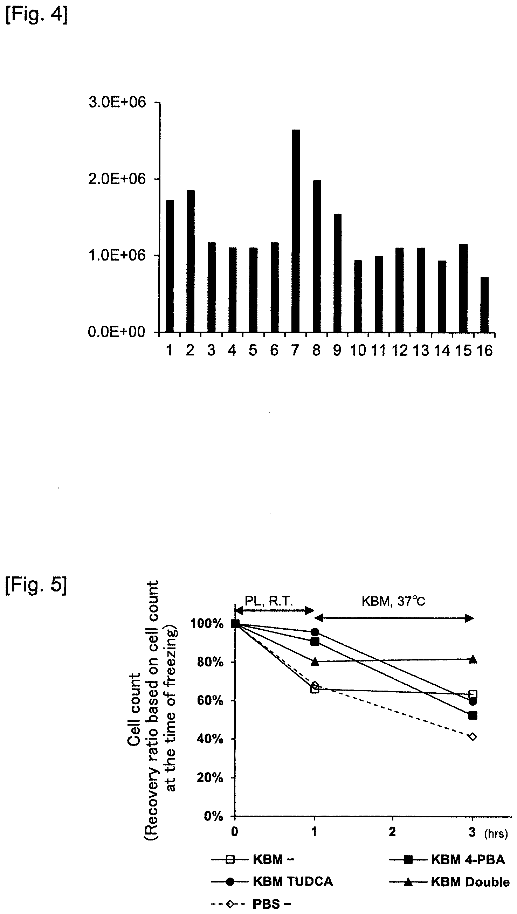

The results are shown in Fig. 4. When the highly active NK cells were treated with KBM501 supplemented with 4-PBA before the freezing, viability after the thawing was improved.

[Example 5]

Numbers of viable cells of highly active NK cells obtained by the procedures described as the culture method and collection methods 1 and 2 for highly active NK cells were counted, and the cells were suspended in various kinds of solvents at a cell density of $1 \times 10^6$ cells/mL, and inoculated onto a low-adsorption 6-well plate for pretreatment before freezing. The NK cells obtained by the collection method 1 were suspended in PBS, and incubated at room temperature for 1 hour. The NK cells obtained by the collection method 2 were suspended in (1) the KBM501 medium, (2) KBM501 medium containing 30 $\mu$M 4-phenylbutyric acid, (3) KBM501 medium containing 30 $\mu$M tauroursodeoxycholic acid dihydrate (TUDCA, Tokyo Kasei Kogyo, T1567), and (4) KBM501 medium containing 30 $\mu$M 4-phenylbutyric acid and 30 $\mu$M TUDCA, and incubated at 37°C for 2 hours under 5% $CO_2$. After the pretreatment, the cells were suspended in STEM-CELLBANKER at a density of $1 \times 10^7$ cells/mL on basis of the number of the cells at the time of the treatment, and cryopreserved at -80°C. The pretreated highly active NK cells frozen for 48 hours or longer were thawed at 37°C in a water bath, and the cells of each group were then diluted 11-fold in Plasma-Lyte A, and incubated at room temperature for 1 hour. After 1 hour, the cells were gently suspended, and number of viable cells was counted. After another 5-fold dilution in the KBM501 medium and incubation at 37°C for 2 hours under 5% $CO_2$, number of viable cells was counted, and the recovery rate based on the number of cells at the time of freezing was calculated.

The results are shown in the following table and in Fig. 5. The pretreatment of the highly active NK cells with the KBM501 medium supplemented with 4-PBA or TUDCA before freezing improved the recovery rate after the thawing.

[Table 4]

| | pre-treat | | | | | Thawing | | | | | |
|---|---|---|---|---|---|---|---|---|---|---|---|
| | base medium | Additive | Temperature | Time | Before freezing | Solvent for dilution at the time of thawing | Temperature | Time | After 1 hour | Medium for culture after thawing | After 3 hours |
| 1 | KBM, 5% UG+Hepa | - | 37°C | 2hrs | 100% | PlasmaLyteA | RT | 1hr | 66.0% | KBM, UG+Heparin | 63.6% |
| 2 | KBM, 5% UG+Hepa | 30μM 4-PBA | 37°C | 2hrs | 100% | PlasmaLyteA | RT | 1hr | 90.8% | KBM, UG+Heparin | 52.6% |
| 3 | KBM, 5% UG+Hepa | 30μM TUDCA | 37°C | 2hrs | 100% | PlasmaLyteA | RT | 1hr | 95.7% | KBM, UG+Heparin | 59.9X |
| 4 | KBM, 5% UG+Hepa | 30μM 4-PBA 30μM TUDCA | 37°C | 2hrs | 100% | PlasmaLyteA | RT | 1hr | 80.3% | KBM, UG+Heparin | 81.9% |
| 5 | PBS | - | RT | 2hrs | 100% | PlasmaLyteA | RT | 1hr | 68.2% | KBM, UG+Heparin | 41.6% |

[Example 6]

Number of viable cells of highly active NK cells obtained by the procedures described as the culture method and collection method 2 for highly active NK cells was counted, the cells were suspended at a density of $1 \times 10^6$ cells/mL in the KBM501 medium or the KBM501 medium containing TUDCA (10 µM, 30 µM, 90 µM, 270 µM, or 810 µM), inoculated onto a low-absorption 6-well plate, and incubated at 37°C for 2 hours under 5% $CO_2$ for a pretreatment before freezing. After the pretreatment, the cells were suspended in STEM-CELLBANKER at a density of $1 \times 10^7$ cells/mL on the basis of the cell count at the time of the treatment, and cryopreserved at -80°C. The pretreated NK cells frozen for 48 hours or longer were thawed at 37°C in a water bath, 10-fold volume of Plasma-Lyte A was added to the cells of each group, and the cells were incubated at room temperature for 3 hours. After 1 and 3 hours, the cells were gently suspended, and viable cells were counted. In addition, after 1 hour, the cells were diluted 5-fold in the KBM501 medium, and incubated at 37°C for 2 hours under 5% $CO_2$, and viable cell were counted. Recovery rate of viable cells after the thawing based on the cell number at the time of freezing was calculated using the number of viable cells at each time point. The cytotoxic activity of the highly active NK cells was also measured at each time point.

(Calculation of cytotoxic activity rate)

For the measurement of cytotoxic activity, a group of the NK cells reacted with the K562 cells, a group solely consisting of the K562 cells as a negative control, and a group of the K562 cells fixed with 10% formalin as a positive control were prepared.

«NK cells»

The cells were thawed and diluted by the method described above, and a necessary amount of the cells were taken on the basis of the number of viable cells at the time of freezing, and then prepared at a density of $2 \times 10^6$ cells/mL in 10% FBS/RPMI 1640.

«K562 cells»

The K562 cells were suspended in serum-free RPMI 1640 medium, stained with PKH26 Red Fluorescent Cell Linker Kit, and then prepared at a density of $2 \times 10^6$ cells/mL in 10% FBS/RPMI 1640.
The NK cells and K562 cells were added and mixed in wells of 96-well plate (IWAKI, 4870-800SP) at a cell ratio of 2:1, and allowed to react at 37°C for 2 hours under 5% $CO_2$. After the reaction, the plate was centrifuged (500 x g, 5 minutes), the supernatant was removed, then a 7-AAD solution diluted with PBS was added to suspend the cells, and the suspension was incubated at room temperature for 20 minutes. Measurement was performed by using a flow cytometer, and the results were analyzed with the FlowJo software to calculate the cytotoxic activity rate (% Lysis).
The recovery rates obtained are shown in the following table along with the pretreatment and thawing conditions. The pretreatment of the highly active NK cells with the KBM501 medium supplemented with various concentrations of TUDCA improved the recovery rate after the thawing.

[Table 5]

| pre-treat | | | | | Thawing | | | | | |
|---|---|---|---|---|---|---|---|---|---|---|
| base medium | Additive | Temperature | Time | | Solvent for thawing (10x) | Additive | Temperature | Time | Note | Recovery rate |
| KBM. 5% UG+Hepa | 810μM TUDCA | 37°C | 2hrs | 100% | PlasmaLyteA | - | RT | 1hr | - | 179.9% |
| | | | | 100% | | | | 3hrs | - | 144.7% |
| | | | | 100% | | | | 1hr+2hr | Diluted 5-fold with KBM after 1 hour | 107.6% |
| KBM, 5% UG+Hepa | 270μM TUDCA | 37°C | 2hrs | 100% | PlasmaLyteA | - | RT | 1hr | - | 154.0% |
| | | | | 100% | | | | 3hrs | - | 141.3% |
| | | | | 100% | | | | 1hr+2hr | Diluted 5-fold with KBM after 1 hour | 118.6% |
| KBM, 5% UG+Hepa | 90μM TUDCA | 37°C | 2hrs | 100% | PlasmaLyteA | - | RT | 1hr | - | 166.7% |
| | | | | 100% | | | | 3hrs | - | 171.1% |
| | | | | 100% | | | | 1hr+2hr | Diluted 5-fold with KBM after 1 hour | 108.8% |
| KBM, 5% UG+Hepa | 30μM TUDCA | 37°C | 2hrs | 100% | PlasmaLyteA | - | RT | 1hr | - | 133.1% |
| | | | | 100% | | | | 3hrs | - | 140.8% |
| | | | | 100% | | | | 1hr+2hr | Diluted 5-fold wilh KBM after 1 hour | 92.9% |
| KBM, 5% UG+Hepa | 10μM TUDCA | 37°C | 2hrs | 100% | PlasmaLyteA | - | RT | 1hr | - | 111.7% |
| | | | | 100% | | | | 3hrs | - | 156.8% |
| | | | | 100% | | | | 1hr+2hr | Diluted 5-fold with KBM after 1 hour | 103.9% |
| KBM, 5% UG+Hepa | - | 37°C | 2hrs | 100% | PlasmaLyteA | - | RT | 1hr | - | 140.3% |
| | | | | 100% | | | | 3hrs | - | 161.2% |
| | | | | 100% | | | | 1hr+2hr | Diluted 5-fold with KBM after 1 hour | 106.3% |

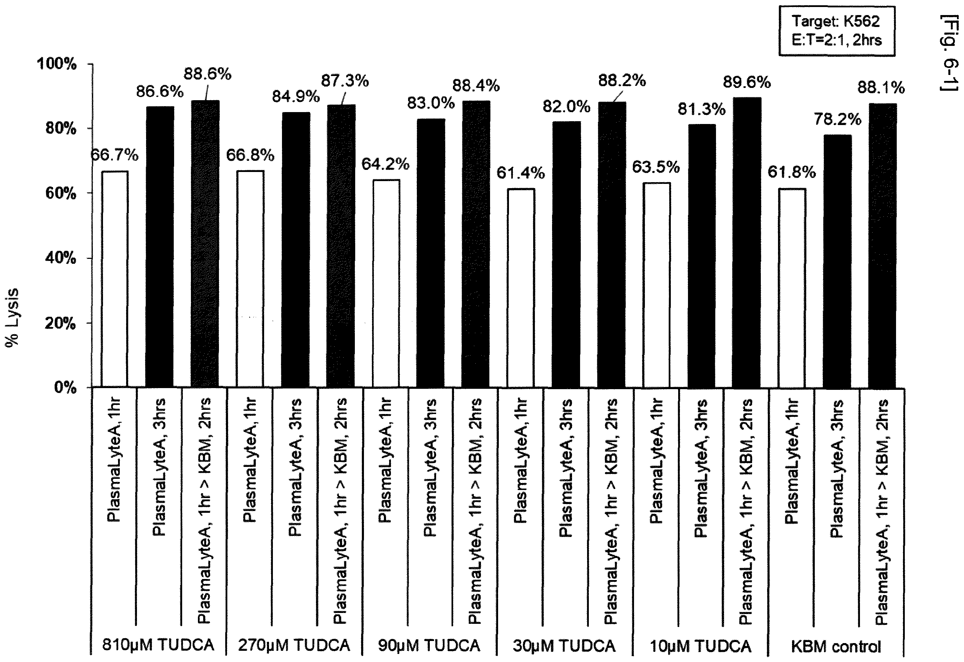

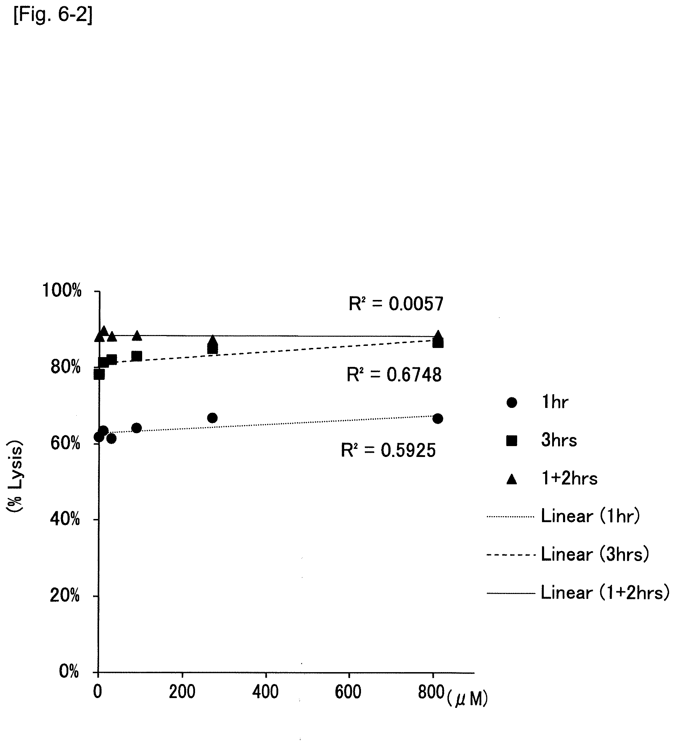

The results of the cytotoxic activity measurements are shown in Figs. 6-1 and 6-2. Improvement of the cytotoxic activity was observed in a TUDCA-concentration- used-in-the-pretreatment-dependent manner.

[Example 7]

Number of viable cells of highly active NK cells obtained by the procedures described as the culture method and collection method 2 for highly active NK cells was counted, and the cells were suspended at a density of $1 \times 10^6$ cells/mL in the KBM501 medium, KBM501 medium containing 266 $\mu$M, 810 $\mu$M, or 2400 $\mu$M TUDCA, or KBM501 medium containing 2.4% DMSO (Nakalai Tesque, 13445-74), inoculated onto a low-absorption 6-well plate, and incubated at 37°C for 2 hours under 5% $CO_2$ for a pretreatment before freezing. After the pretreatment, the cells were suspended in STEM-CELLBANKER at a density of $1 \times 10^7$ cells/mL on basis the cell count at the time of the treatment, and cryopreserved at -80°C. The pretreated NK cells frozen for 48 hours or longer were thawed at 37°C in a water bath, and the cells of each group were diluted 11 -fold in Plasma-Lyte A or Plasma-Lyte A containing 3000 units/mL of IL-2, and incubated at room temperature up to 3 hours. After 1 and 3 hours, the cells were gently suspended, and viable cells were counted. In addition, after 1 hour, the cells were diluted 5-fold in the KBM501 medium, and incubated at 37°C for 2 hours under 5% $CO_2$, and viable cell were counted. Recovery rate of viable cells after the thawing based on the cell number at the time of freezing was calculated on the basis of the number of viable cells at each time point after the thawing.

In addition, the cytotoxic activity of the highly active NK cells for the K562 cells was measured at the time points of thawing and dilution by the method described in Example 6.

Further, the highly active NK cells at the time points of 1 and 3 hours after the thawing were evaluated in a solid tumor model (3D killing assay). In detail, the highly active NK cells were prepared at a density of $1 \times 10^6$ cells/mL in the KBM501 medium on the basis of the cell count at the time of freezing. To one of SKOV3 sphere inoculated beforehand in each well of a 384-well plate, 50 $\mu$L of the prepared NK cells were added, and allowed to react for 4 days at 37°C under 5% $CO_2$. Then, the cells were detached with Accutase, collected, and cultured in the RPMI medium on a 24-well plate for expansion. After 17 days of the expansion culture at 37°C under 5% $CO_2$, the cells were fixed with 4% PFA, stained with DAPI, and observed under a fluorescence microscope (BZ-9000, KEYENCE), and BZ-II Analysis Application was used to quantify the area of SKOV3 that was not injured by the NK cells.

«SKOV3 sphere»

The SKOV3 cells (human ovarian cancer cell line) were prepared at a density of $3 \times 10^4$ cells/mL in 10% FBS/RPMI 1640, and inoculated onto a 96-well plate in an amount of $3 \times 10^3$ cells/100 $\mu$L per well. Spheres were prepared by incubating the cells at 37°C for 3 days under 5% $CO_2$.

The obtained recovery rates are shown in the following table along with the pretreatment and thawing conditions. The recovery rate after thawing was improved by the pretreatment of highly active NK cells with the KBM501 medium supplemented with TUDCA at various concentrations.

[Table 6]

| | pre-treat | | | | | Thawing | | | | | |
|---|---|---|---|---|---|---|---|---|---|---|---|
| | base medium | Additive | Temperature | Time | | Solvent for thawing (10x) | | Temperalure | Time | Note | Recovery rate |
| 1 | KBM, 5% UG+Hepa | 2400μM TUDCA | 37°C | 2hrs | 100% | PlasmaLyteA | - | RT | 1hr | - | 82.0% |
| | | | | | 100% | | | | 3hrs | - | 195.3% |
| | | | | | 100% | | | | 1hr+2hr | Diluted 5-fold with KBM after 1 hour | 134.5% |
| | | | | | 100% | PlasmaLyteA | IL-2 | RT | 1hr | - | 95.7% |
| | | | | | 100% | | | | 3hrs | - | 62.2% |
| | | | | | 100% | | | | 1hr+2hr | Diluted 5-fold with KBM after 1 hour | 85.6% |
| 2 | KBM, 5% UG+Hepa | 800μM TUDCA | 37°C | 2hrs | 100% | PlasmaLyteA | - | RT | 1hr | - | 118.8% |
| | | | | | 100% | | | | 3hrs | - | 127.6% |
| | | | | | 100% | | | | 1hr+2hr | Diluted 5-fold with KBM after 1 hour | 116.1% |
| | | | | | 100% | PlasmaLyteA | IL-2 | RT | 1hr | - | 124.9% |
| | | | | | 100% | | | | 3hrs | - | 123.8% |
| | | | | | 100% | | | | 1hr+2hr | Diluted 5-fold with KBM after 1 hour | 130.8% |
| 3 | KBM, 5% UG+Hepa | 266μM TUDCA | 37°C | 2hrs | 100% | PlasmaLyteA | - | RT | 1hr | - | 200.0% |
| | | | | | 100% | | | | 3hrs | - | 133.7% |
| | | | | | 100% | | | | 1hr+2hr | Diluted 5-fold with KBM after 1 hour | 97.8% |
| | | | | | 100% | PlasmaLyteA | IL-2 | RT | 1hr | - | 54.5% |
| | | | | | 100% | | | | 3hrs | - | 168.3% |
| | | | | | 100% | | | | 1hr+2hr | Diluted 5-fold with KBM after 1 hour | 108.8% |

(continued)

| | pre-treat | | | | | Thawing | | | | | |
|---|---|---|---|---|---|---|---|---|---|---|---|
| | base medium | Additive | Temperature | Time | | Solvent for thawing (10x) | | Temperalure | Time | Note | Recovery rate |
| 4 | KBM, 5% UG+Hepa | - | 37°C | 2hrs | 100% | PlasmaLyteA | - | RT | 1hr | - | 93.0% |
| | | | | | 100% | | | | 3hrs | - | 138.6% |
| | | | | | 100% | | | | 1hr+2hr | Diluted 5-fold with KBM after 1 hour | 116.1% |
| | | | | | 100% | PlasmaLyteA | IL-2 | RT | 1hr | - | 102.9% |
| | | | | | 100% | | | | 3hrs | - | 115.5% |
| | | | | | 100% | | | | 1hr+2hr | Diluted 5-fold with KBM after 1 hour | 146.7% |
| 5 | KBM, 5% UG+Hepa | 2.4% DMSO | 37°C | 2hrs | 100% | PlasmaLyteA | - | RT | 1hr | - | 60.5% |
| | | | | | 100% | | | | 3hrs | - | 128.2% |
| | | | | | 100% | | | | 1hr+2hr | Diluted 5-fold with KBM after 1 hour | 74.6% |
| | | | | | 100% | PlasmaLyteA | IL-2 | RT | 1hr | - | 95.2% |
| | | | | | 100% | | | | 3hrs | - | 154.0% |
| | | | | | 100% | | | | 1hr+2hr | Diluted 5-fold with KBM after 1 hour | 73.3% |

EP 4 151 717 A1

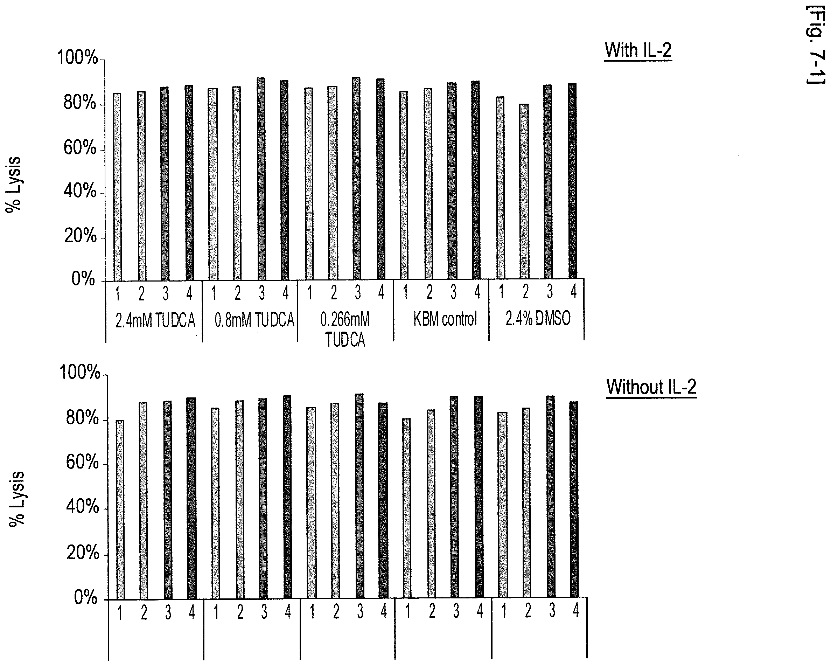

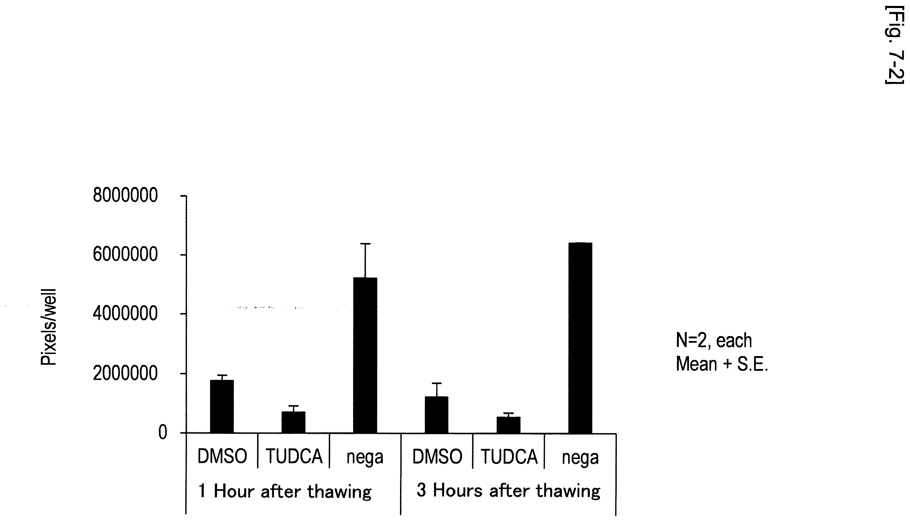

The results of the cytotoxic activity measurement are shown in Fig. 7-1, and the results of the evaluation in a solid tumor model (3D killing assay) are shown in Fig. 7-2. In Fig. 7-2, the results for the SKOV3 spheres to which the NK cells were not added are indicated as "nega".

When the evaluation in the solid tumor model was further performed with widening the TUDCA concentration range, it was found that TUDCA is preferably added at a concentration between 2,400 $\mu$M and 267 $\mu$M before freezing, especially at 800 $\mu$M, which resulted in a higher recovery rate and higher killing activity against the solid tumor model.

[Example 8]

(8-1) Number of viable cells of highly active NK cells obtained by the procedures described as the culture method and collection method 2 for highly active NK cells was counted, and the cells were suspended at a density of 1 $\times$ $10^7$ cells/mL in STEM-CELLBANKER, and cryopreserved at -80°C. The highly active NK cells frozen for 48 hours or longer were thawed at 37°C in a water bath, diluted 10-fold in (1) Plasma-Lyte A, (2) PBS(-) containing 1.48 mM $MgCl_2 \cdot 6H_2O$ (Nacalai Tesque, 20909-55), (3) PBS(-), (4) KBM501 medium, (5) Plasma-Lyte A containing 62.2 mg/L of $CaCl_2 \cdot 2H_2O$ (Nacalai Tesque, 08895-15), (6) Plasma-Lyte A containing 0.1% glucose (50% dextrose, Thermo), RPMI mediums (Nacalai Tesque, 09892-15) not containing (7) glucose, (8) serum ingredients, or (9) RPMI medium containing glucose (Nacalai Tesque, 30264-85), and incubated at room temperature for 2 hours for the cases of (1) to (4), or at 37°C for 3 hours for the cases of (1) and (5) to (9). Then, measurement was performed by the method for staining with 7-AAD described above, and the results were analyzed with the FlowJo software.

(8-2) Number of viable cells of highly active NK cells obtained by the procedures described as the culture method and collection method 1 or 2 for highly active NK cells was counted, and the cells were suspended at a density of 1 $\times$ $10^7$ cells/mL in STEM-CELLBANKER, and cryopreserved at -80°C. The highly active NK cells frozen for 48 hours or longer were thawed at 37°C in a water bath, diluted 10-fold in (1) Plasma-Lyte A, (2) PBS(-) containing 1.48 mM $MgCl_2$ (Nacalai Tesque, 95812-85), (3) PBS(-), (4) KBM501 medium, (5) Lactoringer, or (6) HBSS(+) (Nacalai Tesque, 09735-75), and incubated at 37°C for 3 hours. Then, measurement was performed by the method for staining with 7-AAD described above, and the results were analyzed with the FlowJo software.

(8-3) The experiment was performed in the same manner as in (8-1), but (1) PBS, (2) Plasma-Lyte A, (3) Plasma-Lyte A containing 27.7 mEq/L of lactic acid (Nacalai Tesque, 20006-62), (4) Plasma-Lyte A containing 48.84 mg/L of $MgSO_4 \cdot 7H_2O$ (Nacalai Tesque, 21002-85), (5) Lactoringer, (6) Veen-F (Fuso Pharma), (7) Solita-T1 (AY Pharmaceuticals), (8) Solita-T3 (AY Pharmaceuticals), (9) 5% glucose injection solution (Terumo), and (10) RPMI medium were used as the solvents for dilution of thawed cells, and the cells were incubated at 37°C for 3 hours. In the case of (3), lactic acid was added at the same concentration as the lactate concentration in Lactoringer, and neutrality of the solution was confirmed with litmus test paper. In the case of (4), $MgSO_4$ was added at the same concentration as the $MgSO_4$ concentration in HBSS. Measurement was then performed by the method for staining with 7-AAD described above, and the results were analyzed with the FlowJo software.

(8-4) The experiment was performed in the same manner as in (8-2), but the cells were treated by using (1) PBS, (2) Plasma-Lyte A, (3) saline (OTSUKA NORMAL SALINE, Otsuka Pharmaceutical), (4) Bicanate (Otsuka Pharmaceutical), and (5) Solacet F (Terumo) as the solvents for dilution of thawed cells. Measurement was then performed by the method for staining with 7-AAD described above, and the results were analyzed with the FlowJo software.

(8-5) Numbers of viable cells of the highly active NK cells obtained by the procedures described as the culture method and collection methods 1 and 2 for highly active NK cells were counted, and the cells were suspended in STEM-CELLBANKER at a density of 1 $\times$ $10^7$ cells/mL, and cryopreserved at -80°C. The highly active NK cells frozen for 48 hours or longer were thawed at 37°C in a water bath. Immediately after completely thawed, the cells were diluted 10-fold in (1) Plasma-Lyte A, (2) saline, (3) RPMI 1640 medium, (4) Lactoringer, (5) 0.423 mM $CaCl_2$ prepared in Plasma-Lyte A, and (6) 1.36 mM $CaCl_2$, and left to stand at 37°C for 3 hours. The $CaCl_2$ concentration of (5) was the same as that in the RPMI 1640 medium, and the $CaCl_2$ concentration of (6) was the same as that in Lactoringer. Measurement was then performed by the method for staining with 7-AAD described above, and the results were analyzed with the FlowJo software.

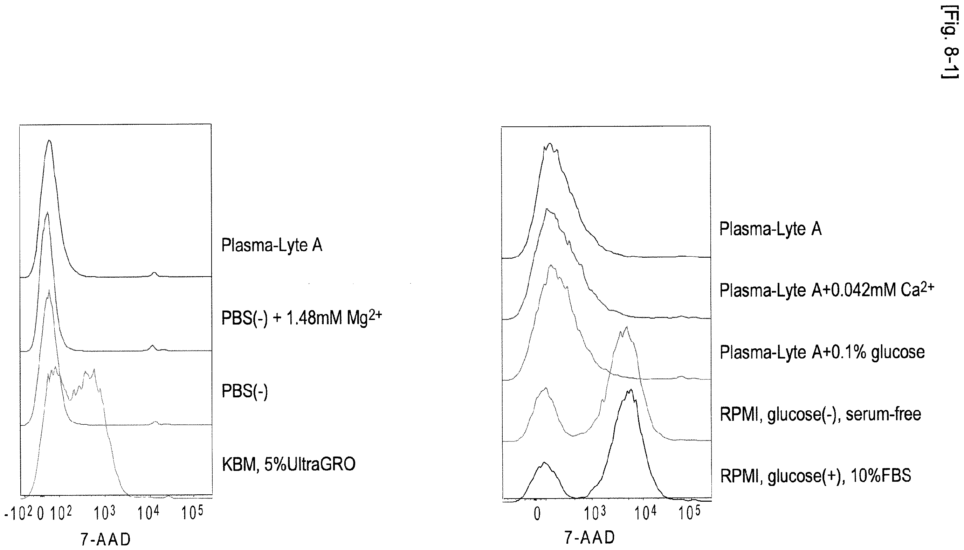

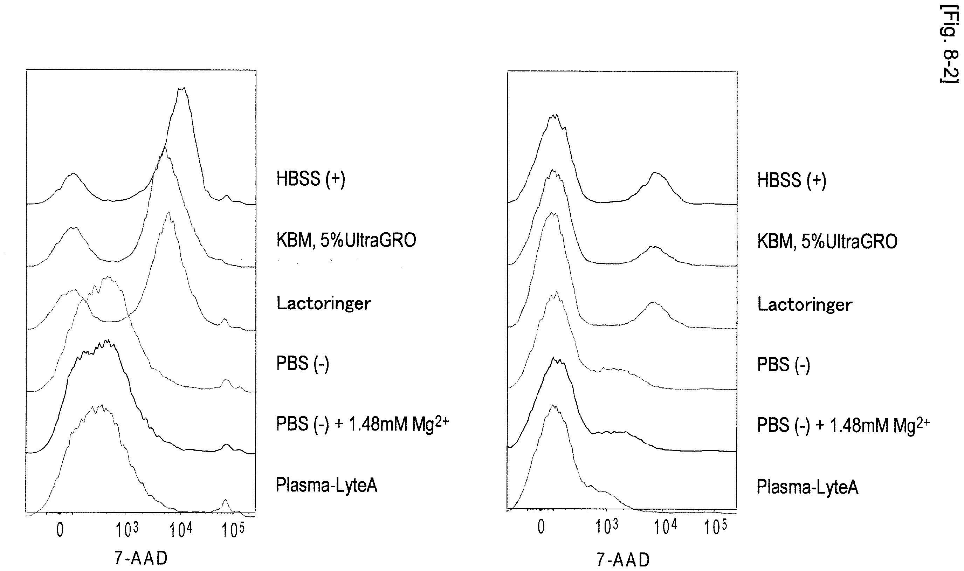

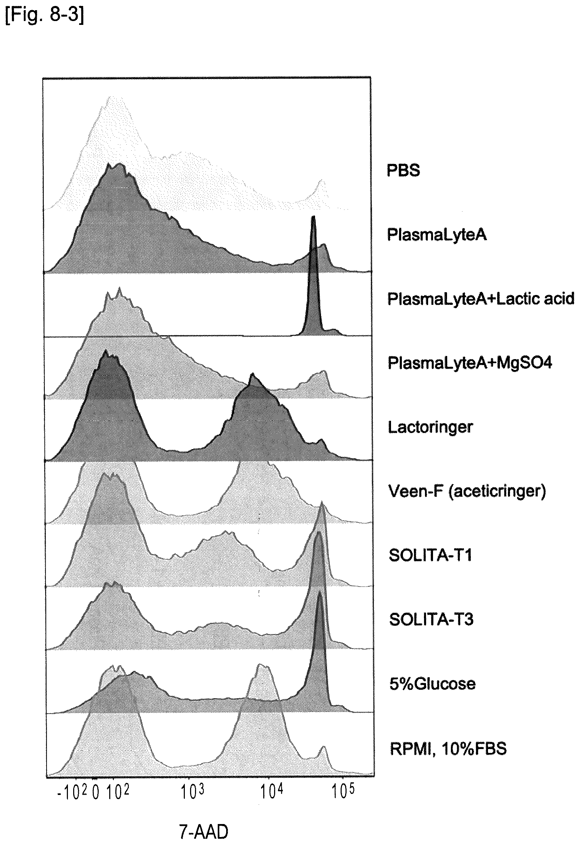

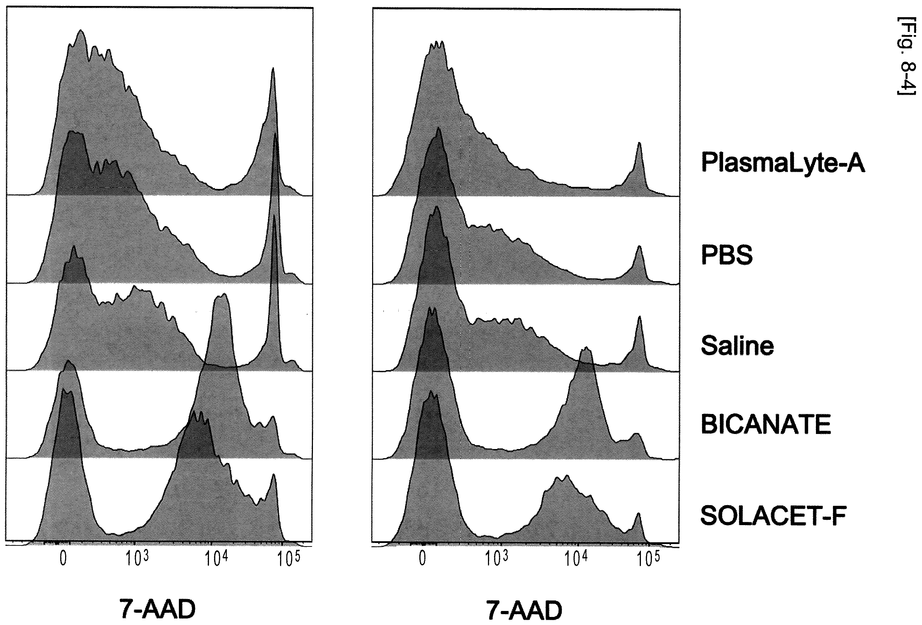

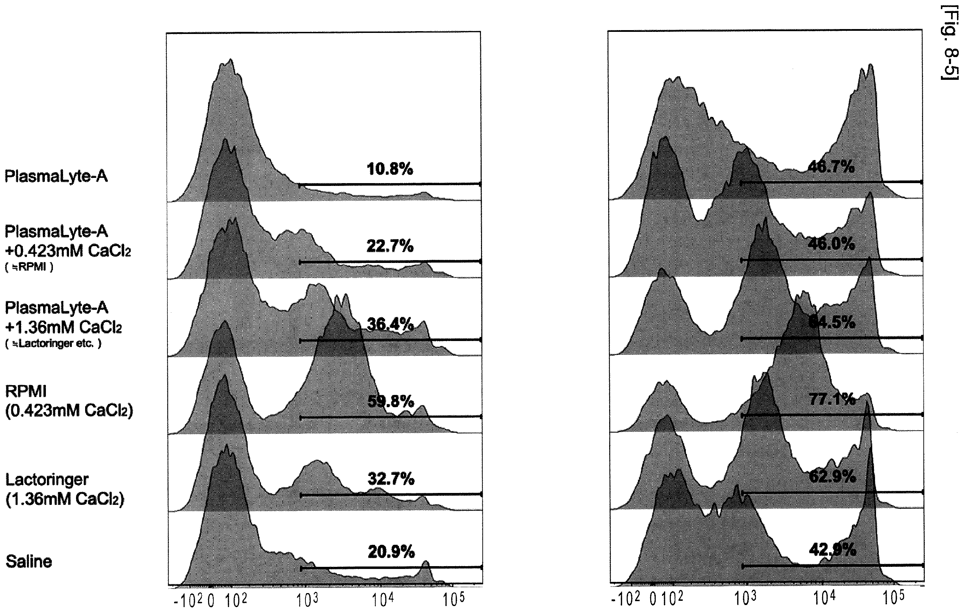

(Results) The results of the analysis with the FlowJo software are shown in Figs. 8-1 to 8-5. Viability could be improved by using the media as the solvent for the pretreatment before freezing, and Plasma-Lyte A after thawing. After thawing and dilution, the cells could be maintained at room temperature (8-1).

[Example 9]

(9-1) Number of viable cells of highly active NK cells obtained by the procedures described as the culture method and collection method 2 for highly active NK cells was counted, and the cells were suspended in STEM-CELLBANKER at a density of 4 $\times$ $10^7$ cells/mL, and cryopreserved at -80°C. The highly active NK cells frozen for 48 hours or longer were thawed at 37°C in a water bath, diluted 10-fold in Plasma-Lyte A containing 3000 units/mL of IL-2, and allowed to stand at room temperature for up to 6 hours. Cytotoxic activity was analyzed at the time points of 2, 5, and 6 hours after the thawing. For the measurement of cytotoxic activity, the NK cells and K562 cells were added to each well of a 96-well plate (IWAKI, 4870-800SP) at a cell ratio of 1:1, mixed, and allowed to react for 2 hours at 37°C under 5% $CO_2$ as described in Example 9-1, b).

Number of viable cells of highly active NK cells obtained by the procedures described as the culture method and collection method 2 for highly active NK cells was counted, and the cells were suspended in STEM-CELLBANKER at a density of $4 \times 10^7$ cells/mL, and cryopreserved at -80°C. The highly active NK cells frozen for 48 hours or longer were thawed at 37°C in a water bath, diluted 10-fold in Plasma-Lyte A containing 3000 units/mL of IL-2, and left to stand at room temperature for up to 4 hours. The change in the viability and cytotoxic activity (after a fixed reaction time of 2 hours, or at a fixed cell ratio E:T of 1:1) were analyzed at the time points immediately after the thawing, and of 1 hour, 2 hours, 3 hours, and 4 hours after the thawing.

a) Change in viability

At each time point, thawed cells were mixed well, $1 \times 10^5$ cells were taken, measurement was performed by the method for staining with 7-AAD described above, and the results were analyzed with the FlowJo software.

b) Cytotoxic activity (1)

For the measurement of cytotoxic activity, a group of NK cells reacted with K562 cells, a group of K562 cells only as a negative control, and a group of K562 cells fixed with 10% formalin as a positive control were prepared.

«NK cells»

A necessary amount of the cells were taken on the basis of the number of viable cells at the time of freezing, and then prepared in 10% FBS/RPMI 1640 at a density of $1 \times 10^6$ cells/ mL.

«K562 cells»

The K562 cells were suspended in serum-free RPMI 1640 medium, stained with PKH26 Red Fluorescent Cell Linker Kit, and then prepared at a density of $2 \times 10^6$ cells/mL in 10% FBS/RPMI 1640.
The NK cells and K562 cells were added and mixed in each well of 96-well plate (IWAKI, 4870-800SP) at cell ratios of 1:2, 1:1, 2:1, and 4:1, and allowed to react at 37°C for 2 hours under 5% $CO_2$. After completion of the reaction, measurement was performed by the method for staining with 7-AAD described above, and the results were analyzed with the FlowJo software to calculate the cytotoxic activity rate (% Lysis).

c) Cytotoxic activity (2)

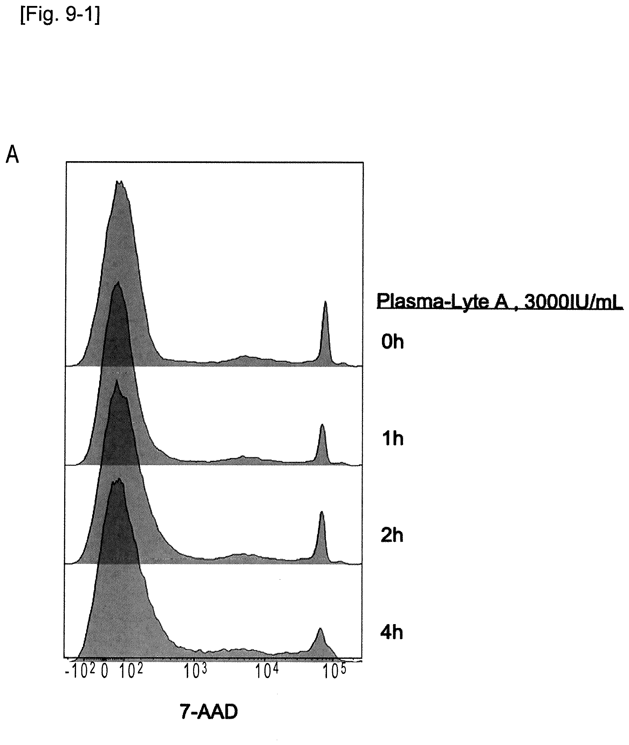

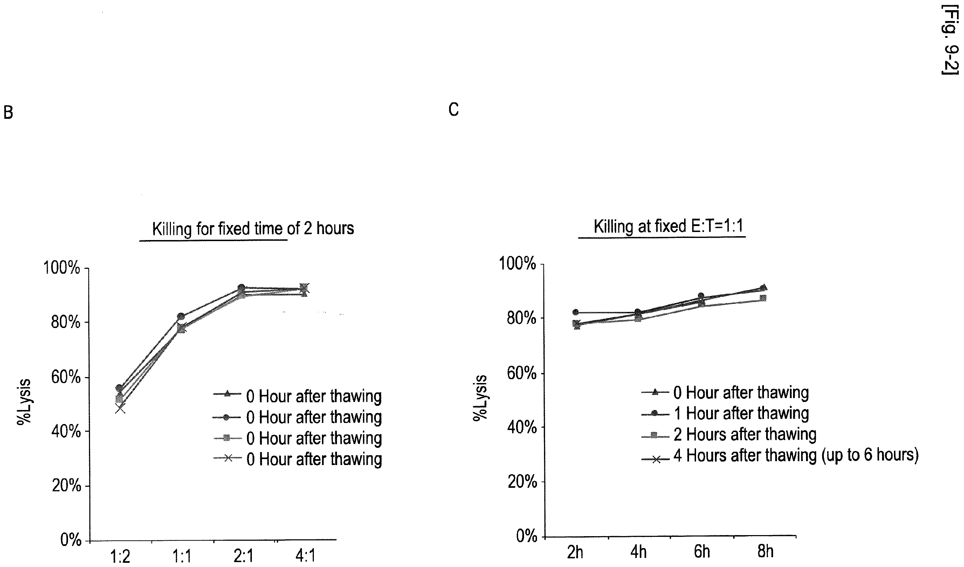

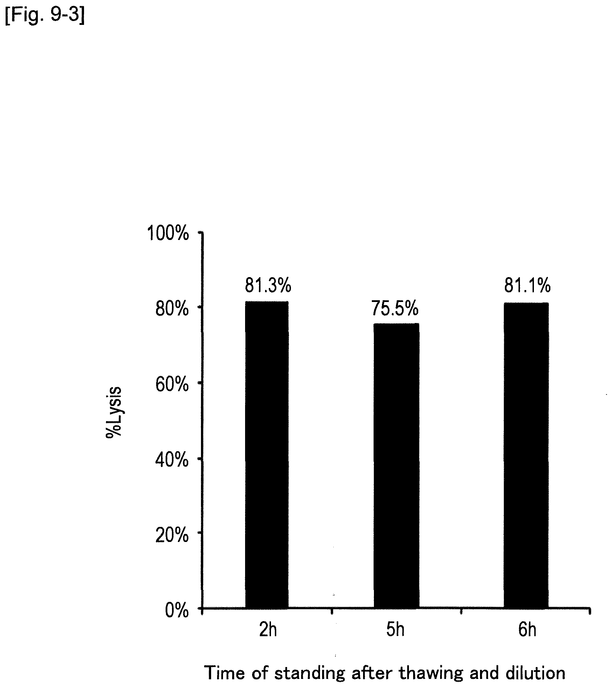

NK cells and K562 cells were prepared by the methods described in the section b) Cytotoxic activity (1) mentioned above, added to each well of a 96-well plate (IWAKI, 4870-800SP) at a cell ratio of 1:1, mixed, and then allowed to react at 37°C for 2, 4, 6 and 8 hours under 5% $CO_2$. After completion of the reaction, measurement was performed by the method for staining with 7-AAD described above, and the results were analyzed with the FlowJo software to calculate the cytotoxic activity rate (% Lysis).
(9-2) Cytotoxic activity was measured in the same manner as described in the section of (9-1), but after thawing and dilution, the cells were left to stand for up to 6 hours, and cytotoxic activity was measured by the method described in the section b) mentioned above at the time points of 2, 5, and 6 hours. In detail, the NK cells and K562 cells were added to each well of a 96-well plate (IWAKI, 4870-800SP) at a cell ratio of 1:1, mixed, and then allowed to react at 37°C for 2 hours under 5% $CO_2$. After the reaction was completed, measurement was performed by the method for staining with 7-AAD described above, and the results were analyzed with the FlowJo software to calculate the cytotoxic activity rate (% Lysis).
The results are shown in Figs. 9-1, 9-2, and 9-3. If highly active NK cells were pretreated, frozen, thawed, and diluted in an appropriate manner, viability and activity thereof could be maintained for 4 hours even when they were left to stand at room temperature in the dosage form. In addition, it was confirmed that the activity could be maintained for up to 6 hours. For example, it is known that the CAR-T cell preparation Kymriah can maintain its viability and activity for only 30 minutes in the dosage form (after thawing). The present invention significantly improves the handling property of cells used for immunotherapy.

[Example 10]

(10-1) Numbers of viable cells of highly active NK cells obtained by the procedures described as the culture method and collection methods 1 and 2 for highly active NK cells were counted, and the cells were suspended in STEM-CELLBANKER at a density of $1 \times 10^7$ cells/mL (1.5 mL tube, in the case of the cells obtained by the collection method 1, i.e., the case where the cells were washed with PBS at the time of collection), or $4 \times 10^7$ cells/mL (5 mL vial, in the case of the cells obtained by the collection method 2, i.e., the case where the cells were washed with the KBM501 medium at the time of collection), and cryopreserved at -80°C. The highly active NK cells frozen for 48 hours or longer were thawed at room temperature. The thawed cells were diluted 10-fold in Plasma-Lyte A or the KBM501 medium at the time points immediately after complete thawing and of 5, 10, 15, 20, 25, 30, 60, 120, and 180 minutes after complete thawing, and left to stand at room temperature for up to 180 minutes. After left to stand up to 180 minutes, measurement was performed by the method for staining with 7-AAD described above, and the results were analyzed with the FlowJo software.

(10-2) Numbers of viable cells of highly active NK cells obtained by the procedures described as the culture method and collection methods 1 and 2 for highly active NK cells were counted, and the cells were suspended in STEM-CELLBANKER at a density of $1 \times 10^7$ cells/mL (1.5 mL tube, in the case of the cells obtained by the collection method 1, i.e., the case where the cells were washed with PBS at the time of collection), or $4 \times 10^7$ cells/mL (5 mL vial, in the case of the cells obtained by the collection method 2, i.e., the case where the cells were washed with the KBM501 medium at the time of collection), and cryopreserved at -80°C. The highly active NK cells frozen for 48 hours or longer were thawed at 37°C in a water bath. The thawed cells were diluted 10-fold in Plasma-Lyte A or the KBM501 medium at the time points immediately after complete thawing and of 5, 10, 15, 20, 25, 30, 60, 120, and 180 minutes after complete thawing, and left to stand at room temperature for up to 180 minutes. After left to stand up to 180 minutes, measurement was performed by the method for staining with 7-AAD described above, and the results were analyzed with the FlowJo software.

The results are shown in Fig. 10. When the cells were washed with PBS at the time of collection before freezing, the viability was remarkably low, although the time required for thawing at room temperature was short (about 15 minutes) due to the small volume. On the other hand, when the cells were washed with the KBM501 medium, their viability was still maintained even after they were spontaneously thawed at room temperature (about 27 minutes), and left at room temperature for 2 to 3 hours. This method extremely widens the acceptable range of thawing operations, and provides extremely good handling property of the cells as a pharmaceutical product.

When the thawing was performed at 37°C, and the cells were washed with the KBM501 medium at the time of collection, the viability was also maintained even when the cells were left at room temperature for 2 to 3 hours after thawing.

[Example 11]

Numbers of viable cells of highly active NK cells obtained by the procedures described as the culture method and collection methods 1 and 2 for highly active NK cells were counted, and the cells were suspended in STEM-CELLBANKER at a density of $1 \times 10^7$ cells/mL (1.5 mL tube), and $4 \times 10^7$ cells/mL (5 mL vial), and cryopreserved at -80°C. The highly active NK cells frozen for 48 hours or longer were then thawed at 37°C in a water bath. Immediately after complete thawing, the cells were diluted 10-fold in Plasma-Lyte A or the KBM501 medium, and left to stand at room temperature for up to 6 hours. The cells were taken in an amount of $1 \times 10^5$ cells immediately after the dilution, and at the time points of 1, 2, 3, and 6 hours after the dilution, measurement was performed by the method for staining with 7-AAD described above, and the results were analyzed with the FlowJo software.

It was found that the cells washed with the KBM501 medium at the time of collection may retain high viability when they are diluted with Plasma-Lyte A after the thawing. In addition, it was also found that, as for the conditions at the time of freezing, the 5 mL vial ($4 \times 10^7$ cells/mL) was better than 1.5 mL tube, although both the 5 mL vial and 1.5 mL tube were acceptable. The characteristic of the highly active NK cells that they can be frozen at high densities for shipment makes the product more compact, and contributes to lower shipping costs.

[Example 12]

(12-1) Number of viable cells of highly active NK cells obtained by the procedures described as the culture method and collection method 2 for highly active NK cells was counted, and the cells were suspended in STEM-CELLBANKER at a density of $4 \times 10^7$ cells/mL, and cryopreserved at -80°C. The highly active NK cells frozen for 48 hours or longer were thawed at 37°C in a water bath. Immediately after complete thawing, the cells were diluted 10-fold with 80%, 50%, and 10% Plasma-Lyte A diluted in UltraPure Distilled Water (Invitrogen, 10977-015), and left to stand at room temperature and 37°C for up to 3 hours. At the time points immediately after the dilution and of 1, 2, and 3 hours after the dilution, $1 \times 10^5$ cells were taken, measurement was performed by the method for staining with 7-AAD described above, and the results were analyzed with the FlowJo software.

(12-2) The cells were treated, and analysis was performed in the same manner as in the section (12-1), provided that the cells were diluted 10-fold with 80%, 50%, 10% Plasma-Lyte A diluted in saline instead of UltraPure Distilled Water (invitrogen, 10977-015), and left to stand at room temperature and 37°C for up to 3 hours.

(12-3) The cells were treated, and analysis was performed in the same manner as in the section (12-1), provided that the cells were diluted 10-fold with Plasma-Lyte A diluted in saline instead of UltraPure Distilled Water (invitrogen, 10977-015), or saline, and left to stand at room temperature and 37°C for up to 3 hours.

(12-4) The cells were treated, and analysis was performed in the same manner as in the section (12-1), provided that the cells were diluted 10-fold immediately after completely thawing with Plasma-Lyte A, saline, 230 mM $NaCl_2$, 150 mM $KCl$, 100 mM $MgCl_2$, 11% trehalose, 10% sucrose, 5% sucrose prepared in UltraPure Distilled Water, 10% and 20% UltraGRO prepared in Plasma-Lyte A, and left to stand at room temperature for up to 3 hours.

(12-5) The cells were treated, and analysis was performed in the same manner as in the section (12-4), provided that the cells were diluted 10-fold with each of the solutions, and left to stand at 37°C for up to 3 hours.

(12-6) Number of viable cells of highly active NK cells obtained by the procedures described as the culture method and collection method 1 for highly active NK cells was counted, and the cells were suspended in STEM-CELLBANKER at a density of $1 \times 10^7$ cells/mL, and cryopreserved at -80°C. The highly active NK cells frozen for 48 hours or longer were thawed at 37°C in a water bath. Immediately after completely thawed, the cells were diluted 10-fold with Plasma-Lyte A, saline, 230 mM $NaCl_2$, 150 mM $KCl$, 100 mM $MgCl_2$, 100 mM $CaCl_2$, 11% trehalose, 10% sucrose, 5% sucrose prepared in UltraPure Distilled Water, 10%, 20% UltraGRO, 10%, 20%, 30%, and 40% AB Serum (CELLect, 2938249) prepared in Plasma-Lyte A, and left to stand at room temperature for up to 3 hours. Immediately after the dilution, and at the time points of of 1, 2, and 3 hours after the dilution, $1 \times 10^5$ cells were taken, measurement was performed by the method for staining with 7-AAD described above, and the results were analyzed with the FlowJo software.

(12-7) The cells were treated, and analysis was performed in the same manner as in the section (12-6), provided that the cells were diluted 10-fold with each of the solutions, and left to stand at 37°C for up to 3 hours.

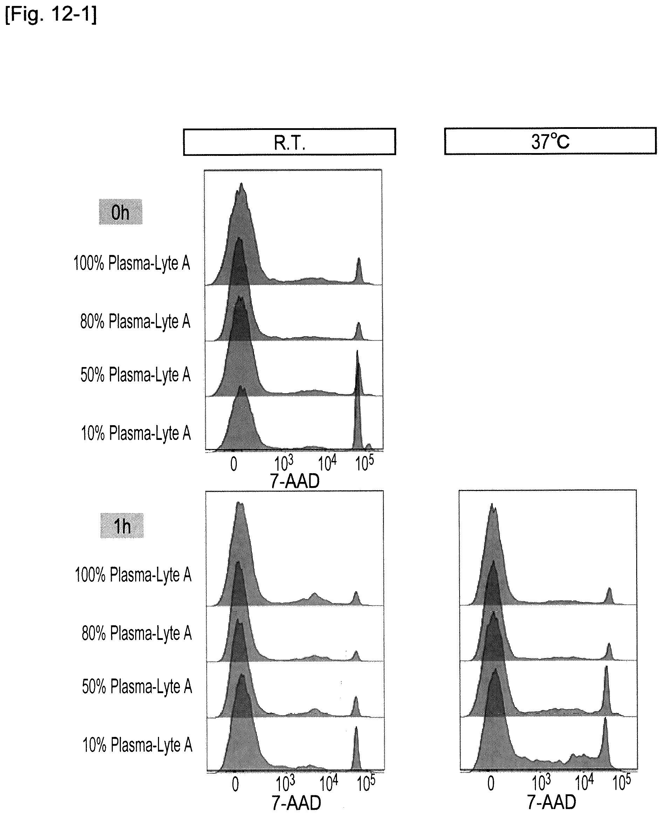

(Results) The results of the analysis with the FlowJo software are shown in Figs. 12-1 to 12-10.

Under the conditions of (12-1) to (12-3), the ingredients of Plasma-Lyte A used for thawing could be diluted to 1/10 of the original concentrations, and the solution used for the dilution could be either saline or distilled water. In other words, it was found that the solution used for thawing could be either hypotonic solution or saline.

Under the conditions of (12-4) and (12-5), when the cells were washed with the KBM501 medium at the time of collection, viability was maintained with an NaCl solution as the solution used at the time of thawing, regardless of whether the solution used at the time of thawing was hypotonic or hypertonic solution. Viability was also maintained at room temperature or for a short period of time even with isotonic solutions using a monosaccharide or polysaccharide. On the other hand, viability was degraded in the case of using 100 mM $MgCl_2$.

It was found that it is difficult to maintain viability with washing with PBS at the time of collection under the conditions of (12-6) and (12-7).

[List of compositions of solutions used at the time of thawing (unit is mM)]

[Table 7]

| | HBSS (+) | Lactoringer | Veen-F | Bicanate | Solacet F | RPMI1640 (Glu -) | PBS (-) | PBS (-) + Mg²⁺ | PlasmaLyte-A | PlasmaLyte-A + Ca²⁺ | PlasmaLyte-A + Glu |
|---|---|---|---|---|---|---|---|---|---|---|---|
| CaCl₂ | ○ | ○ | | | | | | | | ○ | |
| MgCl₂ | ○ | | | | | | | ○ | | ○ | ○ |
| MgSO₄ | ○ | | | ○ | ○ | | | | | | |
| KCl | ○ | ○ | | | | | ○ | ○ | | ○ | ○ |
| KH₂PO₄ | ○ | | | | | | ○ | ○ | | | |
| NaHCO₃ | ○ | | | | | | | | | | |
| NaCl | ○ | ○ | | | | | ○ | ○ | | | |
| Na₂HPO₄ | ○ | | | | | | | | ○ | ○ | ○ |
| C₃H₅NaO₃ | | ○ | | | | | | | | | |
| C₂H₃NaO₂ | | | | | | | | | ○ | ○ | ○ |
| Ca(NO₃)₂ | | | ○ | | | | | | | | |
| HCl | | | | | | | | | | | |
| Na₃C₆H₅O₇ | | | | | | | | | | | |
| C₃H₅O₃Na | | | | | | | | | | | |
| Amino Acids | | | | | ○ | ○ | | | | | |
| Vitamins | | | | | | ○ | | | | | |
| Phenol red | | | | | | ○ | | | | | |
| Maltose | | | | | | | | | | | |
| D-Glucose | ○ | | | | | ○ | | | | | ○ |

Row labels (full names): Saline, Calcium chloride, Magnesium chloride, Magnesium sulfate, Potassium chloride, Potassium hydrogenphosphate, Sodium hydrogencarbonate, Sodium chloride, Disodium hydrogenphosphate, Sodium gluconate, Sodium acetate, Potassium nitrate, Hydrochloric acid, Sodium citrate, Sodium lactate, Amino acid, Vitamins, Phenol Red, Maltose, Glucose

[Summary of Examples 1 to 12]

The results described above are summarized in the tables mentioned below. In the tables mentioned below, the unit for time is minute, except when indicated as "xx hour(s)". For Temperature, "R.T." represents room temperature, otherwise the temperature was 37°C. For Viability, "A" means 70% or higher, "B" means 50% or higher and lower than 70%, "C" means lower than 50%, and "-" means not evaluated. For Activity, A means 70% or higher, B means 50% or higher and lower than 70%, C means lower than 50%, and - means not evaluated. It can be said that evaluation result of A or B means that the object was achieved.

[Table 8-1]

| Collect Method | Pretreatment | | | Freezing | | | Thawing | | Thawing solvent 1 | | | Thawing solvent 2 | | | Results | | Exmp.No |
| | Base | Additive | Time | Cell Den. | Additive | Volume | Condition | Standing | Base | Additive/Temp. | Time | Base | Additive | Time | Viability | Activity | |
|---|---|---|---|---|---|---|---|---|---|---|---|---|---|---|---|---|---|
| 1 | – | – | – | 0.8-1e7/ml | – | 1ml | 37°C | 0 | KBM | – | | – | – | – | C | – | Exmp.1-1 |
| | – | – | – | | – | | | | Lacto | IL-2 | 3 h | – | – | – | C | – | |
| | – | – | – | | | | | | PlasmaLyte | | | – | – | – | A | – | |
| | – | – | – | | | | | | KBM | Gluconate | | – | – | – | C | – | |
| 1 | – | – | – | 0.5e7/ml | – | 1ml | 37°C | 0 | KBM | | | – | – | – | C | – | Exmp.1-2 |
| | – | – | – | | | | | | Lacto | IL-2 | 3 h | – | – | – | C | – | |
| | – | – | – | | | | | | PlasmaLyte | | | – | – | – | A | – | |
| 1 | – | – | – | 0.5-1e7/ml | – | 1ml | 37°C | 0 | KBM | – | | – | – | – | C | – | Exmp.2 |
| | – | – | – | | – | | | | Lacto | IL-2 | 3 h | – | – | – | C | – | |
| | – | – | – | | – | | | | PlasmaLyte | IL-2 | | – | – | – | A | – | |
| | – | – | – | | | | | | | AB Serum | | | | | C | – | |
| 1 | – | – | – | 1e7/ml | – | 1ml | 37°C | 0 | PlasmaLyte | IL-2 | 1 h | KBM | – | 3 h | C | B | Exmp.3 |
| | – | – | – | | – | | | | | | | Lacto | – | | A | C | |
| | – | – | – | | | | | | | | | KBM | – | o'n | C | A | |
| | – | – | – | | | | | | | | | Lacto | – | | C | C | |
| | – | – | – | | | | | | KBM | – | 3 h | – | – | – | A | C | |
| | – | – | – | | – | | | | | – | o'n | – | – | – | C | A | |
| | | | | | | | | | | | | | | | C | A | |
| 1 | KBM | 10μM 4-PBA | 2 h | 1e7/ml | | 0.5mL | 37°C | 0 | PlasmaLyte | IL-2 | 1 h | KBM | – | o'n | A | – | Exmp.4 |
| | | | | | | | | | | IL-2 | | | | | A | – | |
| | | | | | | | | | | 4-PBA | | | | | | | |
| | | – | | | | | | | | IL-2 | | | | | C | – | |
| | | | | | | | | | | IL-2 | | KBM | – | | C | – | |
| | PlasmaLyte | | | | | | | | | 4-PBA | | | | | | | |
| | | 10μM salbrinal | | | | | | | | IL-2 | | | | | C | – | |
| | | | | | | | | | | IL-2 | | | | | C | – | |
| | | | | | | | | | | 4-PBA | | | | | | | |
| | KBM | 100μM 4-PBA | | | | | | | | R.T. | | | IL-2 | | A | – | |
| | | | | | | | | | | | | | – | | C | – | |
| | PlasmaLyte | IL-2 | | | | | | | | – | | | IL-2 | | C | – | |
| | | | | | | | | | | – | | RPMI | – | | C | – | |
| | | | | | | | | | | R.T. | | | IL-2 | | C | – | |
| | PBS | Gluconate | | | | | | | | – | | | IL-2 | | C | – | |
| | | | | | | | | | | – | | | | | C | – | |
| 2 | KBM | 30μM 4-PBA | 2 h | 1e7/ml | | 0.5ml | 37°C | 0 | PlasmaLyte | R.T. | 1 h | KBM | 1 or 3 h | A | – | Exmp.5 |
| | | 30μM TUDCA | | | | | | | | | | | – | | A | – | |
| | | 30μM 4-PBA | | | | | | | | | | | – | | A | – | |
| | | 30μM TUDCA | | | | | | | | | | | – | | A | – | |
| 1 | PBS | – | | | | | | | | – | | | – | | C | – | |
| 2 | KBM | 10μM TUDCA | 2 h | 1e7/ml | | 0.5mL | 37°C | 0 | PlasmaLyte | R.T. | 1 h | | – | – | A | B | Exmp.6 |
| | | 30μM TUDCA | | | | | | | | | | | – | – | A | B | |
| | | 90μM TUDCA | | | | | | | | | | | – | – | A | B | |
| | | 270μM TUDCA | | | | | | | | | | | – | – | A | B | |
| | | 810μM TUDCA | | | | | | | | | | | – | – | A | B | |
| | | | | | | | | | | | | | | | A | A | |
| | KBM | 10μM TUDCA | | | | | | | PlasmaLyte | R.T. | 3 h | | – | – | A | A | |
| | | 30μM TUDCA | | | | | | | | | | | – | – | A | A | |
| | | 90μM TUDCA | | | | | | | | | | | – | – | A | A | |
| | | 270μM TUDCA | | | | | | | | | | | – | – | A | A | |
| | | 810μM TUDCA | | | | | | | | | | | – | | A | A | |
| | | | | | | | | | | | | | | | A | A | |
| | KBM | 10μM TUDCA | | | | | | | PlasmaLyte | R.T. | 1 h | KBM | – | 2 h | A | A | |
| | | 30μM TUDCA | | | | | | | | | | | | | A | A | |
| | | 90μM TUDCA | | | | | | | | | | | | | A | A | |
| | | 270μM TUDCA | | | | | | | | | | | | | A | A | |
| | | 810μM TUDCA | | | | | | | | | | | | | A | A | |
| 2 | KBM | 2400μM TUDCA | 2 h | 1e7/ml | | 0.5mL | 37°C | 0 | PlasmaLyte | R.T. | 1 h | KBM | – | 2 h | A | A | Exmp.7 |
| | | | | | | | | | | | 3 h | – | – | – | A | A | |
| | | | | | | | | | | IL-2, R.T. | 1 h | KBM | – | 2 h | A | A | |
| | | | | | | | | | | | 3 h | – | – | – | A | A | |
| | | 800μM TUDCA | | | | | | | | R.T. | 1 h | KBM | – | 2 h | A | A | |
| | | | | | | | | | | | 3 h | – | – | – | A | A | |
| | | | | | | | | | | IL-2, R.T. | 1 h | KBM | – | 2 h | A | A | |
| | | | | | | | | | | | 3 h | – | – | – | A | A | |
| | KBM | 266μM TUDCA | 2 h | 1e7/ml | | 0.5mL | 37°C | 0 | PlasmaLyte | R.T. | 1 h | KBM | – | 2 h | A | A | |
| | | | | | | | | | | | 3 h | – | – | – | A | A | |
| | | | | | | | | | | IL-2, R.T. | 1 h | KBM | – | 2 h | A | A | |
| | | | | | | | | | | | 3 h | – | – | – | A | A | |
| | | | | | | | | | | R.T. | 1 h | KBM | – | 2 h | A | A | |
| | | – | | | | | | | | | 3 h | – | – | – | A | A | |
| | | | | | | | | | | IL-2, R.T. | 1 h | KBM | – | 2 h | A | A | |
| | | | | | | | | | | | 3 h | – | – | – | A | A | |
| | | 2.4% DMSO | | | | | | | | R.T. | 1 h | KBM | – | 2 h | A | A | |
| | | | | | | | | | | | 3 h | – | – | – | A | A | |
| | | | | | | | | | | IL-2, R.T. | 1 h | KBM | – | 2 h | A | A | |
| | | | | | | | | | | | 3 h | – | – | – | A | A | |

[Table 8-2]

| Collect. Method | Pretreatment | | | Freezing | | | Thawing | | Thawing solvent 1 | | | Thawing 2 | | | Results | | Exmp No. |
|---|---|---|---|---|---|---|---|---|---|---|---|---|---|---|---|---|---|
| | Base | Additive | Time | Cell Den | Additive | Volume | Condition | Standing | Base | Additive/Temp | Time | Base | Additive | Time | Viability | Activity | |
| | -- | -- | -- | | -- | | | | PlasmaLyte | R.T. | | -- | -- | -- | A | -- | |
| | -- | -- | -- | | | | | | PBS | 1.48mM MgCl₂ R.T. | 2 h | -- | -- | -- | A | -- | |
| 2 | -- | -- | -- | 1e7/ml | -- | 1ml | 37°C | 0 | PBS | R.T. | | -- | -- | -- | A | -- | Exmp.8-1 |
| | -- | -- | -- | | | | | | KBM | R.T. | | -- | -- | -- | C | -- | |
| | -- | -- | -- | | | | | | PlasmaLyte | -- | | -- | -- | -- | A | -- | |
| | -- | -- | -- | | | | | | PlasmaLyte | 0.042mM CaCl₂ | | -- | -- | -- | A | -- | |
| | -- | -- | -- | | | | | | PlasmaLyte | 0.1% glucose | 3 h | -- | -- | -- | A | -- | |
| | -- | -- | -- | | | | | | RPMI | Glu(-), FBS(-) | | -- | -- | -- | C | -- | |
| | -- | -- | -- | | | | | | RPMI | Glu(+), 10%FBS | | -- | -- | -- | C | -- | |
| | -- | -- | -- | | | | | | HBSS(+) | -- | | -- | -- | -- | C | -- | |
| | -- | -- | -- | | | | | | KBM | -- | | -- | -- | -- | C | -- | |
| 1 | -- | -- | -- | | | | | | Lacto | -- | | -- | -- | -- | C | -- | |
| | -- | -- | -- | | | | | | PBS | -- | | -- | -- | -- | A | -- | |
| | -- | -- | -- | | | | | | | 1.48mM MgCl₂ | | -- | -- | -- | A | -- | |
| | -- | -- | -- | 1e7/ml | -- | 1ml | 37°C | 0 | PlasmaLyte | -- | 3 h | -- | -- | -- | A | -- | Exmp.8-2 |
| | -- | -- | -- | | | | | | HBSS(+) | -- | | -- | -- | -- | B | -- | |
| | -- | -- | -- | | | | | | KBM | -- | | -- | -- | -- | B | -- | |
| 2 | -- | -- | -- | | | | | | Lacto | -- | | -- | -- | -- | B | -- | |
| | -- | -- | -- | | | | | | PBS | -- | | -- | -- | -- | A | -- | |
| | -- | -- | -- | | | | | | | 1.48mM MgCl₂ | | -- | -- | -- | A | -- | |
| | -- | -- | -- | | | | | | PlasmaLyte | -- | | -- | -- | -- | A | -- | |
| | -- | -- | -- | | | | | | PBS | -- | | -- | -- | -- | B | -- | |
| | -- | -- | -- | | | | | | | | | -- | -- | -- | A | -- | |
| | -- | -- | -- | | | | | | PlasmaLyte | 27.7mEq 乳酸 48.84mg/ml MgSO₄ | | -- | -- | -- | C | -- | |
| 2 | -- | -- | -- | 1e7/ml | -- | 1ml | 37°C | 0 | | | 3 h | -- | -- | -- | A | -- | Exmp.8-3 |
| | -- | -- | -- | | | | | | Lacto | -- | | -- | -- | -- | C | -- | |
| | -- | -- | -- | | | | | | Veen-F | -- | | -- | -- | -- | C | -- | |
| | -- | -- | -- | | | | | | SOLITA-T1 | -- | | -- | -- | -- | C | -- | |
| | -- | -- | -- | | | | | | SOLITA-T3 | -- | | -- | -- | -- | C | -- | |
| | -- | -- | -- | | | | | | | 5% Glu | | -- | -- | -- | C | -- | |
| | -- | -- | -- | | | | | | RPMI | -- | | -- | -- | -- | C | -- | |
| | -- | -- | -- | | | | | | PlasmaLyte | -- | | -- | -- | -- | C | -- | |
| 1 | -- | -- | -- | | | | | | PBS | -- | | -- | -- | -- | C | -- | |
| | -- | -- | -- | | | | | | Saline | -- | | -- | -- | -- | C | -- | |
| | -- | -- | -- | | | | | | BICANATE | -- | | -- | -- | -- | C | -- | |
| | -- | -- | -- | 1e7/ml | -- | 1ml | 37°C | 0 | SOLACET-F | -- | 3 h | -- | -- | -- | C | -- | Exmp.8-4 |
| | -- | -- | -- | | | | | | PlasmaLyte | -- | | -- | -- | -- | A | -- | |
| 2 | -- | -- | -- | | | | | | PBS | -- | | -- | -- | -- | A | -- | |
| | -- | -- | -- | | | | | | Saline | -- | | -- | -- | -- | A | -- | |
| | -- | -- | -- | | | | | | BICANATE | -- | | -- | -- | -- | C | -- | |
| | -- | -- | -- | | | | | | SOLACET-F | -- | | -- | -- | -- | C | -- | |
| | -- | -- | -- | | | | | | | | | -- | -- | -- | A | -- | |
| | -- | -- | -- | | | | | | PlasmaLyte | 0.423mM CaCl₂ | | -- | -- | -- | B | -- | |
| 2 | -- | -- | -- | | | | | | | 1.36mM CaCl₂ | | -- | -- | -- | B | -- | |
| | -- | -- | -- | | | | | | RPMI | -- | | -- | -- | -- | C | -- | |
| | -- | -- | -- | | | | | | Lacto | -- | | -- | -- | -- | B | -- | |
| | -- | -- | -- | 1e7/ml | -- | 1ml | 37°C | 0 | Saline | -- | 3 h | -- | -- | -- | A | -- | Exmp.8-5 |
| | -- | -- | -- | | | | | | | | | -- | -- | -- | C | -- | |
| | -- | -- | -- | | | | | | PlasmaLyte | 0.423mM CaCl₂ | | -- | -- | -- | C | -- | |
| 1 | -- | -- | -- | | | | | | | 1.36mM CaCl₂ | | -- | -- | -- | C | -- | |
| | -- | -- | -- | | | | | | RPMI | -- | | -- | -- | -- | C | -- | |
| | -- | -- | -- | | | | | | Lacto | -- | | -- | -- | -- | C | -- | |
| | -- | -- | -- | | | | | | Saline | -- | | -- | -- | -- | C | -- | |
| | -- | -- | -- | | | | | | | | 0 h | -- | -- | -- | A | A | |
| 2 | -- | -- | -- | 4e7/ml | -- | 5ml | 37°C | 0 | PlasmaLyte | 3,000IU/ml IL-2 R.T. | 1 h | -- | -- | -- | A | A | Exmp.9-1 |
| | -- | -- | -- | | | | | | | | 2 h | -- | -- | -- | A | A | |
| | -- | -- | -- | | | | | | | | 3 h | -- | -- | -- | A | A | |
| | -- | -- | -- | | | | | | | | 4 h | -- | -- | -- | A | A | |
| 2 | -- | -- | -- | 4e7/ml | -- | 5ml | 37°C | 0 | PlasmaLyte | 3,000IU/ml IL-2 R.T. | 2 h | -- | -- | -- | -- | A | Exmp.9-2 |
| | -- | -- | -- | | | | | | | | 5 h | -- | -- | -- | -- | A | |
| | -- | -- | -- | | | | | | | | 6 h | -- | -- | -- | | A | |

[Table 8-3]

| Collect. Method | Pretreatment Base | Additive | Time | Freezing Cell Den | Additive | Volume | Thawing Condition | Standing | Thawing solvent 1 Base | Additive | Temp | Time | Thawing solvent 2 Base | Additive | Time | Results Viability | Activity | Exmp No. |
|---|---|---|---|---|---|---|---|---|---|---|---|---|---|---|---|---|---|---|
| 2 | | | | 4e7/ml | | 5ml | R.T. | 0 | KBM | | | 180 | | | | B | | |
| | | | | | | | | 5 | | | | 175 | | | | A | | |
| | | | | | | | | 10 | | | | 170 | | | | A | | |
| | | | | | | | | 15 | | | | 165 | | | | A | | |
| | | | | | | | | 20 | | | | 160 | | | | A | | |
| | | | | | | | | 25 | | | | 155 | | | | A | | |
| | | | | | | | | 30 | | | | 150 | | | | A | | |
| | | | | | | | | 60 | | | | 120 | | | | A | | |
| | | | | | | | | 120 | | | | 60 | | | | A | | |
| | | | | | | | | 180 | | | | 0 | | | | A | | |
| | | | | | | | | 0 | PlasmaLyte | | | 180 | | | | B | | |
| | | | | | | | | 5 | | | | 175 | | | | A | | |
| | | | | | | | | 10 | | | | 170 | | | | A | | |
| | | | | | | | | 15 | | | | 165 | | | | A | | |
| | | | | | | | | 20 | | | | 160 | | | | A | | |
| | | | | | | | | 25 | | | | 155 | | | | A | | |
| | | | | | | | | 30 | | | | 150 | | | | A | | |
| | | | | | | | | 60 | | | | 120 | | | | A | | |
| | | | | | | | | 120 | | | | 60 | | | | A | | Exmp 10-1 |
| | | | | | | | | 180 | | | | 0 | | | | A | | |
| 1 | | | | 1e7/ml | | 1ml | | 0 | KBM | | | 180 | | | | C | | |
| | | | | | | | | 5 | | | | 175 | | | | C | | |
| | | | | | | | | 10 | | | | 170 | | | | C | | |
| | | | | | | | | 15 | | | | 165 | | | | C | | |
| | | | | | | | | 20 | | | | 160 | | | | C | | |
| | | | | | | | | 25 | | | | 155 | | | | C | | |
| | | | | | | | | 30 | | | | 150 | | | | C | | |
| | | | | | | | | 60 | | | | 120 | | | | C | | |
| | | | | | | | | 120 | | | | 60 | | | | C | | |
| | | | | | | | | 180 | | | | 0 | | | | C | | |
| | | | | | | | | 0 | PlasmaLyte | | | 180 | | | | C | | |
| | | | | | | | | 5 | | | | 175 | | | | C | | |
| | | | | | | | | 10 | | | | 170 | | | | C | | |
| | | | | | | | | 15 | | | | 165 | | | | C | | |
| | | | | | | | | 20 | | | | 160 | | | | C | | |
| | | | | | | | | 25 | | | | 155 | | | | C | | |
| | | | | | | | | 30 | | | | 150 | | | | C | | |
| | | | | | | | | 60 | | | | 120 | | | | C | | |
| | | | | | | | | 120 | | | | 60 | | | | C | | |
| | | | | | | | | 180 | | | | 0 | | | | C | | |
| 2 | | | | 4e7/ml | | 5ml | 37°C | 0 | KBM | | | 180 | | | | B | | |
| | | | | | | | | 5 | | | | 175 | | | | B | | |
| | | | | | | | | 10 | | | | 170 | | | | B | | |
| | | | | | | | | 15 | | | | 165 | | | | A | | |
| | | | | | | | | 20 | | | | 160 | | | | A | | |
| | | | | | | | | 25 | | | | 155 | | | | A | | |
| | | | | | | | | 30 | | | | 150 | | | | A | | |
| | | | | | | | | 60 | | | | 120 | | | | A | | |
| | | | | | | | | 120 | | | | 60 | | | | A | | |
| | | | | | | | | 180 | | | | 0 | | | | A | | |
| | | | | | | | | 0 | PlasmaLyte | | | 180 | | | | B | | |
| | | | | | | | | 5 | | | | 175 | | | | A | | |
| | | | | | | | | 10 | | | | 170 | | | | A | | |
| | | | | | | | | 15 | | | | 165 | | | | A | | |
| | | | | | | | | 20 | | | | 160 | | | | A | | |
| | | | | | | | | 25 | | | | 155 | | | | A | | |
| | | | | | | | | 30 | | | | 150 | | | | A | | |
| | | | | | | | | 60 | | | | 120 | | | | A | | |
| | | | | | | | | 120 | | | | 60 | | | | A | | Exmp 10-2 |
| | | | | | | | | 180 | | | | 0 | | | | A | | |
| 1 | | | | 1e7/ml | | 1ml | | 0 | KBM | | | 180 | | | | C | | |
| | | | | | | | | 5 | | | | 175 | | | | C | | |
| | | | | | | | | 10 | | | | 170 | | | | C | | |
| | | | | | | | | 15 | | | | 165 | | | | C | | |
| | | | | | | | | 20 | | | | 160 | | | | C | | |
| | | | | | | | | 25 | | | | 155 | | | | C | | |
| | | | | | | | | 30 | | | | 150 | | | | C | | |
| | | | | | | | | 60 | | | | 120 | | | | C | | |
| | | | | | | | | 120 | | | | 60 | | | | C | | |
| | | | | | | | | 180 | | | | 0 | | | | C | | |
| | | | | | | | | 0 | PlasmaLyte | | | 180 | | | | C | | |
| | | | | | | | | 5 | | | | 175 | | | | C | | |
| | | | | | | | | 10 | | | | 170 | | | | C | | |
| | | | | | | | | 15 | | | | 165 | | | | C | | |
| | | | | | | | | 20 | | | | 160 | | | | C | | |
| | | | | | | | | 25 | | | | 155 | | | | C | | |
| | | | | | | | | 30 | | | | 150 | | | | C | | |
| | | | | | | | | 60 | | | | 120 | | | | C | | |
| | | | | | | | | 120 | | | | 60 | | | | C | | |
| | | | | | | | | 180 | | | | 0 | | | | C | | |
| 2 | | | | 4e7/ml | | 5ml | 37°C | 0 | PlasmaLyte | | | 0 h | | | | A | | |
| | | | | | | | | | | | | 1 h | | | | A | | |
| | | | | | | | | | | | | 2 h | | | | A | | |
| | | | | | | | | | | | | 3 h | | | | A | | |
| | | | | | | | | | | | | 6 h | | | | A | | |
| | | | | | | | | | KBM | | | 0 h | | | | A | | |
| | | | | | | | | | | | | 1 h | | | | A | | |
| | | | | | | | | | | | | 2 h | | | | B | | |
| | | | | | | | | | | | | 3 h | | | | B | | |
| | | | | | | | | | | | | 6 h | | | | B | | |
| 1 | | | | 1e7/ml | | 1ml | | | PlasmaLyte | | | 0 h | | | | A | | Exmp 11 |
| | | | | | | | | | | | | 1 h | | | | A | | |
| | | | | | | | | | | | | 2 h | | | | A | | |
| | | | | | | | | | | | | 3 h | | | | A | | |
| | | | | | | | | | | | | 6 h | | | | B | | |
| | | | | | | | | | KBM | | | 0 h | | | | C | | |
| | | | | | | | | | | | | 1 h | | | | C | | |
| | | | | | | | | | | | | 2 h | | | | C | | |
| | | | | | | | | | | | | 3 h | | | | C | | |
| | | | | | | | | | | | | 6 h | | | | C | | |
| | | | | | | | | | PlasmaLyte | | | 0 h | | | | C | | |
| | | | | | | | | | | | | 1 h | | | | C | | |
| | | | | | | | | | | | | 2 h | | | | C | | |
| | | | | | | | | | | | | 3 h | | | | C | | |
| | | | | | | | | | | | | 6 h | | | | C | | |

[Table 8-4]

| Collect. Method | Pretreatment | | | Freezing | | | Thawing | | Thawing solvent 1 | | | Time | Thawing solvent 2 | | | Results | | Exmp.No |
|---|---|---|---|---|---|---|---|---|---|---|---|---|---|---|---|---|---|---|
| | Base | Additive | Time | Cell Den | Additive | Volume | Condition | Standing | Base | Additive | Temp | | Base | Additive | Time | Viability | Activity | |
| | – | – | – | | – | | | | 100% PL | | R.T. | 0 h | – | – | – | A | – | |
| | – | – | – | | | | | | | | | 1 h | – | – | – | A | – | |
| | – | – | – | | | | | | | | | 2 h | – | – | – | A | – | |
| | – | – | – | | | | | | | | | 3 h | – | – | – | A | – | |
| | – | – | – | | | | | | 80% PL 20% dH₂O | | R.T. | 0 h | – | – | – | A | – | |
| | – | – | – | | | | | | | | | 1 h | – | – | – | A | – | |
| | – | – | – | | | | | | | | | 2 h | – | – | – | A | – | |
| | – | – | – | | | | | | | | | 3 h | – | – | – | A | – | |
| | – | – | – | | | | | | | | 37°C | 0 h | – | – | – | A | – | |
| | – | – | – | | | | | | | | | 1 h | – | – | – | A | – | |
| | – | – | – | | | | | | | | | 2 h | – | – | – | A | – | |
| | – | – | – | | | | | | | | | 3 h | – | – | – | A | – | |
| 2 | – | – | – | 4e7/ml | – | 5ml | 37°C | 0 | 50% PL 50% dH₂O | | R.T. | 0 h | – | – | – | A | – | Exmp.12-1 |
| | – | – | – | | | | | | | | | 1 h | – | – | – | A | – | |
| | – | – | – | | | | | | | | | 2 h | – | – | – | A | – | |
| | – | – | – | | | | | | | | | 3 h | – | – | – | A | – | |
| | – | – | – | | | | | | | | 37°C | 0 h | – | – | – | A | – | |
| | – | – | – | | | | | | | | | 1 h | – | – | – | A | – | |
| | – | – | – | | | | | | | | | 2 h | – | – | – | A | – | |
| | – | – | – | | | | | | | | | 3 h | – | – | – | A | – | |
| | – | – | – | | | | | | 10% PL 90% dH₂O | | R.T. | 0 h | – | – | – | A | – | |
| | – | – | – | | | | | | | | | 1 h | – | – | – | A | – | |
| | – | – | – | | | | | | | | | 2 h | – | – | – | A | – | |
| | – | – | – | | | | | | | | | 3 h | – | – | – | A | – | |
| | – | – | – | | | | | | | | 37°C | 0 h | – | – | – | – | – | |
| | – | – | – | | | | | | | | | 1 h | – | – | – | B | – | |
| | – | – | – | | | | | | | | | 2 h | – | – | – | B | – | |
| | – | – | – | | | | | | | | | 3 h | – | – | – | B | – | |
| | – | – | – | | – | | | | 100% PL | | R.T. | 0 h | – | – | – | A | – | |
| | – | – | – | | | | | | | | | 1 h | – | – | – | A | – | |
| | – | – | – | | | | | | | | | 2 h | – | – | – | A | – | |
| | – | – | – | | | | | | | | | 3 h | – | – | – | A | – | |
| | – | – | – | | | | | | 80% PL 20% Saline | | R.T. | 0 h | – | – | – | A | – | |
| | – | – | – | | | | | | | | | 1 h | – | – | – | A | – | |
| | – | – | – | | | | | | | | | 2 h | – | – | – | A | – | |
| | – | – | – | | | | | | | | | 3 h | – | – | – | A | – | |
| | – | – | – | | | | | | | | 37°C | 0 h | – | – | – | A | – | |
| | – | – | – | | | | | | | | | 1 h | – | – | – | A | – | |
| | – | – | – | | | | | | | | | 2 h | – | – | – | A | – | |
| | – | – | – | | | | | | | | | 3 h | – | – | – | A | – | |
| 2 | – | – | – | 4e7/ml | – | 5ml | 37°C | 0 | 50% PL 50% Saline | | R.T. | 0 h | – | – | – | A | – | Exmp.12-2 |
| | – | – | – | | | | | | | | | 1 h | – | – | – | A | – | |
| | – | – | – | | | | | | | | | 2 h | – | – | – | A | – | |
| | – | – | – | | | | | | | | | 3 h | – | – | – | A | – | |
| | – | – | – | | | | | | | | 37°C | 0 h | – | – | – | A | – | |
| | – | – | – | | | | | | | | | 1 h | – | – | – | A | – | |
| | – | – | – | | | | | | | | | 2 h | – | – | – | A | – | |
| | – | – | – | | | | | | | | | 3 h | – | – | – | A | – | |
| | – | – | – | | | | | | 10% PL 90% Saline | | R.T. | 0 h | – | – | – | A | – | |
| | – | – | – | | | | | | | | | 1 h | – | – | – | A | – | |
| | – | – | – | | | | | | | | | 2 h | – | – | – | A | – | |
| | – | – | – | | | | | | | | | 3 h | – | – | – | A | – | |
| | – | – | – | | | | | | | | 37°C | 0 h | – | – | – | A | – | |
| | – | – | – | | | | | | | | | 1 h | – | – | – | A | – | |
| | – | – | – | | | | | | | | | 2 h | – | – | – | A | – | |
| | – | – | – | | | | | | | | | 3 h | – | – | – | A | – | |
| | – | – | – | | | | | | 100% PL | | R.T. | 0 h | – | – | – | A | – | |
| | – | – | – | | | | | | | | | 1 h | – | – | – | A | – | |
| | – | – | – | | | | | | | | | 2 h | – | – | – | A | – | |
| | – | – | – | | | | | | | | | 3 h | – | – | – | A | – | |
| | – | – | – | | | | | | 80% PL 20% Saline | | R.T. | 0 h | – | – | – | A | – | |
| | – | – | – | | | | | | | | | 1 h | – | – | – | A | – | |
| | – | – | – | | | | | | | | | 2 h | – | – | – | A | – | |
| | – | – | – | | | | | | | | | 3 h | – | – | – | A | – | |
| | – | – | – | | | | | | | | 37°C | 0 h | – | – | – | A | – | |
| | – | – | – | | | | | | | | | 1 h | – | – | – | A | – | |
| | – | – | – | | | | | | | | | 2 h | – | – | – | A | – | |
| | – | – | – | | | | | | | | | 3 h | – | – | – | A | – | |
| | – | – | – | | | | | | 50% PL 50% Saline | | R.T. | 0 h | – | – | – | A | – | |
| | – | – | – | | | | | | | | | 1 h | – | – | – | A | – | |
| | – | – | – | | | | | | | | | 2 h | – | – | – | A | – | |
| | – | – | – | | | | | | | | | 3 h | – | – | – | A | – | |
| 2 | – | – | – | 4e7/ml | – | 5ml | 37°C | 0 | | | 37°C | 0 h | – | – | – | A | – | Exmp.12-3 |
| | – | – | – | | | | | | | | | 1 h | – | – | – | A | – | |
| | – | – | – | | | | | | | | | 2 h | – | – | – | A | – | |
| | – | – | – | | | | | | | | | 3 h | – | – | – | A | – | |
| | – | – | – | | | | | | 10% PL 90% Saline | | R.T. | 0 h | – | – | – | A | – | |
| | – | – | – | | | | | | | | | 1 h | – | – | – | A | – | |
| | – | – | – | | | | | | | | | 2 h | – | – | – | A | – | |
| | – | – | – | | | | | | | | | 3 h | – | – | – | A | – | |
| | – | – | – | | | | | | | | 37°C | 0 h | – | – | – | A | – | |
| | – | – | – | | | | | | | | | 1 h | – | – | – | A | – | |
| | – | – | – | | | | | | | | | 2 h | – | – | – | A | – | |
| | – | – | – | | | | | | | | | 3 h | – | – | – | A | – | |
| | – | – | – | | | | | | Saline | | R.T. | 0 h | – | – | – | A | – | |
| | – | – | – | | | | | | | | | 1 h | – | – | – | A | – | |
| | – | – | – | | | | | | | | | 2 h | – | – | – | A | – | |
| | – | – | – | | | | | | | | | 3 h | – | – | – | A | – | |
| | – | – | – | | | | | | | | 37°C | 0 h | – | – | – | A | – | |
| | – | – | – | | | | | | | | | 1 h | – | – | – | A | – | |
| | – | – | – | | | | | | | | | 2 h | – | – | – | A | – | |
| | – | – | – | | | | | | | | | 3 h | – | – | – | A | – | |

[Table 8-5]

| Collect. Method | Pretreatment | | | Freezing | | | Thawing | | Thawing solvent 1 | | | Thawing solvent 2 | | | Results | | Exmp.No. |
|---|---|---|---|---|---|---|---|---|---|---|---|---|---|---|---|---|---|
| | Base | Additive | Time | Cell Den. | Additive | Volume | Condition | Standing | Base | Additive/Temp | Time | Base | Additive | Time | Viability | Activity | |
| | -- | -- | -- | | -- | | | | PlasmaLyte | | 0 h | -- | -- | -- | A | -- | |
| | -- | -- | -- | | | | | | | | 1 h | -- | -- | -- | A | -- | |
| | -- | -- | -- | | | | | | | | 2 h | -- | -- | -- | A | -- | |
| | -- | -- | -- | | | | | | | | 3 h | -- | -- | -- | A | -- | |
| | -- | -- | -- | | | | | | Saline | | 0 h | -- | -- | -- | A | -- | |
| | -- | -- | -- | | | | | | | | 1 h | -- | -- | -- | A | -- | |
| | -- | -- | -- | | | | | | | | 2 h | -- | -- | -- | A | -- | |
| | -- | -- | -- | | | | | | | | 3 h | -- | -- | -- | A | -- | |
| | -- | -- | -- | | | | | | 230mM NaCl | | 0 h | -- | -- | -- | A | -- | |
| | -- | -- | -- | | | | | | | | 1 h | -- | -- | -- | A | -- | |
| | -- | -- | -- | | | | | | | | 2 h | -- | -- | -- | A | -- | |
| | -- | -- | -- | | | | | | | | 3 h | -- | -- | -- | A | -- | |
| | -- | -- | -- | | | | | | 150mM KCl | | 0 h | -- | -- | -- | A | -- | |
| | -- | -- | -- | | | | | | | | 1 h | -- | -- | -- | A | -- | |
| | -- | -- | -- | | | | | | | | 2 h | -- | -- | -- | A | -- | |
| | -- | -- | -- | | | | | | | | 3 h | -- | -- | -- | A | -- | |
| | -- | -- | -- | | | | | | 100mM MgCl₂ | | 0 h | -- | -- | -- | B | -- | |
| | -- | -- | -- | | | | | | | | 1 h | -- | -- | -- | B | -- | |
| | -- | -- | -- | | | | | | | | 2 h | -- | -- | -- | B | -- | |
| 2 | -- | -- | -- | 4e7/ml | -- | 5ml | 37°C | 0 | | R.T. | 3 h | -- | -- | -- | B | -- | Exmp.12-4 |
| | -- | -- | -- | | | | | | 11% Trehalose | | 0 h | -- | -- | -- | A | -- | |
| | -- | -- | -- | | | | | | | | 1 h | -- | -- | -- | A | -- | |
| | -- | -- | -- | | | | | | | | 2 h | -- | -- | -- | A | -- | |
| | -- | -- | -- | | | | | | | | 3 h | -- | -- | -- | A | -- | |
| | -- | -- | -- | | | | | | 10% Sucrose | | 0 h | -- | -- | -- | A | -- | |
| | -- | -- | -- | | | | | | | | 1 h | -- | -- | -- | A | -- | |
| | -- | -- | -- | | | | | | | | 2 h | -- | -- | -- | A | -- | |
| | -- | -- | -- | | | | | | | | 3 h | -- | -- | -- | A | -- | |
| | -- | -- | -- | | | | | | 5% Sucrose | | 0 h | -- | -- | -- | A | -- | |
| | -- | -- | -- | | | | | | | | 1 h | -- | -- | -- | A | -- | |
| | -- | -- | -- | | | | | | | | 2 h | -- | -- | -- | A | -- | |
| | -- | -- | -- | | | | | | | | 3 h | -- | -- | -- | A | -- | |
| | -- | -- | -- | | | | | | 10% UG in PlasmaLyte | | 0 h | -- | -- | -- | A | -- | |
| | -- | -- | -- | | | | | | | | 1 h | -- | -- | -- | A | -- | |
| | -- | -- | -- | | | | | | | | 2 h | -- | -- | -- | A | -- | |
| | -- | -- | -- | | | | | | | | 3 h | -- | -- | -- | A | -- | |
| | -- | -- | -- | | | | | | 20% UG in PlasmaLyte | | 0 h | -- | -- | -- | A | -- | |
| | -- | -- | -- | | | | | | | | 1 h | -- | -- | -- | A | -- | |
| | -- | -- | -- | | | | | | | | 2 h | -- | -- | -- | A | -- | |
| | -- | -- | -- | | | | | | | | 3 h | -- | -- | -- | A | -- | |
| | -- | -- | -- | | -- | | | | PlasmaLyte | | 1 h | -- | -- | -- | A | -- | |
| | -- | -- | -- | | | | | | | | 2 h | -- | -- | -- | A | -- | |
| | -- | -- | -- | | | | | | | | 3 h | -- | -- | -- | A | -- | |
| | -- | -- | -- | | | | | | Saline | | 1 h | -- | -- | -- | A | -- | |
| | -- | -- | -- | | | | | | | | 2 h | -- | -- | -- | A | -- | |
| | -- | -- | -- | | | | | | | | 3 h | -- | -- | -- | A | -- | |
| | -- | -- | -- | | | | | | 230mM NaCl | | 1 h | -- | -- | -- | A | -- | |
| | -- | -- | -- | | | | | | | | 2 h | -- | -- | -- | A | -- | |
| | -- | -- | -- | | | | | | | | 3 h | -- | -- | -- | A | -- | |
| | -- | -- | -- | | | | | | 150mM KCl | | 1 h | -- | -- | -- | A | -- | |
| | -- | -- | -- | | | | | | | | 2 h | -- | -- | -- | A | -- | |
| | -- | -- | -- | | | | | | | | 3 h | -- | -- | -- | B | -- | |
| | -- | -- | -- | | | | | | 100mM MgCl₂ | | 1 h | -- | -- | -- | B | -- | |
| | -- | -- | -- | | | | | | | | 2 h | -- | -- | -- | C | -- | |
| 2 | -- | -- | -- | 4e7/ml | -- | 5ml | 37°C | > 0 | | 37°C | 3 h | -- | -- | -- | C | -- | Exmp.12-5 |
| | -- | -- | -- | | | | | | 11% Trehalose | | 1 h | -- | -- | -- | A | -- | |
| | -- | -- | -- | | | | | | | | 2 h | -- | -- | -- | A | -- | |
| | -- | -- | -- | | | | | | | | 3 h | -- | -- | -- | B | -- | |
| | -- | -- | -- | | | | | | 10% Sucrose | | 1 h | -- | -- | -- | A | -- | |
| | -- | -- | -- | | | | | | | | 2 h | -- | -- | -- | A | -- | |
| | -- | -- | -- | | | | | | | | 3 h | -- | -- | -- | B | -- | |
| | -- | -- | -- | | | | | | 5% Sucrose | | 1 h | -- | -- | -- | A | -- | |
| | -- | -- | -- | | | | | | | | 2 h | -- | -- | -- | A | -- | |
| | -- | -- | -- | | | | | | | | 3 h | -- | -- | -- | B | -- | |
| | -- | -- | -- | | | | | | 10% UG in PlasmaLyte | | 1 h | -- | -- | -- | A | -- | |
| | -- | -- | -- | | | | | | | | 2 h | -- | -- | -- | A | -- | |
| | -- | -- | -- | | | | | | | | 3 h | -- | -- | -- | A | -- | |
| | -- | -- | -- | | | | | | 20% UG in PlasmaLyte | | 1 h | -- | -- | -- | A | -- | |
| | -- | -- | -- | | | | | | | | 2 h | -- | -- | -- | A | -- | |
| | -- | -- | -- | | | | | | | | 3 h | -- | -- | -- | A | -- | |

[Table 8-6]

| Collect. Method | Pretreatment Base | Additive | Time | Freezing Cell Den. | Additive | Volume | Thawing Condition | Standing | Thawing solvent 1 Base | Additive | Temp. | Time | Thawing solvent 2 Base | Additive | Time | Results Viability | Activity | Exmp No |
|---|---|---|---|---|---|---|---|---|---|---|---|---|---|---|---|---|---|---|
| | - | - | - | | - | | | | PlasmaLyte | | | 0 h | - | - | - | C | - | |
| | - | - | - | | - | | | | | | | 1 h | | | | C | - | |
| | - | - | - | | - | | | | | | | 2 h | | | | C | - | |
| | - | - | - | | - | | | | | | | 3 h | | | | C | - | |
| | - | - | - | | - | | | | Saline | | | 0 h | | | | C | - | |
| | - | - | - | | - | | | | | | | 1 h | | | | C | - | |
| | - | - | - | | - | | | | | | | 2 h | | | | C | - | |
| | - | - | - | | - | | | | | | | 3 h | | | | C | - | |
| | - | - | - | | - | | | | 230mM NaCl | | | 0 h | | | | C | - | |
| | - | - | - | | - | | | | | | | 1 h | | | | C | - | |
| | - | - | - | | - | | | | | | | 2 h | | | | C | - | |
| | - | - | - | | - | | | | | | | 3 h | | | | C | - | |
| | - | - | - | | - | | | | 150mM KCl | | | 0 h | | | | C | - | |
| | - | - | - | | - | | | | | | | 1 h | | | | C | - | |
| | - | - | - | | - | | | | | | | 2 h | | | | C | - | |
| | - | - | - | | - | | | | | | | 3 h | | | | C | - | |
| | - | - | - | | - | | | | 100mM MgCl₂ | | | 0 h | | | | C | - | |
| | - | - | - | | - | | | | | | | 1 h | | | | C | - | |
| | - | - | - | | - | | | | | | | 2 h | | | | C | - | |
| | - | - | - | | - | | | | | | | 3 h | | | | C | - | |
| | - | - | - | | - | | | | 100mM CaCl₂ | | | 0 h | | | | C | - | |
| | - | - | - | | - | | | | | | | 1 h | | | | C | - | |
| | - | - | - | | - | | | | | | | 2 h | | | | C | - | |
| | - | - | - | | - | | | | | | | 3 h | | | | C | - | |
| | - | - | - | | - | | | | 11% Trehalose | | | 0 h | | | | B | - | |
| | - | - | - | | - | | | | | | | 1 h | | | | C | - | |
| | - | - | - | | - | | | | | | | 2 h | | | | C | - | |
| | - | - | - | | - | | | | | | | 3 h | | | | C | - | |
| 1 | - | - | - | 1e7/ml | - | 1ml | 37°C | 0 | 10% Sucrose | | R.T | 0 h | | | | B | - | Exmp 12-6 |
| | - | - | - | | - | | | | | | | 1 h | | | | C | - | |
| | - | - | - | | - | | | | | | | 2 h | | | | C | - | |
| | - | - | - | | - | | | | | | | 3 h | | | | C | - | |
| | - | - | - | | - | | | | 5% Sucrose | | | 0 h | | | | B | - | |
| | - | - | - | | - | | | | | | | 1 h | | | | C | - | |
| | - | - | - | | - | | | | | | | 2 h | | | | C | - | |
| | - | - | - | | - | | | | | | | 3 h | | | | C | - | |
| | - | - | - | | - | | | | 10% UG in PlasmaLyte | | | 0 h | | | | B | - | |
| | - | - | - | | - | | | | | | | 1 h | | | | C | - | |
| | - | - | - | | - | | | | | | | 2 h | | | | C | - | |
| | - | - | - | | - | | | | | | | 3 h | | | | C | - | |
| | - | - | - | | - | | | | 20% UG in PlasmaLyte | | | 0 h | | | | B | - | |
| | - | - | - | | - | | | | | | | 1 h | | | | C | - | |
| | - | - | - | | - | | | | | | | 2 h | | | | C | - | |
| | - | - | - | | - | | | | | | | 3 h | | | | C | - | |
| | - | - | - | | - | | | | 10% AB in PlasmaLyte | | | 0 h | | | | B | - | |
| | - | - | - | | - | | | | | | | 1 h | | | | C | - | |
| | - | - | - | | - | | | | | | | 2 h | | | | C | - | |
| | - | - | - | | - | | | | | | | 3 h | | | | C | - | |
| | - | - | - | | - | | | | 20% AB in PlasmaLyte | | | 0 h | | | | B | - | |
| | - | - | - | | - | | | | | | | 1 h | | | | C | - | |
| | - | - | - | | - | | | | | | | 2 h | | | | C | - | |
| | - | - | - | | - | | | | | | | 3 h | | | | C | - | |
| | - | - | - | | - | | | | 30% AB in PlasmaLyte | | | 0 h | | | | B | - | |
| | - | - | - | | - | | | | | | | 1 h | | | | C | - | |
| | - | - | - | | - | | | | | | | 2 h | | | | C | - | |
| | - | - | - | | - | | | | | | | 3 h | | | | C | - | |
| | - | - | - | | - | | | | 40% AB in PlasmaLyte | | | 0 h | | | | B | - | |
| | - | - | - | | - | | | | | | | 1 h | | | | C | - | |
| | - | - | - | | - | | | | | | | 2 h | | | | C | - | |
| | - | - | - | | - | | | | | | | 3 h | | | | C | - | |
| | - | - | - | | - | | | | PlasmaLyte | | | 1 h | | | | C | - | |
| | - | - | - | | - | | | | | | | 2 h | | | | C | - | |
| | - | - | - | | - | | | | | | | 3 h | | | | C | - | |
| | - | - | - | | - | | | | Saline | | | 1 h | | | | C | - | |
| | - | - | - | | - | | | | | | | 2 h | | | | C | - | |
| | - | - | - | | - | | | | | | | 3 h | | | | C | - | |
| | - | - | - | | - | | | | 230mM NaCl | | | 1 h | | | | C | - | |
| | - | - | - | | - | | | | | | | 2 h | | | | C | - | |
| | - | - | - | | - | | | | | | | 3 h | | | | C | - | |
| | - | - | - | | - | | | | 150mM KCl | | | 1 h | | | | C | - | |
| | - | - | - | | - | | | | | | | 2 h | | | | C | - | |
| | - | - | - | | - | | | | | | | 3 h | | | | C | - | |
| | - | - | - | | - | | | | 100mM MgCl₂ | | | 1 h | | | | C | - | |
| | - | - | - | | - | | | | | | | 2 h | | | | C | - | |
| | - | - | - | | - | | | | | | | 3 h | | | | C | - | |
| | - | - | - | | - | | | | 100mM CaCl₂ | | | 1 h | | | | C | - | |
| | - | - | - | | - | | | | | | | 2 h | | | | C | - | |
| | - | - | - | | - | | | | | | | 3 h | | | | C | - | |
| | - | - | - | | - | | | | 11% Trehalose | | | 1 h | | | | C | - | |
| | - | - | - | | - | | | | | | | 2 h | | | | C | - | |
| | - | - | - | | - | | | | | | | 3 h | | | | C | - | |
| 1 | - | - | - | 1e7/ml | - | 1ml | 37°C | 0 | 10% Sucrose | | 37°C | 1 h | | | | C | - | Exmp 12-7 |
| | - | - | - | | - | | | | | | | 2 h | | | | C | - | |
| | - | - | - | | - | | | | | | | 3 h | | | | C | - | |
| | - | - | - | | - | | | | 5% Sucrose | | | 1 h | | | | C | - | |
| | - | - | - | | - | | | | | | | 2 h | | | | C | - | |
| | - | - | - | | - | | | | | | | 3 h | | | | C | - | |
| | - | - | - | | - | | | | 10% UG in PlasmaLyte | | | 1 h | | | | C | - | |
| | - | - | - | | - | | | | | | | 2 h | | | | C | - | |
| | - | - | - | | - | | | | | | | 3 h | | | | C | - | |
| | - | - | - | | - | | | | 20% UG in PlasmaLyte | | | 1 h | | | | C | - | |
| | - | - | - | | - | | | | | | | 2 h | | | | C | - | |
| | - | - | - | | - | | | | | | | 3 h | | | | C | - | |
| | - | - | - | | - | | | | 10% AB in PlasmaLyte | | | 1 h | | | | C | - | |
| | - | - | - | | - | | | | | | | 2 h | | | | C | - | |
| | - | - | - | | - | | | | | | | 3 h | | | | C | - | |
| | - | - | - | | - | | | | 20% AB in PlasmaLyte | | | 1 h | | | | C | - | |
| | - | - | - | | - | | | | | | | 2 h | | | | C | - | |
| | - | - | - | | - | | | | | | | 3 h | | | | C | - | |
| | - | - | - | | - | | | | 30% AB in PlasmaLyte | | | 1 h | | | | C | - | |
| | - | - | - | | - | | | | | | | 2 h | | | | C | - | |
| | - | - | - | | - | | | | | | | 3 h | | | | C | - | |
| | - | - | - | | - | | | | 40% AB in PlasmaLyte | | | 1 h | | | | C | - | |
| | - | - | - | | - | | | | | | | 2 h | | | | C | - | |
| | - | - | - | | - | | | | | | | 3 h | | | | C | - | |

43

[Table 7]

| | HBSS (+) | Lactoringer | Veen-F | Bicanate | Solacet F | SOLITA-T1 | SOLITA-T3 | PlasmaLite-A +Glu | RPMI1640 (Glu) | PlasmaLite-A +Ca²⁺ | PlasmaLite-A +Ca²⁺ | PBS(-) | PBS(-) +Mg²⁺ | Saline | PlasmaLite-A | PlasmaLite-A +Ca²⁺ | Saline |
|---|---|---|---|---|---|---|---|---|---|---|---|---|---|---|---|---|---|
| $CaCl_2$ | 1,25 | 1,36 | 1,36 | 1,5 | 1,36 | | | | | 0,423 | 1,36 | | | | | 0,042 | Calcium chloride |
| $MgCl_2$ | | | | 2,1 | | | | 1,48 | | 1,48 | 1,48 | | 1,48 | | 1,48 | 1,48 | Magnesium chloride |
| $MgSO_4$ | 0,81 | | | | | | | | 0,406 | | | | | | | | Magnesium sulfate |
| $KCl$ | 5,37 | 4,02 | 4,02 | 4,02 | 4,02 | | 20 | 4,96 | 5,37 | 4,96 | 4,96 | 2,68 | 2,68 | | 4,96 | 4,96 | Potassium chloride |
| $KH_2PO_4$ | 0,44 | | | | | | | | | | | 1,47 | 1,47 | | | | Potassium hydrogenphosphate |
| $NaHCO_3$ | 4,12 | | | 27,97 | | | | | 23,8 | | | | | | | | Sodium hydrogencarbonate |
| $NaCl$ | 136,9 | 102,7 | 102,7 | 9,99 | 102,7 | 70,8 | 15,4 | 90 | 102,7 | 90 | 90 | 136,9 | 136,9 | 154 | 90 | 90 | Sodium chloride |
| $Na_2HPO_4$ | 0,34 | | | | | | | | 5,64 | | | 8,1 | 8,1 | | | | Disodium hydrogenphosphate |
| $C_6H_{11}NaO_7$ | | | | | | | | 23,01 | | 23,01 | 23,01 | | | | 23,01 | 23,01 | Sodium gluconate |
| $C_2H_3NaO_2$ | | | 27,9 | | 27,9 | | | 27,05 | | 27,05 | 27,05 | | | | 27,05 | 27,05 | Sodium acetate |
| | | | | | | | | | 0,423 | | | | | | | | Potassium nitrate |
| $HCl$ | | | pH Adjustment | | pH Adjustment | | | | | | | | | | | | Hydrochloric acid |
| $Na_3C_6H_5O_7$ | | | | 0,68 | | | | | | | | | | | | | Sodium citrate |
| $C_3H_5O_3Na$ | | 27,7 | | | | 20 | 20 | | | | | | | | | | Sodium lactate |
| Amino Acids | | | | | | | | | O | | | | | | | | Amino acid |
| Vitamins | | | | | | | | | O | | | | | | | | Vitamins |
| Phenol red | | | | | | | | | 0,0141 | | | | | | | | Phenol Red |
| Maltose | | 138,8 | | | | | | | | | | | | | | | Maltose |
| D-Glucose | 5,55 | | | | | 144,3 | 238,7 | 5,55 | | | | | | | | | Glucose |

[Summary of Examples 1 to 12]

The results described above are summarized in the tables mentioned below. In the tables mentioned below, the unit for time is minute, except when indicated as "xx hour(s)". For Temperature, "R.T." represents room temperature, otherwise the temperature was 37°C. For Viability, "A" means 70% or higher, "B" means 50% or higher and lower than 70%, "C" means lower than 50%, and "-" means not evaluated. For Activity, A means 70% or higher, B means 50% or higher and lower than 70%, C means lower than 50%, and - means not evaluated. It can be said that evaluation result of A or B means that the object was achieved.

[Table 8-1]

| Collect. Method | Pretreatment Base | Pretreatment Additive | Pretreatment Time | Freezing Cell Den. | Freezing Additive | Freezing Volume | Thawing Condition | Thawing Standing | Solvent 1 Base | Solvent 1 Additive/Temp. | Solvent 1 Time | Solvent 2 Base | Solvent 2 Additive | Solvent 2 Time | Viability | Activity | Exmp.No. |
|---|---|---|---|---|---|---|---|---|---|---|---|---|---|---|---|---|---|
| 1 | - | - | - | 0.8-1e7/m | - | 1ml | 37°C | 0 | KBM | - | 3 h | - | - | - | C | - | Exmp.1-1 |
|  |  |  |  |  |  |  |  |  | Lacto | IL-2 |  | - | - | - | C | - |  |
|  |  |  |  |  |  |  |  |  | PlasmaLyte | - |  | - | - | - | A | - |  |
|  |  |  |  |  |  |  |  |  | KBM | Gluconate |  | - | - | - | C | - |  |
| 1 | - | - | - | 0.5e7/ml | - | 1ml | 37°C | 0 | KBM | - | 3 h | - | - | - | C | - | Exmp.1-2 |
|  |  |  |  |  |  |  |  |  | Lacto | IL-2 |  | - | - | - | C | - |  |
|  |  |  |  |  |  |  |  |  | PlasmaLyte | - |  | - | - | - | A | - |  |
| 1 | - | - | - | 0.5-1e7/m | - | 1ml | 37□ | 0 | KBM | - | 3 h | - | - | - | C | - | Exmp.2 |
|  |  |  |  |  |  |  |  |  | Lacto | IL-2 |  | - | - | - | C | - |  |
|  |  |  |  |  |  |  |  |  | PlasmaLyte | IL-2 |  | - | - | - | A | - |  |
|  |  |  |  |  |  |  |  |  |  | AB Serum |  | - | - | - | C | - |  |
| 1 | - | - | - | 1e7/ml | - | 1ml | 37□ | 0 | PlasmaLyte | IL-2 | 1 h | KBM | - | 3 h | C | B | Exmp.3 |
|  |  |  |  |  |  |  |  |  |  |  |  | Lacto | - | 3 h | A | C |  |
|  |  |  |  |  |  |  |  |  |  |  |  | KBM | - | o/n | C | A |  |
|  |  |  |  |  |  |  |  |  |  |  |  | Lacto | - | o/n | C | C |  |
|  |  |  |  |  |  |  |  |  | KBM | - | 3 h | - | - | - | A | C |  |
|  |  |  |  |  |  |  |  |  |  | - | o/n | - | - | - | C | A |  |
|  |  |  |  |  |  |  |  |  |  |  |  | - | - | - | C | A |  |
| 1 | KBM | 10µM 4-PBA | - | 1e7/ml | - | 0.5mL | 37□ | 0 | PlasmaLyte | IL-2 | 1 h | KBM | - | o/n | A | A | Exmp.4 |
|  | PlasmaLyte | 10µM salbrinal |  |  |  |  |  |  |  | IL-2 4-PBA |  |  | - |  | A | A |  |
|  | KBM | 100µM 4-PBA | 2 h |  |  |  |  |  |  | IL-2 |  |  | IL-2 | o/n | C | C |  |
|  | PlasmaLyte | IL-2 |  |  |  |  |  |  |  | IL-2 4-PBA |  |  | IL-2 |  | C | C |  |
|  | PBS | Gluconate |  |  |  |  |  |  |  | IL-2 | R.T. | RPMI | IL-2 |  | C | C |  |
|  |  |  |  |  |  |  |  |  |  | IL-2 4-PBA |  |  | IL-2 |  | C | C |  |
|  |  |  |  |  |  |  |  |  |  | R.T. |  |  | IL-2 |  | A | A |  |

| | | | | | | | | | | | | | | | | | |
|---|---|---|---|---|---|---|---|---|---|---|---|---|---|---|---|---|---|
| 2 | KBM | – | 2 h | 1e7/ml | – | 0.5ml | 37□ | 0 | PlasmaLyte | R.T. | 1 h | KBM | – | 1 or 3 h | A | – | Exmp.5 |
| | | 30µM 4-PBA | | | – | | | | | | | | – | | A | – | |
| | | 30µM TUDCA | | | – | | | | | | | | – | | A | – | |
| | | 30µM 4-PBA 30µM TUDCA | | | – | | | | | | | | – | | A | – | |
| 1 | PBS | – | | | – | | | | | | | | – | | C | – | |
| 2 | KBM | - | 2 h | 1e7/ml | – | 0.5mL | 37□ | 0 | PlasmaLyte | R.T. | 1 h | – | – | – | A | B | Exmp.6 |
| | | 10µM TUDCA | | | – | | | | | | | – | – | – | A | B | |
| | | 30µM TUDCA | | | – | | | | | | | – | – | – | A | B | |
| | | 90µM TUDCA | | | – | | | | | | | – | – | – | A | B | |
| | | 270µM TUDCA | | | – | | | | | | | – | – | – | A | B | |
| | | 810µM TUDCA | | | – | | | | | | | – | – | – | A | B | |
| | KBM | - | | | – | | | | PlasmaLyte | R.T. | 3 h | | – | – | A | A | |
| | | 10µM TUDCA | | | – | | | | | | | | – | – | A | A | |
| | | 30µM TUDCA | | | – | | | | | | | | – | – | A | A | |
| | | 90µM TUDCA | | | – | | | | | | | | – | – | A | A | |
| | | 270µM TUDCA | | | – | | | | | | | | – | – | A | A | |
| | | 810µM TUDCA | | | – | | | | | | | | – | – | A | A | |
| | KBM | - | | | – | | | | PlasmaLyte | R.T. | 1 h | KBM | – | 2 h | A | A | |
| | | 10µM TUDCA | | | – | | | | | | | | – | | A | A | |
| | | 30µM TUDCA | | | – | | | | | | | | – | | A | A | |
| | | 90µM TUDCA | | | – | | | | | | | | – | | A | A | |
| | | 270µM TUDCA | | | – | | | | | | | | – | | A | A | |
| | | 810µM TUDCA | | | – | | | | | | | | – | | A | A | |

| | | | | | | | | | | | | | | | | | |
|---|---|---|---|---|---|---|---|---|---|---|---|---|---|---|---|---|---|
| 2 | KBM | 2400μM TUDCA | 2 h | 1e7/ml | – | 0.5mL | 37□ | 0 | PlasmaLyte | R.T. | 1 h | – | – | – | A | A | Exmp.7 |
| | | | | | – | | | | | | | KBM | – | 2 h | A | A | |
| | | | | | – | | | | | | 3 h | – | – | – | A | A | |
| | | | | | – | | | | | IL-2, R.T. | 1 h | – | – | – | A | A | |
| | | | | | – | | | | | | | KBM | – | 2 h | A | A | |
| | | 800μM TUDCA | | | – | | | | | | 3 h | – | – | – | A | A | |
| | | | | | – | | | | | R.T. | 1 h | – | – | – | A | A | |
| | | | | | – | | | | | | | KBM | – | 2 h | A | A | |
| | | | | | – | | | | | | 3 h | – | – | – | A | A | |
| | | | | | – | | | | | IL-2, R.T. | 1 h | – | – | – | A | A | |
| | | | | | – | | | | | | | KBM | – | 2 h | A | A | |
| | | 266μM TUDCA | | | – | | | | | | 3 h | – | – | – | A | A | |
| | | | | | – | | | | | R.T. | 1 h | – | – | – | A | A | |
| | | | | | – | | | | | | | KBM | – | 2 h | A | A | |
| | | | | | – | | | | | | 3 h | – | – | – | A | A | |
| | | | | | – | | | | | IL-2, R.T. | 1 h | – | – | – | A | A | |
| | | – | | | – | | | | | | | KBM | – | 2 h | A | A | |
| | | | | | – | | | | | | 3 h | – | – | – | A | A | |
| | | | | | – | | | | | R.T. | 1 h | – | – | – | A | A | |
| | | | | | – | | | | | | | KBM | – | 2 h | A | A | |
| | | | | | – | | | | | | 3 h | – | – | – | A | A | |
| | | | | | – | | | | | IL-2, R.T. | 1 h | – | – | – | A | A | |
| | | 2.4% DMSO | | | – | | | | | | | KBM | – | 2 h | A | A | |
| | | | | | – | | | | | | 3 h | – | – | – | A | A | |

48

[Table 8-2]

| Collect. Method | Pretreatment Base | Pretreatment Additive | Pretreatment Time | Freezing Cell Den. | Freezing Additive | Freezing Volume | Thawing Condition | Thawing Standing | Thawing solvent 1 Base | Thawing solvent 1 Additive/Temp. | Thawing solvent 1 Time | Thawing 2 Base | Thawing 2 Additive | Thawing 2 Time | Results Viability | Results Activity | Exmp.No. |
|---|---|---|---|---|---|---|---|---|---|---|---|---|---|---|---|---|---|
| 2 | – | – | – | 1e7/ml | – | 1ml | 37℃ | 0 | PlasmaLyte | R.T. | 2 h | – | – | – | A | – | Exmp.8-1 |
|  |  |  |  |  | – |  |  |  | PBS | 1.48mMMgCl₂ R.T. |  | – | – | – | A | – |  |
|  |  |  |  |  | – |  |  |  | PBS | R.T. |  | – | – | – | A | – |  |
|  |  |  |  |  | – |  |  |  | KBM | R.T. |  | – | – | – | C | – |  |
|  |  |  |  |  | – |  |  |  | PlasmaLyte | – |  | – | – | – | A | – |  |
|  |  |  |  |  | – |  |  |  | PlasmaLyte | 0.042mM CaCl₂ |  | – | – | – | A | – |  |
|  |  |  |  |  | – |  |  |  | PlasmaLyte | 0.1% glucose |  | – | – | – | A | – |  |
|  |  |  |  |  | – |  |  |  | RPMI | Glu(-), FBS(-) |  | – | – | – | C | – |  |
|  |  |  |  |  | – |  |  |  | RPMI | Glu(+), 10%FBS |  | – | – | – | C | – |  |
| 1 | – | – | – | 1e7/ml | – | 1ml | 37℃ | 0 | HBSS(+) | – | 3 h | – | – | – | C | – | Exmp.8-2 |
|  |  |  |  |  | – |  |  |  | KBM | – |  | – | – | – | C | – |  |
|  |  |  |  |  | – |  |  |  | Lacto | – |  | – | – | – | C | – |  |
|  |  |  |  |  | – |  |  |  | PBS | 1.48mMMgCl₂ |  | – | – | – | A | – |  |
|  |  |  |  |  | – |  |  |  | PlasmaLyte | – |  | – | – | – | A | – |  |
| 2 |  |  |  |  | – |  |  |  | HBSS(+) | – |  | – | – | – | A | – |  |
|  |  |  |  |  | – |  |  |  | KBM | – |  | – | – | – | B | – |  |
|  |  |  |  |  | – |  |  |  | Lacto | – |  | – | – | – | B | – |  |
|  |  |  |  |  | – |  |  |  | PBS | 1.48mMMgCl₂ |  | – | – | – | B | – |  |
|  |  |  |  |  | – |  |  |  | PlasmaLyte | – |  | – | – | – | A | – |  |
|  |  |  |  |  | – |  |  |  | PBS | 1.48mMMgCl₂ |  | – | – | – | A | – |  |
|  |  |  |  |  | – |  |  |  | PlasmaLyte | – |  | – | – | – | A | – |  |
| 2 | – | – | – | 1e7/ml | – | 1ml | 37℃ | 0 | PBS | 1.48mMMgCl₂ | 3 h | – | – | – | B | – | Exmp.8-3 |
|  |  |  |  |  | – |  |  |  | PlasmaLyte | 27.7mEq 乳酸 48.84mg/ml MgSO₄ |  | – | – | – | A | – |  |
|  |  |  |  |  | – |  |  |  | Lacto | – |  | – | – | – | C | – |  |
|  |  |  |  |  | – |  |  |  | Veen-F | – |  | – | – | – | C | – |  |
|  |  |  |  |  | – |  |  |  | SOLITA-T1 | – |  | – | – | – | C | – |  |
|  |  |  |  |  | – |  |  |  | SOLITA-T3 | 5% Glu |  | – | – | – | C | – |  |
|  |  |  |  |  | – |  |  |  | RPMI | – |  | – | – | – | C | – |  |

| Example | | | Time | Medium | Additive | | Temp | Volume | Conc. | No. |
|---|---|---|---|---|---|---|---|---|---|---|
| **Exmp.8-4** | – | C | 3 h | PlasmaLyte | – | 0 | 37℃ | 1ml | 1e7/ml | 1 |
| | – | C | | PBS | – | | | | | |
| | – | C | | Saline | – | | | | | |
| | – | C | | BICANATE | – | | | | | |
| | – | A | | SOLACET-F | – | | | | | |
| | – | A | | PlasmaLyte | – | | | | | 2 |
| | – | A | | PBS | – | | | | | |
| | – | C | | Saline | – | | | | | |
| | – | C | | BICANATE | – | | | | | |
| | | | | SOLACET-F | – | | | | | |
| **Exmp.8-5** | – | A | 3 h | PlasmaLyte | 0.423mM CaCl₂ | 0 | 37℃ | 1ml | 1e7/ml | 2 |
| | – | B | | | 1.36mM CaCl₂ | | | | | |
| | – | B | | RPMI | – | | | | | |
| | – | C | | Lacto | – | | | | | |
| | – | B | | Saline | – | | | | | |
| | – | A | | PlasmaLyte | 0.423mM CaCl₂ | | | | | 1 |
| | – | C | | | 1.36mM CaCl₂ | | | | | |
| | – | C | | RPMI | – | | | | | |
| | – | C | | Lacto | – | | | | | |
| | – | C | | Saline | – | | | | | |
| **Exmp.9-1** | A | A | 0 h | PlasmaLyte | 3,000IU/ml IL-2 R.T. | 0 | 37℃ | 5ml | 4e7/ml | 2 |
| | A | A | 1 h | | | | | | | |
| | A | A | 2 h | | | | | | | |
| | A | A | 3 h | | | | | | | |
| | A | A | 4 h | | | | | | | |
| **Exmp.9-2** | A | – | 2 h | PlasmaLyte | 3,000IU/ml IL-2 R.T. | 0 | 37℃ | 5ml | 4e7/ml | 2 |
| | A | – | 5 h | | | | | | | |
| | A | A | 6 h | | | | | | | |

[Table 8-3]

| Collect. Method | Pretreatment Base | Pretreatment Additive | Pretreatment Time | Freezing Cell Den. | Freezing Additive | Freezing Volume | Thawing Condition | Thawing Standing | Thawing solvent 1 Base | Thawing solvent 1 Additive/Temp. | Thawing solvent 1 Time | Thawing solvent 2 Base | Thawing solvent 2 Additive | Thawing solvent 2 Time | Results Viability | Results Activity | Exmp.No. |
|---|---|---|---|---|---|---|---|---|---|---|---|---|---|---|---|---|---|
| 2 | -- | -- | -- | 4e7/ml | -- | 5ml | R.T. | 0 | KBM | -- | 180 | -- | -- | -- | B | -- | |
| | | | | | | | | 5 | | -- | 175 | -- | -- | -- | A | -- | |
| | | | | | | | | 10 | | -- | 170 | -- | -- | -- | A | -- | |
| | | | | | | | | 15 | | -- | 165 | -- | -- | -- | A | -- | |
| | | | | | | | | 20 | | -- | 160 | -- | -- | -- | A | -- | |
| | | | | | | | | 25 | | -- | 155 | -- | -- | -- | A | -- | |
| | | | | | | | | 30 | | -- | 150 | -- | -- | -- | A | -- | |
| | | | | | | | | 60 | | -- | 120 | -- | -- | -- | A | -- | |
| | | | | | | | | 120 | | -- | 60 | -- | -- | -- | A | -- | |
| | | | | | | | | 180 | | -- | 0 | -- | -- | -- | B | -- | |
| | | | | | | | | 0 | PlasmaLyte | -- | 180 | -- | -- | -- | A | -- | |
| | | | | | | | | 5 | | -- | 175 | -- | -- | -- | A | -- | |
| | | | | | | | | 10 | | -- | 170 | -- | -- | -- | A | -- | |
| | | | | | | | | 15 | | -- | 165 | -- | -- | -- | A | -- | |
| | | | | | | | | 20 | | -- | 160 | -- | -- | -- | A | -- | |
| | | | | | | | | 25 | | -- | 155 | -- | -- | -- | A | -- | |
| | | | | | | | | 30 | | -- | 150 | -- | -- | -- | A | -- | Exmp.10-1 |
| | | | | | | | | 60 | | -- | 120 | -- | -- | -- | A | -- | |
| | | | | | | | | 120 | | -- | 60 | -- | -- | -- | A | -- | |
| | | | | | | | | 180 | | -- | 0 | -- | -- | -- | A | -- | |
| 1 | -- | -- | -- | 1e7/ml | -- | 1ml | R.T. | 0 | KBM | -- | 180 | -- | -- | -- | C | -- | |
| | | | | | | | | 5 | | -- | 175 | -- | -- | -- | C | -- | |
| | | | | | | | | 10 | | -- | 170 | -- | -- | -- | C | -- | |
| | | | | | | | | 15 | | -- | 165 | -- | -- | -- | C | -- | |
| | | | | | | | | 20 | | -- | 160 | -- | -- | -- | C | -- | |
| | | | | | | | | 25 | | -- | 155 | -- | -- | -- | C | -- | |
| | | | | | | | | 30 | | -- | 150 | -- | -- | -- | C | -- | |
| | | | | | | | | 60 | | -- | 120 | -- | -- | -- | C | -- | |
| | | | | | | | | 120 | | -- | 60 | -- | -- | -- | C | -- | |
| | | | | | | | | 180 | | -- | 0 | -- | -- | -- | C | -- | |
| | | | | | | | | 0 | PlasmaLyte | -- | 180 | -- | -- | -- | C | -- | |
| | | | | | | | | 5 | | -- | 175 | -- | -- | -- | C | -- | |
| | | | | | | | | 10 | | -- | 170 | -- | -- | -- | C | -- | |
| | | | | | | | | 15 | | -- | 165 | -- | -- | -- | C | -- | |
| | | | | | | | | 20 | | -- | 160 | -- | -- | -- | C | -- | |
| | | | | | | | | 25 | | -- | 155 | -- | -- | -- | C | -- | |
| | | | | | | | | 30 | | -- | 150 | -- | -- | -- | C | -- | |
| | | | | | | | | 60 | | -- | 120 | -- | -- | -- | C | -- | |
| | | | | | | | | 120 | | -- | 60 | -- | -- | -- | C | -- | |
| | | | | | | | | 180 | | -- | 0 | -- | -- | -- | C | -- | |

| | Group | Conc | Volume | Temp | Medium | Time | | Value | | | | Grade | | Example |
|---|---|---|---|---|---|---|---|---|---|---|---|---|---|---|
| | | | | | | 0 | — | 180 | — | — | — | B | — | |
| | | | | | | 5 | — | 175 | — | — | — | B | — | |
| | | | | | | 10 | — | 170 | — | — | — | B | — | |
| | | | | | | 15 | — | 165 | — | — | — | A | — | |
| | | | | | KBM | 20 | — | 160 | — | — | — | A | — | |
| | | | | | | 25 | — | 155 | — | — | — | A | — | |
| | | | | | | 30 | — | 150 | — | — | — | A | — | |
| | | | | | | 60 | — | 120 | — | — | — | A | — | |
| | | | | | | 120 | — | 60 | — | — | — | A | — | |
| | | | | | | 180 | — | 0 | — | — | — | A | — | |
| | 2 | 4e7/ml | 5ml | | | 0 | — | 180 | — | — | — | B | — | |
| | | | | | | 5 | — | 175 | — | — | — | A | — | |
| | | | | | | 10 | — | 170 | — | — | — | A | — | |
| | | | | | | 15 | — | 165 | — | — | — | A | — | |
| | | | | | PlasmaLyte | 20 | — | 160 | — | — | — | A | — | |
| | | | | | | 25 | — | 155 | — | — | — | A | — | |
| | | | | | | 30 | — | 150 | — | — | — | A | — | |
| | | | | | | 60 | — | 120 | — | — | — | A | — | |
| | | | | | | 120 | — | 60 | — | — | — | A | — | |
| | | | | 37□ | | 180 | — | 0 | — | — | — | A | — | Exmp.10-2 |
| | | | | | | 0 | — | 180 | — | — | — | C | — | |
| | | | | | | 5 | — | 175 | — | — | — | C | — | |
| | | | | | | 10 | — | 170 | — | — | — | C | — | |
| | | | | | | 15 | — | 165 | — | — | — | C | — | |
| | | | | | KBM | 20 | — | 160 | — | — | — | C | — | |
| | | | | | | 25 | — | 155 | — | — | — | C | — | |
| | | | | | | 30 | — | 150 | — | — | — | C | — | |
| | | | | | | 60 | — | 120 | — | — | — | C | — | |
| | | | | | | 120 | — | 60 | — | — | — | C | — | |
| | | | | | | 180 | — | 0 | — | — | — | C | — | |
| | 1 | 1e7/ml | 1ml | | | 0 | — | 180 | — | — | — | C | — | |
| | | | | | | 5 | — | 175 | — | — | — | C | — | |
| | | | | | | 10 | — | 170 | — | — | — | C | — | |
| | | | | | | 15 | — | 165 | — | — | — | C | — | |
| | | | | | PlasmaLyte | 20 | — | 160 | — | — | — | C | — | |
| | | | | | | 25 | — | 155 | — | — | — | C | — | |
| | | | | | | 30 | — | 150 | — | — | — | C | — | |
| | | | | | | 60 | — | 120 | — | — | — | C | — | |
| | | | | | | 120 | — | 60 | — | — | — | C | — | |
| | | | | | | 180 | — | 0 | — | — | — | C | — | |

52

| | Exmp.11 | | | | | | | | | | | | | | | | | | | | |
|---|---|---|---|---|---|---|---|---|---|---|---|---|---|---|---|---|---|---|---|---|---|
| | A | A | A | A | A | A | B | B | B | A | A | A | B | C | C | C | C | C | C | C | C |
| | 0 h | 1 h | 2 h | 3 h | 6 h | 0 h | 1 h | 2 h | 3 h | 6 h | 0 h | 1 h | 2 h | 3 h | 6 h | 0 h | 1 h | 2 h | 3 h | 6 h | |
| | PlasmaLyte | | | | KBM | | | | | PlasmaLyte | | | | KBM | | | | | PlasmaLyte | | |
| 0 | | | | | | | | | | | | | | | | | | | | | |
| 37 □ | | | | | | | | | | | | | | | | | | | | | |
| 5ml | | | | | | | | | | 1ml | | | | | | | | | | | |
| 4e7/ml | | | | | | | | | | 1e7/ml | | | | | | | | | | | |
| 2 | | | | | | | | | | 1 | | | | | | | | | | | |

53

EP 4 151 717 A1

[Table 8-4]

| Collect. Method | Pretreatment | | | Freezing | | | Thawing | | Thawing solvent 1 | | | Thawing solvent 2 | | | Results | | Exmp.No. |
|---|---|---|---|---|---|---|---|---|---|---|---|---|---|---|---|---|---|
| | Base | Additive | Time | Cell Den. | Additive | Volume | Condition | Standing | Base | Additive/Temp. | Time | Base | Additive | Time | Viability | Activity | |
| 2 | − | − | − | 4e7/ml | − | 5ml | 37□ | 0 | 100% PL | R.T. | 0 h | − | − | − | A | − | Exmp.12-1 |
| | − | − | − | | − | | | | | | 1 h | − | − | − | A | − | |
| | − | − | − | | − | | | | | | 2 h | − | − | − | A | − | |
| | − | − | − | | − | | | | | | 3 h | − | − | − | A | − | |
| | − | − | − | | − | | | | 80% PL 20% dH₂O | R.T. | 0 h | − | − | − | A | − | |
| | − | − | − | | − | | | | | | 1 h | − | − | − | A | − | |
| | − | − | − | | − | | | | | | 2 h | − | − | − | A | − | |
| | − | − | − | | − | | | | | | 3 h | − | − | − | A | − | |
| | − | − | − | | − | | | | | 37□ | 0 h | − | − | − | A | − | |
| | − | − | − | | − | | | | | | 1 h | − | − | − | A | − | |
| | − | − | − | | − | | | | | | 2 h | − | − | − | A | − | |
| | − | − | − | | − | | | | | | 3 h | − | − | − | A | − | |
| | − | − | − | | − | | | | 50% PL 50% dH₂O | R.T. | 0 h | − | − | − | A | − | |
| | − | − | − | | − | | | | | | 1 h | − | − | − | A | − | |
| | − | − | − | | − | | | | | | 2 h | − | − | − | A | − | |
| | − | − | − | | − | | | | | | 3 h | − | − | − | A | − | |
| | − | − | − | | − | | | | | 37□ | 0 h | − | − | − | A | − | |
| | − | − | − | | − | | | | | | 1 h | − | − | − | A | − | |
| | − | − | − | | − | | | | | | 2 h | − | − | − | A | − | |
| | − | − | − | | − | | | | | | 3 h | − | − | − | A | − | |
| | − | − | − | | − | | | | 10% PL 90% dH₂O | R.T. | 0 h | − | − | − | A | − | |
| | − | − | − | | − | | | | | | 1 h | − | − | − | A | − | |
| | − | − | − | | − | | | | | | 2 h | − | − | − | A | − | |
| | − | − | − | | − | | | | | | 3 h | − | − | − | A | − | |
| | − | − | − | | − | | | | | 37□ | 0 h | − | − | − | − | − | |
| | − | − | − | | − | | | | | | 1 h | − | − | − | B | − | |
| | − | − | − | | − | | | | | | 2 h | − | − | − | B | − | |
| | − | − | − | | − | | | | | | 3 h | − | − | − | B | − | |

| | | | | | | | | | | | | | | | |
|---|---|---|---|---|---|---|---|---|---|---|---|---|---|---|---|
| Exmp.12-2 | | | A | | | 0 h | R.T. | 100% PL | 0 | 37℃ | 5ml | 4e7/ml | 2 | − | − |
| | | − | A | − | − | 1 h | | | | | | | | − | − |
| | | − | A | − | − | 2 h | | | | | | | | − | − |
| | | − | A | − | − | 3 h | | | | | | | | − | − |
| | | − | A | − | − | 0 h | R.T. | 80% PL 20% Saline | | | | | | − | − |
| | | − | A | − | − | 1 h | | | | | | | | − | − |
| | | − | A | − | − | 2 h | | | | | | | | − | − |
| | | − | A | − | − | 3 h | | | | | | | | − | − |
| | | − | A | − | − | 0 h | 37℃ | | | | | | | − | − |
| | | − | A | − | − | 1 h | | | | | | | | − | − |
| | | − | A | − | − | 2 h | | | | | | | | − | − |
| | | − | A | − | − | 3 h | | | | | | | | − | − |
| | | − | A | − | − | 0 h | R.T. | 50% PL 50% Saline | | | | | | − | − |
| | | − | A | − | − | 1 h | | | | | | | | − | − |
| | | − | A | − | − | 2 h | | | | | | | | − | − |
| | | − | A | − | − | 3 h | | | | | | | | − | − |
| | | − | A | − | − | 0 h | 37℃ | | | | | | | − | − |
| | | − | A | − | − | 1 h | | | | | | | | − | − |
| | | − | A | − | − | 2 h | | | | | | | | − | − |
| | | − | A | − | − | 3 h | | | | | | | | − | − |
| | | − | A | − | − | 0 h | R.T. | 10% PL 90% Saline | | | | | | − | − |
| | | − | A | − | − | 1 h | | | | | | | | − | − |
| | | − | A | − | − | 2 h | | | | | | | | − | − |
| | | − | A | − | − | 3 h | | | | | | | | − | − |
| | | − | A | − | − | 0 h | 37℃ | | | | | | | − | − |
| | | − | A | − | − | 1 h | | | | | | | | − | − |
| | | − | A | − | − | 2 h | | | | | | | | − | − |
| | | − | A | − | − | 3 h | | | | | | | | − | − |

| | | | | | | | | | PL composition | Temp. | Time | | | | | | Exmp. |
|---|---|---|---|---|---|---|---|---|---|---|---|---|---|---|---|---|---|
| 2 | – | – | – | 4e7/ml | – | 5ml | 37℃ | 0 | 100% PL | R.T. | 0 h | – | – | – | A | – | Exmp.12-3 |
| | – | – | – | – | | | | | | | 1 h | – | – | – | A | – | |
| | – | – | – | – | | | | | | | 2 h | – | – | – | A | – | |
| | – | – | – | – | | | | | | | 3 h | – | – | – | A | – | |
| | – | – | – | – | | | | | 80% PL 20% Saline | R.T. | 0 h | – | – | – | A | – | |
| | – | – | – | – | | | | | | | 1 h | – | – | – | A | – | |
| | – | – | – | – | | | | | | | 2 h | – | – | – | A | – | |
| | – | – | – | – | | | | | | | 3 h | – | – | – | A | – | |
| | – | – | – | – | | | | | | 37℃ | 0 h | – | – | – | A | – | |
| | – | – | – | – | | | | | | | 1 h | – | – | – | A | – | |
| | – | – | – | – | | | | | | | 2 h | – | – | – | A | – | |
| | – | – | – | – | | | | | | | 3 h | – | – | – | A | – | |
| | – | – | – | – | | | | | 50% PL 50% Saline | R.T. | 0 h | – | – | – | A | – | |
| | – | – | – | – | | | | | | | 1 h | – | – | – | A | – | |
| | – | – | – | – | | | | | | | 2 h | – | – | – | A | – | |
| | – | – | – | – | | | | | | | 3 h | – | – | – | A | – | |
| | – | – | – | – | | | | | | 37℃ | 0 h | – | – | – | A | – | |
| | – | – | – | – | | | | | | | 1 h | – | – | – | A | – | |
| | – | – | – | – | | | | | | | 2 h | – | – | – | A | – | |
| | – | – | – | – | | | | | | | 3 h | – | – | – | A | – | |
| | – | – | – | – | | | | | 10% PL 90% Saline | R.T. | 0 h | – | – | – | A | – | |
| | – | – | – | – | | | | | | | 1 h | – | – | – | A | – | |
| | – | – | – | – | | | | | | | 2 h | – | – | – | A | – | |
| | – | – | – | – | | | | | | | 3 h | – | – | – | A | – | |
| | – | – | – | – | | | | | | 37℃ | 0 h | – | – | – | A | – | |
| | – | – | – | – | | | | | | | 1 h | – | – | – | A | – | |
| | – | – | – | – | | | | | | | 2 h | – | – | – | A | – | |
| | – | – | – | – | | | | | | | 3 h | – | – | – | A | – | |
| | – | – | – | – | | | | | Saline | R.T. | 0 h | – | – | – | A | – | |
| | – | – | – | – | | | | | | | 1 h | – | – | – | A | – | |
| | – | – | – | – | | | | | | | 2 h | – | – | – | A | – | |
| | – | – | – | – | | | | | | | 3 h | – | – | – | A | – | |
| | – | – | – | – | | | | | | 37℃ | 0 h | – | – | – | A | – | |
| | – | – | – | – | | | | | | | 1 h | – | – | – | A | – | |
| | – | – | – | – | | | | | | | 2 h | – | – | – | A | – | |
| | – | – | – | – | | | | | | | 3 h | – | – | – | A | – | |

[Table 8-5]

| Collect. | Pretreatment | | | Freezing | | | Thawing | | Thawing solvent 1 | | | Thawing solvent 2 | | | Results | | |
|---|---|---|---|---|---|---|---|---|---|---|---|---|---|---|---|---|---|
| Method | Base | Additive | Time | Cell Den. | Additive | Volume | Condition | Standing | Base | Additive/Temp. | Time | Base | Additive | Time | Viability | Activity | Exmp.No. |
| 2 | – | – | – | 4e7/ml | – | 5ml | 37° | 0 | PlasmaLyte | R.T. | 0 h | – | – | – | A | – | Exmp.12-4 |
|  | – | – | – |  | – |  |  |  | PlasmaLyte |  | 1 h | – | – | – | A | – |  |
|  | – | – | – |  | – |  |  |  | PlasmaLyte |  | 2 h | – | – | – | A | – |  |
|  | – | – | – |  | – |  |  |  | PlasmaLyte |  | 3 h | – | – | – | A | – |  |
|  | – | – | – |  | – |  |  |  | Saline |  | 0 h | – | – | – | A | – |  |
|  | – | – | – |  | – |  |  |  | Saline |  | 1 h | – | – | – | A | – |  |
|  | – | – | – |  | – |  |  |  | Saline |  | 2 h | – | – | – | A | – |  |
|  | – | – | – |  | – |  |  |  | Saline |  | 3 h | – | – | – | A | – |  |
|  | – | – | – |  | – |  |  |  | 230mM NaCl |  | 0 h | – | – | – | A | – |  |
|  | – | – | – |  | – |  |  |  | 230mM NaCl |  | 1 h | – | – | – | A | – |  |
|  | – | – | – |  | – |  |  |  | 230mM NaCl |  | 2 h | – | – | – | A | – |  |
|  | – | – | – |  | – |  |  |  | 230mM NaCl |  | 3 h | – | – | – | A | – |  |
|  | – | – | – |  | – |  |  |  | 150mM KCl |  | 0 h | – | – | – | A | – |  |
|  | – | – | – |  | – |  |  |  | 150mM KCl |  | 1 h | – | – | – | A | – |  |
|  | – | – | – |  | – |  |  |  | 150mM KCl |  | 2 h | – | – | – | A | – |  |
|  | – | – | – |  | – |  |  |  | 150mM KCl |  | 3 h | – | – | – | A | – |  |
|  | – | – | – |  | – |  |  |  | 100mM MgCl$_2$ |  | 0 h | – | – | – | B | – |  |
|  | – | – | – |  | – |  |  |  | 100mM MgCl$_2$ |  | 1 h | – | – | – | B | – |  |
|  | – | – | – |  | – |  |  |  | 100mM MgCl$_2$ |  | 2 h | – | – | – | B | – |  |
|  | – | – | – |  | – |  |  |  | 100mM MgCl$_2$ |  | 3 h | – | – | – | B | – |  |
|  | – | – | – |  | – |  |  |  | 11% Trehalose |  | 0 h | – | – | – | A | – |  |
|  | – | – | – |  | – |  |  |  | 11% Trehalose |  | 1 h | – | – | – | A | – |  |
|  | – | – | – |  | – |  |  |  | 11% Trehalose |  | 2 h | – | – | – | A | – |  |
|  | – | – | – |  | – |  |  |  | 11% Trehalose |  | 3 h | – | – | – | A | – |  |
|  | – | – | – |  | – |  |  |  | 10% Sucrose |  | 0 h | – | – | – | A | – |  |
|  | – | – | – |  | – |  |  |  | 10% Sucrose |  | 1 h | – | – | – | A | – |  |
|  | – | – | – |  | – |  |  |  | 10% Sucrose |  | 2 h | – | – | – | A | – |  |
|  | – | – | – |  | – |  |  |  | 10% Sucrose |  | 3 h | – | – | – | A | – |  |
|  | – | – | – |  | – |  |  |  | 5% Sucrose |  | 0 h | – | – | – | A | – |  |
|  | – | – | – |  | – |  |  |  | 5% Sucrose |  | 1 h | – | – | – | A | – |  |
|  | – | – | – |  | – |  |  |  | 5% Sucrose |  | 2 h | – | – | – | A | – |  |
|  | – | – | – |  | – |  |  |  | 5% Sucrose |  | 3 h | – | – | – | A | – |  |
|  | – | – | – |  | – |  |  |  | 10% UG in PlasmaLyte |  | 0 h | – | – | – | A | – |  |
|  | – | – | – |  | – |  |  |  | 10% UG in PlasmaLyte |  | 1 h | – | – | – | A | – |  |
|  | – | – | – |  | – |  |  |  | 10% UG in PlasmaLyte |  | 2 h | – | – | – | A | – |  |
|  | – | – | – |  | – |  |  |  | 10% UG in PlasmaLyte |  | 3 h | – | – | – | A | – |  |
|  | – | – | – |  | – |  |  |  | 20% UG in PlasmaLyte |  | 0 h | – | – | – | A | – |  |
|  | – | – | – |  | – |  |  |  | 20% UG in PlasmaLyte |  | 1 h | – | – | – | A | – |  |
|  | – | – | – |  | – |  |  |  | 20% UG in PlasmaLyte |  | 2 h | – | – | – | A | – |  |
|  | – | – | – |  | – |  |  |  | 20% UG in PlasmaLyte |  | 3 h | – | – | – | A | – |  |

57

Exmp.12-5

| Solution | 37 °C | Time | Result |
|---|---|---|---|
| PlasmaLyte | 0 | 1 h | A |
| | | 2 h | A |
| | | 3 h | A |
| Saline | | 1 h | A |
| | | 2 h | A |
| | | 3 h | A |
| 230mM NaCl | | 1 h | A |
| | | 2 h | A |
| | | 3 h | A |
| 150mM KCl | | 1 h | B |
| | | 2 h | B |
| | | 3 h | C |
| 100mM MgCl$_2$ | | 1 h | C |
| | | 2 h | A |
| | | 3 h | A |
| 11% Trehalose | | 1 h | B |
| | | 2 h | A |
| | | 3 h | B |
| 10% Sucrose | | 1 h | A |
| | | 2 h | A |
| | | 3 h | B |
| 5% Sucrose | | 1 h | A |
| | | 2 h | A |
| | | 3 h | A |
| 10% UG in PlasmaLyte | | 1 h | A |
| | | 2 h | A |
| | | 3 h | A |
| 20% UG in PlasmaLyte | | 1 h | A |
| | | 2 h | A |
| | | 3 h | A |

37 °C   5ml   4e7/ml   2

EP 4 151 717 A1

[Table 8-6]

| Collect. Method | Pretreatment Base | Pretreatment Additive | Pretreatment Time | Freezing Cell Den. | Freezing Additive | Freezing Volume | Thawing Condition | Thawing Standing | Thawing solvent 1 Base | Thawing solvent 1 Additive/Temp. | Thawing solvent 1 Time | Thawing solvent 2 Base | Thawing solvent 2 Additive | Thawing solvent 2 Time | Results Viability | Results Activity | Exmp.No. |
|---|---|---|---|---|---|---|---|---|---|---|---|---|---|---|---|---|---|
| | − | − | − | | − | | | | PlasmaLyte | | 0 h | − | − | − | C | − | |
| | − | − | − | | − | | | | | | 1 h | − | − | − | C | − | |
| | − | − | − | | − | | | | | | 2 h | − | − | − | C | − | |
| | − | − | − | | − | | | | | | 3 h | − | − | − | C | − | |
| | − | − | − | | − | | | | Saline | | 0 h | − | − | − | C | − | |
| | − | − | − | | − | | | | | | 1 h | − | − | − | C | − | |
| | − | − | − | | − | | | | | | 2 h | − | − | − | C | − | |
| | − | − | − | | − | | | | | | 3 h | − | − | − | C | − | |
| | − | − | − | | − | | | | 230mM NaCl | | 0 h | − | − | − | C | − | |
| | − | − | − | | − | | | | | | 1 h | − | − | − | C | − | |
| | − | − | − | | − | | | | | | 2 h | − | − | − | C | − | |
| | − | − | − | | − | | | | | | 3 h | − | − | − | C | − | |
| | − | − | − | | − | | | | 150mM KCl | | 0 h | − | − | − | C | − | |
| | − | − | − | | − | | | | | | 1 h | − | − | − | C | − | |
| | − | − | − | | − | | | | | | 2 h | − | − | − | C | − | |
| | − | − | − | | − | | | | | | 3 h | − | − | − | C | − | |
| | − | − | − | | − | | | | 100mM MgCl$_2$ | | 0 h | − | − | − | C | − | |
| | − | − | − | | − | | | | | | 1 h | − | − | − | C | − | |
| | − | − | − | | − | | | | | | 2 h | − | − | − | C | − | |
| | − | − | − | | − | | | | | | 3 h | − | − | − | C | − | |
| | − | − | − | | − | | | | 100mM CaCl$_2$ | | 0 h | − | − | − | C | − | |
| | − | − | − | | − | | | | | | 1 h | − | − | − | C | − | |
| | − | − | − | | − | | | | | | 2 h | − | − | − | C | − | |
| | − | − | − | | − | | | | | | 3 h | − | − | − | C | − | |
| | − | − | − | | − | | | | 11% Trehalose | | 0 h | − | − | − | B | − | |
| | − | − | − | | − | | | | | | 1 h | − | − | − | C | − | |
| | − | − | − | | − | | | | | | 2 h | − | − | − | C | − | |
| | − | − | − | | − | | | | | | 3 h | − | − | − | C | − | |
| | − | − | − | | − | | | | 10% Sucrose | | 0 h | − | − | − | B | − | |
| 1 | − | − | − | 1e7/ml | − | 1ml | 37□ | 0 | | R.T. | 1 h | − | − | − | C | − | Exmp.12-6 |
| | − | − | − | | − | | | | | | 2 h | − | − | − | C | − | |
| | − | − | − | | − | | | | | | 3 h | − | − | − | C | − | |

| | | | | | | |
|---|---|---|---|---|---|---|
| | 3 h | | | | C | |
| 5% Sucrose | 0 h | — | — | — | B | — |
| | 1 h | — | — | — | C | — |
| | 2 h | — | — | — | C | — |
| | 3 h | — | — | — | C | — |
| 10% UG in PlasmaLyte | 0 h | — | — | — | B | — |
| | 1 h | — | — | — | C | — |
| | 2 h | — | — | — | C | — |
| | 3 h | — | — | — | C | — |
| 20% UG in PlasmaLyte | 0 h | — | — | — | B | — |
| | 1 h | — | — | — | C | — |
| | 2 h | — | — | — | C | — |
| | 3 h | — | — | — | C | — |
| 10% AB in PlasmaLyte | 0 h | — | — | — | B | — |
| | 1 h | — | — | — | C | — |
| | 2 h | — | — | — | C | — |
| | 3 h | — | — | — | C | — |
| 20% AB in PlasmaLyte | 0 h | — | — | — | B | — |
| | 1 h | — | — | — | C | — |
| | 2 h | — | — | — | C | — |
| | 3 h | — | — | — | C | — |
| 30% AB in PlasmaLyte | 0 h | — | — | — | B | — |
| | 1 h | — | — | — | C | — |
| | 2 h | — | — | — | C | — |
| | 3 h | — | — | — | C | — |
| 40% AB in PlasmaLyte | 0 h | — | — | — | B | — |
| | 1 h | — | — | — | C | — |
| | 2 h | — | — | — | C | — |
| | 3 h | — | — | — | C | — |

Exmp.12-7

| Condition | | |
|---|---|---|
| PlasmaLyte | 1 h | 2 h | 3 h |
| Saline | 1 h | 2 h | 3 h |
| 230mM NaCl | 1 h | 2 h | 3 h |
| 150mM KCl | 1 h | 2 h | 3 h |
| 100mM MgCl$_2$ | 1 h | 2 h | 3 h |
| 100mM CaCl$_2$ | 1 h | 2 h | 3 h |
| 11% Trehalose | 1 h | 2 h | 3 h |
| 10% Sucrose | 1 h | 2 h | 3 h |
| 5% Sucrose | 1 h | 2 h | 3 h |
| 10% UG in PlasmaLyte | 1 h | 2 h | 3 h |
| 20% UG in PlasmaLyte | 1 h | 2 h | 3 h |
| 10% AB in PlasmaLyte | 1 h | 2 h | 3 h |
| 20% AB in PlasmaLyte | 1 h | 2 h | 3 h |
| 30% AB in PlasmaLyte | 1 h | 2 h | 3 h |
| 40% AB in PlasmaLyte | 1 h | 2 h | 3 h |

37℃

0

37℃

1ml

1e7/ml

1

**Claims**

1. A method for treating cells, the method comprising:

    (1) collecting in vitro-activated cells in a medium for cell culture;
    (2) suspending the collected cells in a solution for cryopreservation; and
    (3) freezing the suspended cells.

2. The method according to claim 1, wherein the step (1) includes a treatment with a medium supplemented with anyselected from the group consisting of a bile acid and phenylbutyric acid.

3. The method according to claim 1 or 2, which further comprises the step of:
   (4) thawing the cryopreserved cells and suspending them in a thawing solvent I.

4. The method according to any one of claims 1 to 3, wherein the thawing solvent I is an aqueous solution containing the followings:

    - Sodium chloride 9.00 to 108 mM
    - Sodium gluconate 2.30 to 27.7 mM
    - Sodium acetate 2.70 to 32.5 mM
    - Potassium chloride 0.496 to 5.96 mM, and
    - Magnesium chloride 0.148 to 1.78 mM.

5. The method according to any one of claims 1 to 4, wherein the thawing solvent I satisfies at least one of the following criteria:

    - The solvent does not contain calcium ions at a concentration of 0.423 mM or higher,
    - The solvent does not contain glucose at a concentration of 5.55 mM or higher, and
    - The solvent does not contain lactate at a concentration of 27.7 mM or higher.

6. A solution for suspending highly active NK cells, the solution comprising:

    - Sodium chloride 9.00 to 108 mM,
    - Sodium gluconate 2.30 to 27.7 mM
    - Sodium acetate 2.70 to 32.5 mM
    - Potassium chloride 0.496 to 5.96 mM, and
    - Magnesium chloride 0.148 to 1.78 mM.

7. The solution according to claim 6, which is for suspending highly active NK cells that have been suspended in a solution for cryopreservation and frozen.

8. A pharmaceutical composition comprising:

    highly active NK cells,
    a solution for cryopreservation, and
    the solution according to claim 6.

9. A method for producing a pharmaceutical composition containing cells, the method comprising:

    (1) collecting in vitro-activated cells in a medium for cell culture;
    (2) suspending the collected cells in a solution for cryopreservation; and
    (3) freezing the suspended cells.

10. The method according to claim 9, wherein the step (1) comprises a treatment with a medium supplemented with any selected from the group consisting of a bile acid and phenylbutyric acid.

[Fig. 1-1]

[Fig. 1-2]

[Fig. 1-3]

37°C, 3hrs (containing IL-2)

[Fig. 1-4]

[Fig. 2]

[Fig. 3]

[Fig. 4]

[Fig. 5]

[Fig. 6-1]

[Fig. 6-2]

[Fig. 7-1]

[Fig. 7-2]

N=2, each
Mean + S.E.

[Fig. 8-1]

[Fig. 8-2]

HBSS (+)

KBM, 5%UltraGRO

Lactoringer

PBS (-)

PBS (-) + 1.48mM Mg$^{2+}$

Plasma-LyteA

7-AAD

HBSS (+)

KBM, 5%UltraGRO

Lactoringer

PBS (-)

PBS (-) + 1.48mM Mg$^{2+}$

Plasma-LyteA

7-AAD

[Fig. 8-3]

PBS

PlasmaLyteA

PlasmaLyteA+Lactic acid

PlasmaLyteA+MgSO4

Lactoringer

Veen-F (aceticringer)

SOLITA-T1

SOLITA-T3

5%Glucose

RPMI, 10%FBS

$-10^2\ 0\ 10^2 \qquad 10^3 \qquad 10^4 \qquad 10^5$

7-AAD

[Fig. 8-4]

[Fig. 8-5]

[Fig. 9-1]

A

Plasma-Lyte A , 3000IU/mL

0h

1h

2h

4h

$-10^2\ 0\ 10^2\qquad 10^3\qquad 10^4\qquad 10^5$

7-AAD

[Fig. 9-2]

[Fig. 9-3]

Time of standing after thawing and dilution

[Fig. 10]

[Fig. 11]

[Fig. 12-1]

[Fig. 12-2]

[Fig. 12-3]

[Fig. 12-4]

[Fig. 12-5]

[Fig. 12-6]

[Fig. 12-7]

[Fig. 12-8]

[Fig. 12-9]

[Fig. 12-10]

## INTERNATIONAL SEARCH REPORT

| International application No. |
| --- |
| PCT/JP2021/007863 |

**A. CLASSIFICATION OF SUBJECT MATTER**
Int.Cl. C12N1/04(2006.01)i, A61K9/10(2006.01)i, A61K35/17(2015.01)i,
A61K47/02(2006.01)i, A61K47/12(2006.01)i, A61P31/12(2006.01)i,
A61P35/00(2006.01)i, A61P37/04(2006.01)i, C12N5/071(2010.01)i,
C12N5/0783(2010.01)i
FI: C12N1/04, C12N5/071, A61K9/10, A61K47/02, A61K47/12, A61K35/17A,
A61P35/00, A61P31/12, A61P37/04, C12N5/0783
According to International Patent Classification (IPC) or to both national classification and IPC

**B. FIELDS SEARCHED**

Minimum documentation searched (classification system followed by classification symbols)
Int.Cl. C12N1/04, A61K9/10, A61K35/17, A61K47/02, A61K47/12, A61P31/12,
A61P35/00, A61P37/04, C12N5/071, C12N5/0783

Documentation searched other than minimum documentation to the extent that such documents are included in the fields searched
Published examined utility model applications of Japan      1922-1996
Published unexamined utility model applications of Japan    1971-2021
Registered utility model specifications of Japan            1996-2021
Published registered utility model applications of Japan    1994-2021

Electronic data base consulted during the international search (name of data base and, where practicable, search terms used)
JSTPlus/JMEDPlus/JST7580(JDreamIII), CAplus/MEDLINE/EMBASE/BIOSIS(STN)

**C. DOCUMENTS CONSIDERED TO BE RELEVANT**

| Category* | Citation of document, with indication, where appropriate, of the relevant passages | Relevant to claim No. |
| --- | --- | --- |
| X<br>Y<br>A | JP 2002-233356 A (HUMANTEC LTD.) 20 August 2002<br>(2002-08-20), examples | 1, 3, 9<br>1, 3-5, 9<br>2, 6-8, 10 |
| Y<br>A | JP 2020-518260 A (MESOBLAST INT SARL) 25 June 2020<br>(2020-06-25), claim 1, paragraph [0228] | 1, 3-5, 9<br>2, 6-8, 10 |

☐ Further documents are listed in the continuation of Box C.    ☒ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search<br>15 April 2021 | Date of mailing of the international search report<br>27 April 2021 |
| --- | --- |
| Name and mailing address of the ISA/<br>Japan Patent Office<br>3-4-3, Kasumigaseki, Chiyoda-ku,<br>Tokyo 100-8915, Japan | Authorized officer<br><br>Telephone No. |

Form PCT/ISA/210 (second sheet) (January 2015)

| INTERNATIONAL SEARCH REPORT | International application No. |
|---|---|
| Information on patent family members | PCT/JP2021/007863 |

| | | |
|---|---|---|
| JP 2002-233356 A | 20 August 2002 | US 2003/0013074 A1<br>embodiment |
| JP 2020-518260 A | 25 June 2020 | WO 2018/202853 A1<br>claim 1, paragraph [0233] |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2006230396 A
- JP 2002233356 A
- WO 2011021618 A
- JP 2018193303 A
- JP 2019170176 A

**Non-patent literature cited in the description**

- **LIU E. et al.** *N. Engl. J. Med.,* 2020, vol. 382, 545-53
- **LEONG JW et al.** *Biol. Blood Marrow Transplant,* 2014, vol. 20, 463-473